# EUROPEAN PATENT APPLICATION

(11) **EP 1 806 415 A2**
(43) Date of publication of application: **11.07.2007**
(21) Application number: 07007406.7
(22) Date of filing: 08.03.2004
(51) Int. Cl.: C12Q 1/68

(54) **Standardized and optimized real-time quantitative reverse transcriptase polymerase chain reaction method for detection of MRD in leukemia**

(30) Priority: 07.03.2003 EP 03290572
(62) Divisional of application: 04718310.8
(71) Applicant: Université de la Méditerranée, 13284 Marseille Cédex 07 (FR)
(72) Inventor: Gabert, Jean, 13480 Callas (FR); Beillard, Emmanuel, Los Angeles, CA 90024 (US); Bi, Wanli, San Ramon, CA 94583 (US); Van Dongen, Jacques, 1914 La Nieuwerkerk a/d IJssel (NL)
(74) Representative: Peaucelle, Chantal

(57) **Abstract**

The invention relates to a real-time quantitative reverse transcriptase polymerase chain reaction (RQ-PCR) method for minimal residual disease detection in leukemic patients through amplification of a fusion gene transcript, comprising:
(i) selecting amplifiable and qualified patient samples for subsequent analysis;
(ii) defining optimal conditions for performing the RT reaction ;
(iii) defining optimal conditions for RQ-PCR protocol; and
(iv) establishing a standardized procedure for data analysis,
wherein GUS is amplified in parallel to the fusion gene transcript as control gene transcript.

SEQUENCE LISTING

<110>  UNIVERSITE DE LA MEDITERRANEE

<120>  Standardized and optimized real-time quantitative reverse
       transcriptase polymerase chain reaction method for detection of
       MRD in leukemia

<130>  60938-2764Div1

<150>  EP 03 290 572.1
<151>  2003-03-07

<150>  PCT/EP04/004008
<151>  2004-03-08

<150>  EP 04 718.310.8
<151>  2004-03-08

<160>  47

<170>  PatentIn version 3.3

<210>  1
<211>  31
<212>  DNA
<213>  Human

<400>  1
tggagataac actctaagca taactaaagg t                                    31


<210>  2
<211>  28
<212>  DNA
<213>  Human

<400>  2
ccatttttgg tttgggcttc acaccatt                                        28


<210>  3
<211>  21
<212>  DNA
<213>  Human

<400>  3
gatgtagttg cttgggaccc a                                               21


<210>  4
<211>  18
<212>  DNA
<213>  Human

<400>  4
gagtatgcct gccgtgtg                                                   18


<210>  5
<211>  26
<212>  DNA
<213>  Human

<400>  5
cctccatgat gctgcttaca tgtctc                                          26

<210>  6
<211>  18
<212>  DNA
<213>  Human

<400>  6
aatccaaatg cggcatct                                                    18


<210>  7
<211>  26
<212>  DNA
<213>  Human

<400>  7
gaaaatatgt ggttggagag ctcatt                                           26


<210>  8
<211>  26
<212>  DNA
<213>  Human

<400>  8
ccagcactct cgtcggtgac tgttca                                           26


<210>  9
<211>  22
<212>  DNA
<213>  Human

<400>  9
   ccgagtgaag atcccctttt ta                                              22


<210>  10
<211>  19
<212>  DNA
<213>  Human

<400>  10
ccagcctcat gcacaacca                                                   19


<210>  11
<211>  22
<212>  DNA
<213>  Human

<400>  11
ccctccctga cctgtctcgg cc                                               22


<210>  12
<211>  21
<212>  DNA
<213>  Human

<400>  12
gggctcctcg gatactcaaa a                                                21


<210>  13
<211>  25
<212>  DNA
<213>  Human

<400> 13
cccaagtatc cctgtaaaac aaaaa 25

<210> 14
<211> 23
<212> DNA
<213> Human

<400> 14
gatggagtcc acaggatcag agt 23

<210> 15
<211> 22
<212> DNA
<213> Human

<400> 15
catggccgcc tcctttgaca gc 22

<210> 16
<211> 22
<212> DNA
<213> Human

<400> 16
gaaaggaaac ttggatggct ca 22

<210> 17
<211> 19
<212> DNA
<213> Human

<400> 17
ctctgtctcc ccgcctgaa 19

<210> 18
<211> 20
<212> DNA
<213> Human

<400> 18
tcccaatggg catggcgtgc 20

<210> 19
<211> 16
<212> DNA
<213> Human

<400> 19
cggctcgtgc tggcat 16

<210> 20
<211> 18
<212> DNA
<213> Human

<400> 20
ctggcccaac gatggcga 18

<210> 21

<211>  25
<212>  DNA
<213>  Human

<400>  21
cccttcagcg gccagtagca tctga                                          25


<210>  22
<211>  21
<212>  DNA
<213>  Human

<400>  22
cactcagacc ctgaggctca a                                              21


<210>  23
<211>  21
<212>  DNA
<213>  Human

<400>  23
tccgctgacc atcaayaagg a                                              21


<210>  24
<211>  21
<212>  DNA
<213>  Human

<400>  24
tccgctgacc atcaayaagg a                                              21


<210>  25
<211>  21
<212>  DNA
<213>  Human

<400>  25
cactcagacc ctgaggctca a                                              21


<210>  26
<211>  19
<212>  DNA
<213>  Human

<400>  26
cgctcctacc ctgcaaaca                                                 19


<210>  27
<211>  22
<212>  DNA
<213>  Human

<400>  27
acctcagctc cgcggaagtt gc                                             22


<210>  28
<211>  19
<212>  DNA
<213>  Human

<400>  28

ccgaggaaga ggatgcaca 19

<210> 29
<211> 19
<212> DNA
<213> Human

<400> 29
tcttcctgcc caacagcaa 19

<210> 30
<211> 19
<212> DNA
<213> Human

<400> 30
acctggatgg accgcctag 19

<210> 31
<211> 19
<212> DNA
<213> Human

<400> 31
ccgatggctt cgacgagtt 19

<210> 32
<211> 20
<212> DNA
<213> Human

<400> 32
agtgcccagc cctccctcgc 20

<210> 33
<211> 21
<212> DNA
<213> Human

<400> 33
gcttgtagat gcggggtaga g 21

<210> 34
<211> 22
<212> DNA
<213> Human

<400> 34
cattagcaca acaggccttt ga 22

<210> 35
<211> 22
<212> DNA
<213> Human

<400> 35
tcgcgtgtcc ttctccgagc ct 22

<210> 36
<211> 17

<212>  DNA
<213>  Human

<400>  36
agggcccgct tggactt                                                    17


<210>  37
<211>  21
<212>  DNA
<213>  Human

<400>  37
cctcgttaag catccctgtg a                                               21


<210>  38
<211>  23
<212>  DNA
<213>  Human

<400>  38
ctctttctcc agcgtctgct tat                                             23


<210>  39
<211>  22
<212>  DNA
<213>  Human

<400>  39
cacctaccac agagccatca aa                                              22


<210>  40
<211>  30
<212>  DNA
<213>  Human

<400>  40
aacctcgaaa tcgtactgag aagcactcca                                      30


<210>  41
<211>  22
<212>  DNA
<213>  Human

<400>  41
atccacaggt gagtctggca tt                                              22


<210>  42
<211>  18
<212>  DNA
<213>  Human

<400>  42
ccttcagcgg ccagtagc                                                   18


<210>  43
<211>  19
<212>  DNA
<213>  Human

<400>  43
ggacacaggc ccatggtac                                                  19

<210>  44
<211>  19
<212>  DNA
<213>  Human

<400>  44
ccttgaggct atccagcgt                                                    19


<210>  45
<211>  23
<212>  DNA
<213>  Human

<400>  45
cctgctcaga tacatcaaac atg                                               23


<210>  46
<211>  20
<212>  DNA
<213>  Human

<400>  46
cctgtgacct ttgtgagcaa                                                   20


<210>  47
<211>  19
<212>  DNA
<213>  Human

<400>  47
gtctgccgtg aacagtcca                                                    19

## Description

### FIELD OF THE INVENTION :

The present invention relates to a real-time quantitative PCR (RQ-PCR) method for minimal residual disease detection in leukemic patients through the amplification of a fusion gene transcript.

### BACKGROUNG OF THE INVENTION :

Current treatment protocols for acute lymphoblastic leukemia (ALL), acute myeloid leukemia (AML) and chronic myeloid leukemia (CML) are based on prognostic factors, which contribute to therapy stratification.(1-3). Key prognostic factors identified in leukemia over the years include pre-treatment characteristics such as age, WBC count, immunophenotypic profiles, specific chromosomal abnormalities, aberrant fusion genes, and mutations, such as FLT3 gene alterations in AML. Response to initial therapy provides a further well-known prognostic marker; in particular the presence or absence of blasts in the bone marrow after induction therapy in ALL and AML, or cytogenetic response in CML patients.

However, it is important to stress that patient outcome cannot be reliably predicted on the basis of such classical parameters, thereby underlining the potential importance of minimal residual disease (MRD) testing. Over the last ten years, technological developments have enabled the detection of leukemic cells beyond the threshold of cytomorphology or karyotyping (4).

Three different techniques are currently used with a sensitivity of at least one leukemic cell in a background of 103 normal cells : 1. immunophenotyping, 2. polymerase chain reaction (PCR) using genomic DNA for detection of clonal rearrangements of immunoglobulin (Ig) and T cell receptor (TCR) genes in ALL, and 3. reverse-transcriptase-PCR (RT-PCR) for detection of gene rearrangements, mainly FG transcripts resulting from chromosomal translocations. While the first two directly measure the tumor load, the latter method measures gene expression.

MRD information in leukemia patients has been clearly established as an independent prognostic factor in three pathological situations: 1) childhood ALL, in particular after induction therapy ; 2) BCR-ABL detection in CML patients after allogeneic stem cell transplantation, enabling direction of donor leukocyte infusions and 3) PML-RARA detection in acute promyelocytic leukemia (APL) patients after consolidation therapy, confering benefit for pre-emptive therapy at the point of molecular relapse in comparison to frank relapse. These data have led to the introduction of molecular monitoring in the stratification strategy in some current multicenter therapeutic trials.

However, the clinical impact of MRD detection in other types of leukemias remains to be demonstrated in large series of patients.

In order to tackle the problem of lack of standardized diagnostic methodology, eight years ago European laboratories conducted a collaborative program through a BIOMED-1 Concerted Action.

This Concerted Action led to the development of standardized nested RT-PCR assays achieving sensitivities of at least 10⁻⁴ (RNA diluted into RNA) suitable for detection of MRD as well as diagnostic screening (5).

However, "end-point" PCR analyses do not permit precise quantification of MRD levels. This limitation was underlined by the finding of low levels of AML1-ETO, CBFB-MYH111 or PML-RARA,(1) transcripts in patients in long-term clinical remission and by the detection of BCR-ABL mRNA at very low levels in healthy individuals. Quantitative PCR analysis has been achieved by competitive PCR. Expert laboratories showed that this technique enables accurate prediction of relapse suggesting that such analysis could be used for adapting treatment in BCR-ABL-positive CML or in AML patients with CBFB-MYH 11 or AML1-ETO transcripts.

However, this competitive PCR is labor intensive and time consuming which prohibits both standardization and large-scale multicenter analysis.

More recently, real-time quantitative PCR (RQ-PCR) has been introduced (6). There have been numerous manuscripts from individual laboratories demonstrating the reliability of this technology and its potential clinical value for MRD studies using FG transcripts as PCR targets such as BCR-ABL in CML, or ALL, PML-RARA, AML1-ETO and CBFB-MYH11 in AML and TEL-AML1 in ALL patients.

However, standardized RQ-PCR procedures are still warranted in order to apply this innovative technology for large-scale MRD studies within multicenter therapeutic trials.

The present inventors designed a joint project of the health and consumer protection of the European Commission (SANCO) via the "Europe Against Cancer" (EAC) program in order to develop standardization and quality control analysis for RQ-PCR, based on the ABI 7700 platform (Applied Biosystems, Foster City - USA) as it was the first such technology available. The major aim was to establish a standardized protocol allowing comparison of MRD data in order to assess the relative efficiency of each therapeutic strategy for leukemia bearing an appropriate molecular marker. The most frequently occurring fusion gene transcripts in leukemia were selected, covering up to 30 to 40 % of childhood and adult ALL and AML and more than 95 % of CML patients.

As mentioned hereinabove, compared to standard RT-PCR, RQ-PCR enable accurate quantification of gene expression. An attractive feature of this technique is that crucial parameters such as the RNA quality and quantity can be evaluated. This is accomplished by parallel amplification of the target gene and one or more control genes (CG), also called house-keeping or endogenous reference genes.

A suitable CG in any applications of RQ-PCR analysis can be defined as a gene with a stable expression in all nucleated cells among different analyzed samples which is unaffected by any experimental treatment. Impaired amplification of CGs should be accompanied by a corresponding reduction of target gene transcript quantity, reflecting variations in RNA quality, quantity and cDNA synthesis efficiency. Thus, quantification of CG expression could be used for detecting poor quality samples, based on reference values observed in a large number of fresh samples. Furthermore, RQ-PCR would allow to assess an experimental sensitivity per sample, which is particularly important for PCR negative follow-up samples.

Although the literature on RQ-PCR assays is rapidly expanding, no concerted effort related to the selection of appropriate CGs has been published so far.

The choice of a CG remains a crucial issue and a consensus has still to be found. To date, numerous CG for MRD detection by RQ-PCR are still used: ABL, ACTB, B2M, GAPDH, PBGD, TBP, and 18s rRNA. Inclusion of CG analysis should significantly enhance the reliability of MRD detection in leukemic patients.

However, this is critically dependent upon optimization and standardization of CG and FG assays.

### SUMMARY OF THE INVENTION:

For these reasons, inventors of the present application more particularly focused on the design, optimization and standardization of the FG RQ-PCR analysis with special reference to the selection and validation of suitable CGs for RQ-PCR assays in a large panel of normal and leukemic samples.

Accordingly, an aim of the present invention is to provide a significant improvement in a quantitative reverse transcriptase polymerase chain reaction (RQ-PCR) method for minimal residual disease detection in leukemic patients through the amplification of a fusion gene transcript, said improvement comprising :
(i) selecting amplifiable and qualified patient sample for subsequent analysis ;
(ii) defining optimal conditions for performing the RT reaction;
(iii) defining optimal conditions for RQ-PCR protocol ; and
(iv) establishing standardized procedure for data analysis.

The present invention could advantageously provide the basis for an International reference of MRD studies using RQ-PCR analysis of FG transcripts.

In the invention method, an appropriate control gene transcript should be amplified in parallel to the fusion gene transcript.

Inclusion of control genes to correct for sample variations makes the invention method applicable for gene transcript detection, thus creating a platform for future studies, where RQ-PCR assays should be crucial to assess therapeutic efficiency, notably for innovative drugs like inhibitors for tyrosine kinase, phosphatidyl-inositol-3-kinase or farnesyl-transferase proteins.

Therapeutic strategies will be adapted according to such standardized MRD evaluations. It will be a great improvement to be assisted by such biological data assist in therapeutic decision making, such as in unrelated allogeneic transplant, infusion of donor lymphocytes or basically the choice of the most efficient therapeutic strategy.

Furthermore, the standardized method defined for markers in acute and chronic leukemia according to the present invention, could be a model for other biological markers in onco-hematology and more broadly in the oncology field.

Standardization and quality control programs for novel technologies such as semi-automated MRD detection today, but also high throughput chip DNA technologies tomorrow, are mandatory in ensuring that advances achieved through innovative genomic methodologies yield maximal benefit in improving the outcome of patients with leukemia.

According to a preferred embodiment, the control gene to be used in the invention method, is selected in the group consisting of *ABL, B2M* and *GUS.*

Preferably forward primer, probe, and reverse primer sequences for *ABL, B2M* and *GUS* are respectively as follows :
SEQ ID N°1 (ENF1003), SEQ ID N°2 (ENPr1043) and SEQ ID N°3 (ENR1063) ; and
SEQ ID N°7 (ENF1102), SEQ ID N° 8 (ENPr1142) and SEQ ID N°9 (ENR 1162).

The sequences and localisation of these three preferred control genes sets are given in Table 37.

Most preferably, the selected control gene to be used in the invention method is *ABL.*

In a preferred aspect of this embodiment all samples with a *ABL,* value within a reference range, respectively from 1.3x10³ to 1.3x10⁵ copies or Ct from 21.9 to 29.3, are considered as an amplifiable sample and are qualified for subsequent analysis.

It is another object of the present invention to provide a set of primers and probes specific for each fusion gene transcript to be used in the invention method.

Accordingly in the invention method :
- when the fusion gene is *E2A-PBX1,* the corresponding forward primer, probe, and reverse primer sequences thereof are respectively as follows :
   SEQ ID N°10 (ENF101), SEQ ID N°11 (ENP141) and SEQ ID N°12 (ENR161).
- when the fusion gene is *MLL-AF4,* the corresponding forward primers, probe, and reverse primer sequences thereof are respectively as follows :
   SEQ ID N° 13 (ENF207), SEQ ID N° 14 (ENF208), SEQ ID N° 15 (ENP242) and SEQ ID N° 16 (ENR262).
- when the fusion gene is *TEL-AML1,* the corresponding forward primer, probe, and reverse primer sequences thereof are respectively as follows :
   SEQ ID N°17 (ENF301), SEQ ID N°18 (ENPr341) and SEQ ID N°19 (ENR361).
- when the fusion gene is *BCR-ABL m-bcr,* the corresponding forward primer, probe, and reverse primer sequences thereof are respectively as follows :
   SEQ ID N°20 (ENF402), SEQ ID N°21 (ENP541) and SEQ ID N°22 (ENR561).
- when the fusion gene is *BCR-ABL M-bcr,* the corresponding forward primer, probe, and reverse primer sequences thereof are respectively as follows :
   SEQ ID N°23 (ENF501), SEQ ID N°24 (ENP541) and SEQ ID N°25 (ENR561).
- when the fusion gene is *SIL-TAL1,* the corresponding forward primer, probe, and reverse primer sequences thereof are respectively as follows :
   SEQ ID N°26 (ENF601), SEQ ID N°27 (ENP641) and SEQ ID N°28 (ENR664).
- when the fusion gene is *PML-RARA,* the corresponding forward primers, probe, and reverse primer sequences thereof are respectively as follows :
   SEQ ID N°29 (ENF903), SEQ ID N°30 (ENF906), SEQ ID N° 31 (ENF905), SEQ ID N°32 (ENP942) and SEQ ID N°33 (ENR962).
- when the fusion gene is *CBFB-MYH11,* the corresponding forward primer, probe, and reverse primers sequences thereof are respectively as follows :
   SEQ ID N°34 (ENF803), SEQ ID N°35 (ENPr843), SEQ ID N°36 (ENR862), SEQ ID N°37 (ENR863) and SEQ ID N°38 (ENR865).
- when the fusion gene is *AML-ETO,* the corresponding forward primer, probe, and reverse primer sequences thereof are respectively as follows :
   SEQ ID N°39 (ENF701), SEQ ID N°40 (ENP747), and SEQ ID N°41 (ENR761).

The sequences and positions of these primers and probes are given in Tables 5,8,11,14,17,21,24,27 and 30.

In a most prefered embodiment of the method of the present invention step (ii) of said method is as follows :
Add 1 µg of total RNA in 10 µl of H2O ;
Incubate at 70°C for 10 min ;
Cool on ice and add following reagents to final volume of 20 µl:
Reverse Transcriptase (either MMLV or Superscript I or II): 100 U
RT Buffer (according to the RTase used)
DNTP: 1 Mm
DTT: 10 Mm
Random Hexamers: 25 µM
RNAse inhibitor: 20 U
Incubate subsequently at:
   Room temperature for 10 min ;
   42°C for 45 min ; and
   99°C for 3 min.
   Place the sample at 4°C after RT step ; and
   Dilute the final cDNA with 30 µl of H2O.

More preferably step (iii) of the invention method is as follows :
Final volume: 25 µl ;
   5 µl of final cDNA (100 ng RNA equivalent) ;
   Primers: 300 nM each ;
   Probe: 200 nM except for the AML1-ETO probe (100 nM) ;
   Master Mix: 12.5 µl (1X)
   Incubate the sample:
   At 50°C for 2 min ; and
   At 95°C for 10 min ;
   Followed by 50 cycles:
   95°C for 15 sec ; and
   60°C for 1 min.

Advantageously, step (iv) of the invention method proceeds as follows :
Set a common threshold at 0,1 except for PML-RARA (0,05) ; and
Set a common baseline between cycle 3 and 15 except for B2M (3-10).

The present invention will now be described in more detail with reference to the following Tables and to the appended drawings wherein :

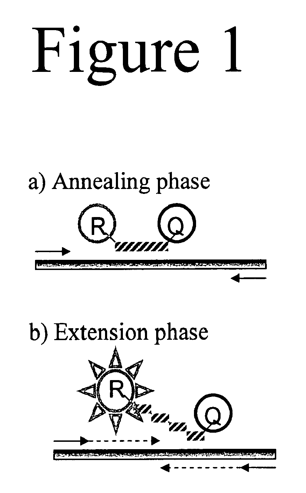

Figure 1 illustrates the principle of RQ PCR detection using hydrolysis probe (TaqMan™). a) The bi-fluorescent probe (R: reporter, Q: quencher) hybridizes on the complementary nucleotide sequence of the template during the PCR reaction. b) The probe is degraded during the extension phase by the DNA polymerase enzyme resulting in an increasing fluorescence detected by the machine in the PCR tube ;

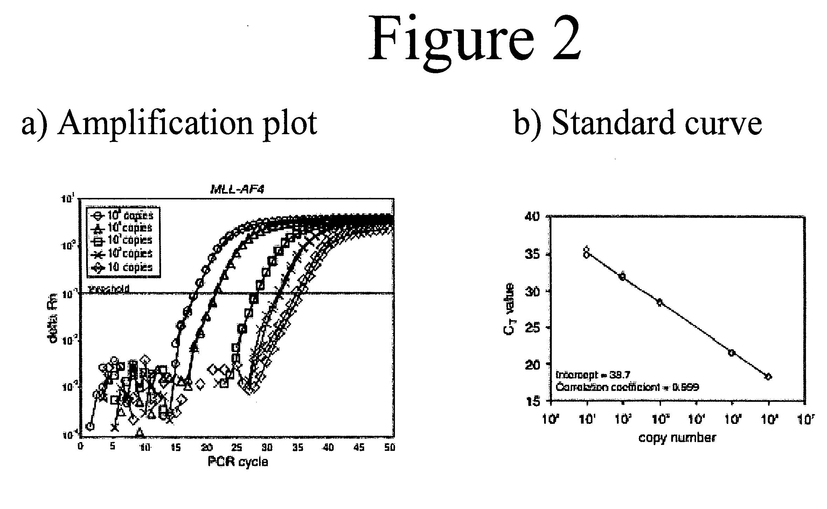

Figure 2 illustrates the amplification of MLL (ex 10) - AF4 (ex 4) plasmid using EAC primer and probe sets. a) Amplification plots (Log₁₀ scale) of five DNA plasmid dilutions (10⁶ to 10 molecules), b) Standard curve obtained with the five plasmid dilutions ;

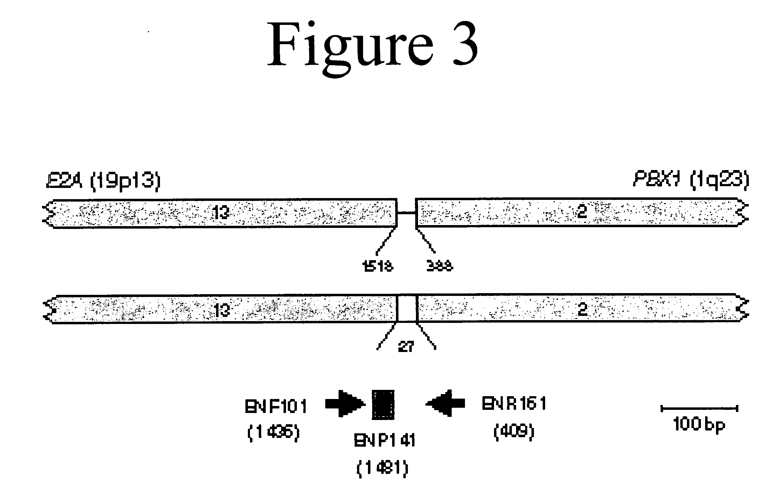

Figure 3 is a schematic diagram of the E2A-PBX1 fusion gene transcript covered by the RQ-PCR primer and probe set (ENF101 - ENP141 - ENR161). The number under the primers and probe refers to their 5' nucleotide position in the normal gene transcript (see Table 5) ;

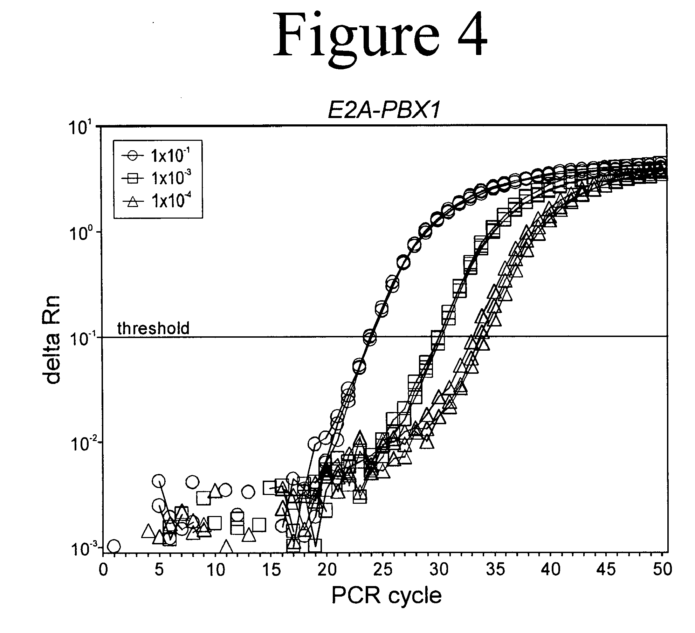

Figure 4 illustrates the amplification plots of 10⁻¹, 10⁻³ and 10⁻⁴ dilutions of a E2A-PBX1 positive RNA sample in a negative RNA sample ;

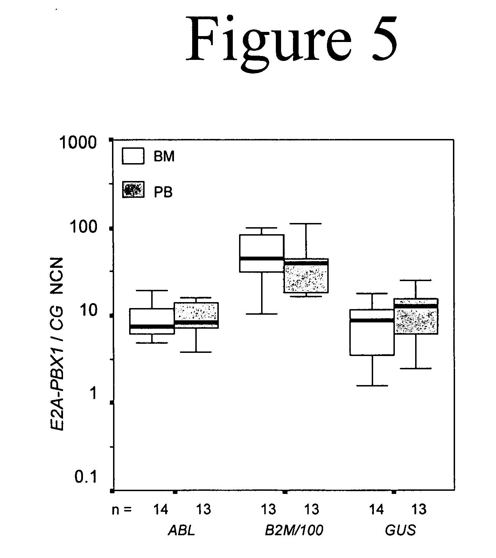

Figure 5 illustrates the comparison of E2A-PBX1 fusion gene transcript expression between BM and PB at diagnosis using either ABL, B2M or GUS as control gene. The ratio (NCN) is defined as the ratio between the copy number of E2A-PBX1 per one copy of ABL or GUS gene and 100 copies of B2M gene. The median value corresponds to the black bold line. The box refers to the range defined by the 25th and the 75th percentile. All differences between BM and PB per control gene are not significant on paired samples (n=10, Wilcoxon test). n: number of patient samples ;

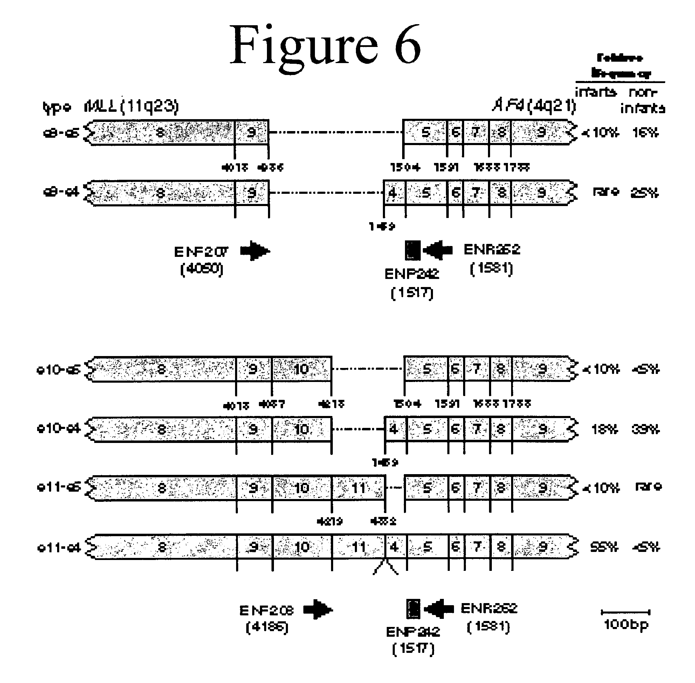

Figure 6 is a schematic diagram of the MLL-AF4 fusion gene transcript covered by the RQ-PCR primer and probe set. Two RQ-PCR sets are available with a common probe and a common reverse primer on AF4 exon 5 (ENR262 - ENP242). The first set uses a forward primer on MLL exon 9 (ENF207) and the second set uses a forward primer on MLL exon 10 (ENF208). MLL-AF4 FG transcripts lacking AF4 exon 5 can not be amplified by the EAC sets. Taken together, the sets cover approximately 75% of the MLL-AF4 variants either in infant or non infant patients (see relative frequency on the right side of the diagram). The number under the primers and probe refers to their 5' nucleotide position in the normal gene transcript (see Table 8) ;

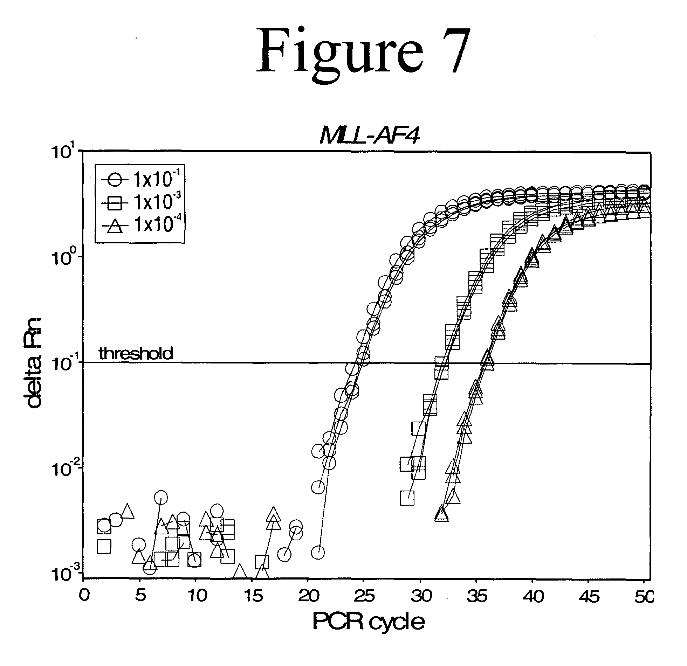

Figure 7 illustrates the amplification plots of 10⁻¹, 10⁻³ and 10⁻⁴ dilutions of a RS4;11 cell line RNA in a negative PB MNC RNA sample ;

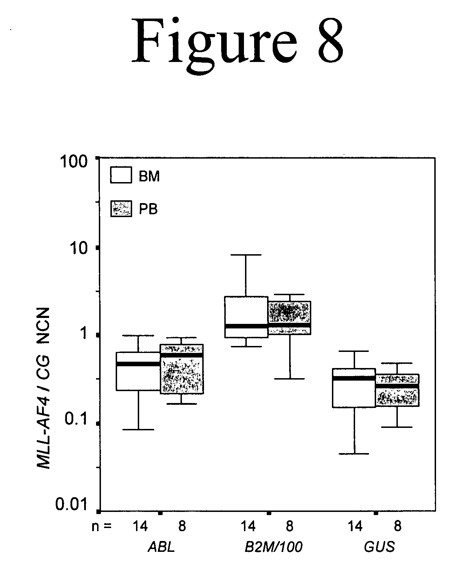

Figure 8 illustrates the comparison of MLL-AF4 fusion gene transcript expression between BM and PB at diagnosis using either ABL, B2M or GUS as control gene. The ratio (NCN) is defined as the ratio between the copy number of MLL-AF4 per one copy of ABL or GUS gene and 100 copies of B2M gene. The median value corresponds to the black bold line. The box refers to the range defined by the 25th and the 75th percentile. n: number of patient samples ;

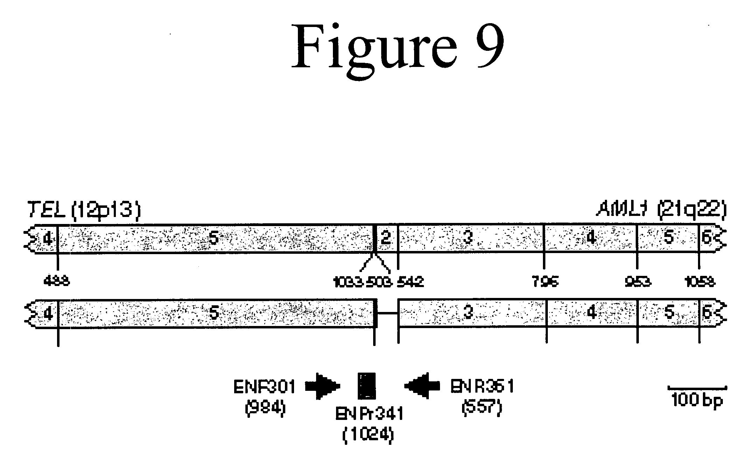

Figure 9 is a schematic diagram of the TEL-AML1 fusion gene transcript covered by the RQ-PCR primer and probe set (ENF301 - ENPr341 - ENR361). The number under the primers and probe refers to their 5' nucleotide position in the normal gene transcript (see Table 11) ;

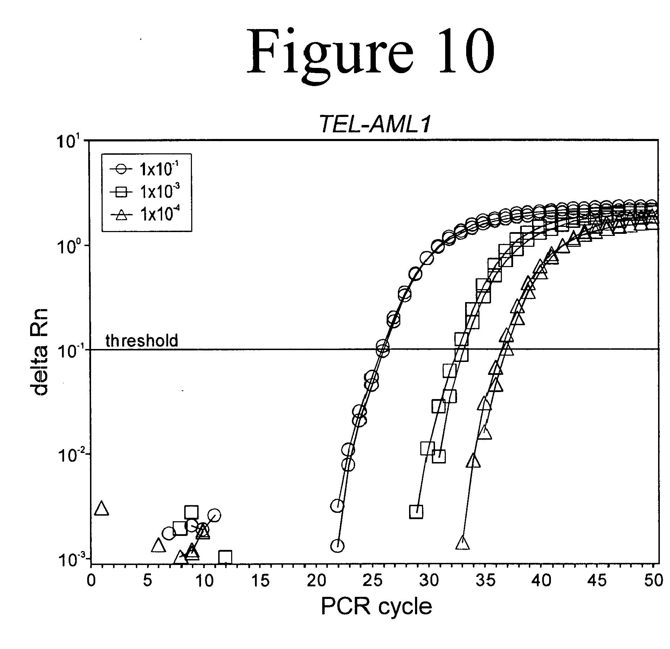

Figure 10 illustrates the amplification plots of 10⁻¹, 10⁻³ and 10⁻⁴ dilutions of a TEL-AML1 positive RNA sample in a negative RNA sample ;

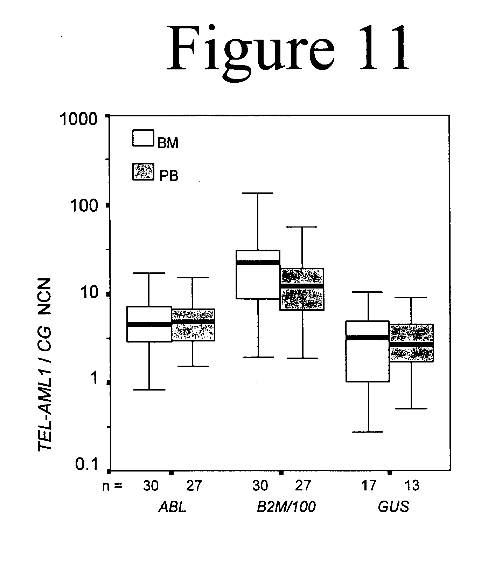

Figure 11 illustrates the comparison of TEL-AML1 fusion gene transcript expression between BM and PB at diagnosis using either ABL, B2M or GUS as control gene. The ratio (NCN) is defined as the ratio between the copy number of TEL-AML1 per one copy of ABL or GUS gene and 100 copies of B2M gene. The median value corresponds to the black bold line. The box refers to the range defined by the 25th and the 75th percentile. All differences between BM and PB per control gene are not significant on paired samples (n=23, Wilcoxon test). n: number of patient samples ;

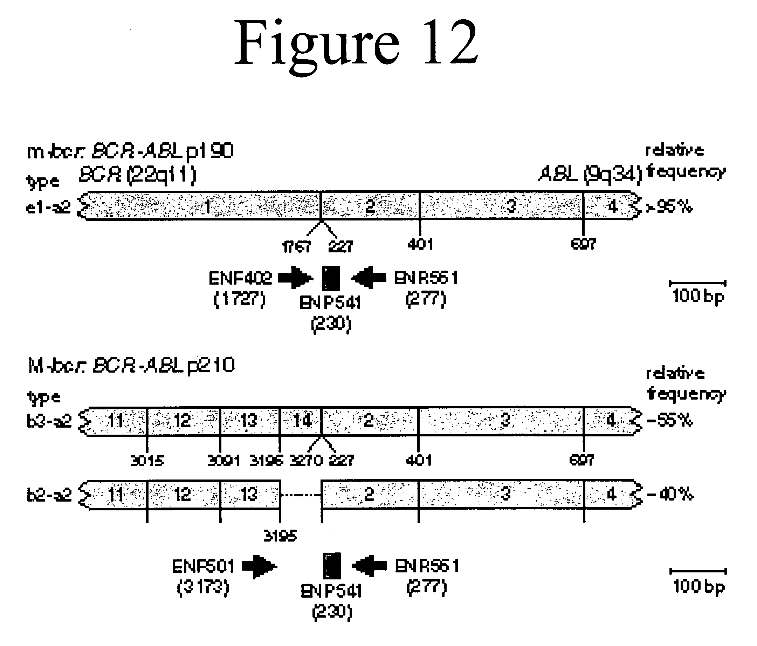

Figure 12 is a schematic diagram of the BCR-ABL fusion gene transcript covered by the RQ-PCR primer and probe set. For m-bcr, set: ENF402 - ENP541 - ENR561 and for M-bcr, set: ENF501 - ENP541 - ENR561. The number under the primers and probe refers to their 5' nucleotide position in the normal gene transcript (see Tables 14 and 17). Relative frequency refers to the proportion of e1-a2 transcript among m-bcr variants and proportion of b3-a2 and b2-a2 transcripts among M-bcr variants ;

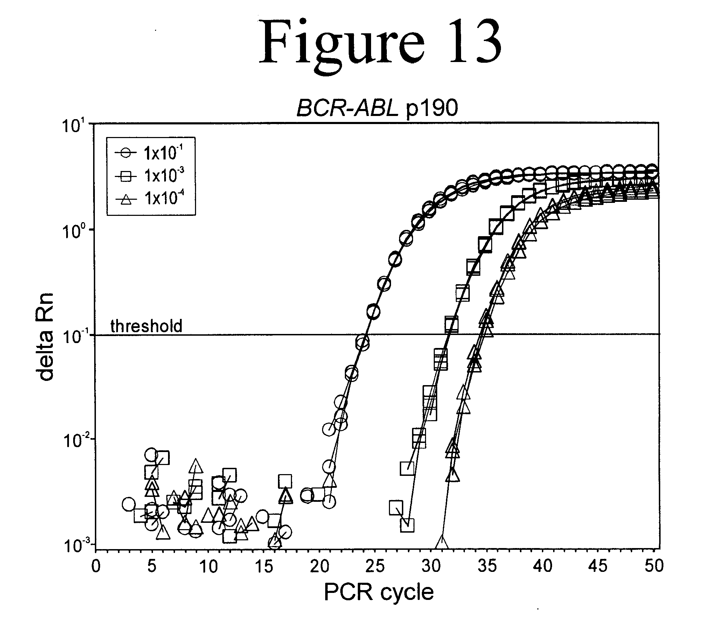

Figure 13 illustrates the amplification plots of 10⁻¹, 10⁻³ and 10⁻⁴ dilutions of a BCR-ABL m-bcr positive RNA sample in a negative RNA sample ;

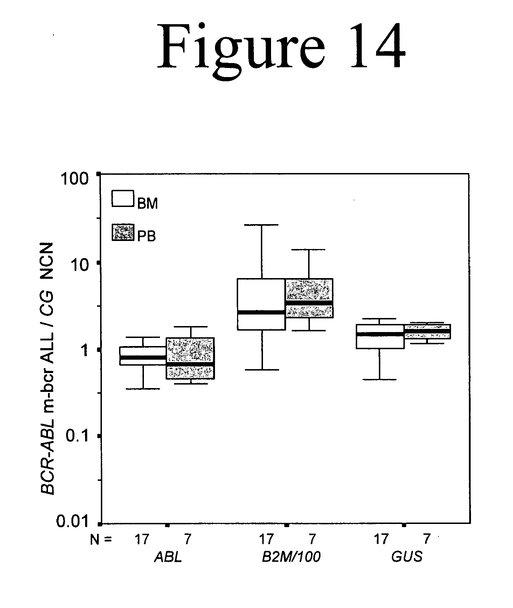

Figure 14 illustrates the comparison of BCR-ABL m-bcr fusion gene transcript expression between BM and PB at diagnosis using either ABL, B2M or GUS as control gene. The ratio (NCN) is defined as the ratio between the copy number of BCR-ABL m-bcr per one copy of ABL or GUS gene and 100 copies of B2M gene. The median value corresponds to the black bold line. The box refers to the range defined by the 25th and the 75th percentile. n: number of patient samples ;

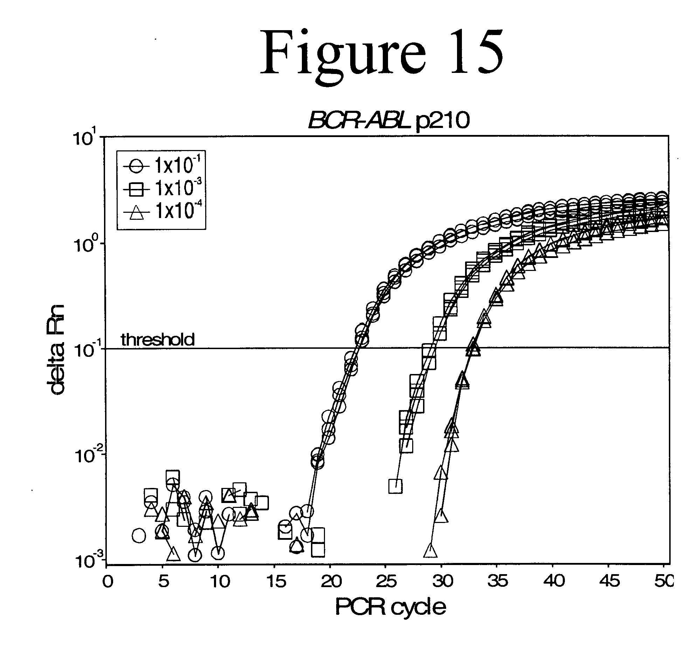

Figure 15 illustrates the amplification plots of 10⁻¹, 10⁻³ and 10⁻⁴ dilutions of a BCR-ABL M-bcr positive RNA sample in a negative RNA sample ;

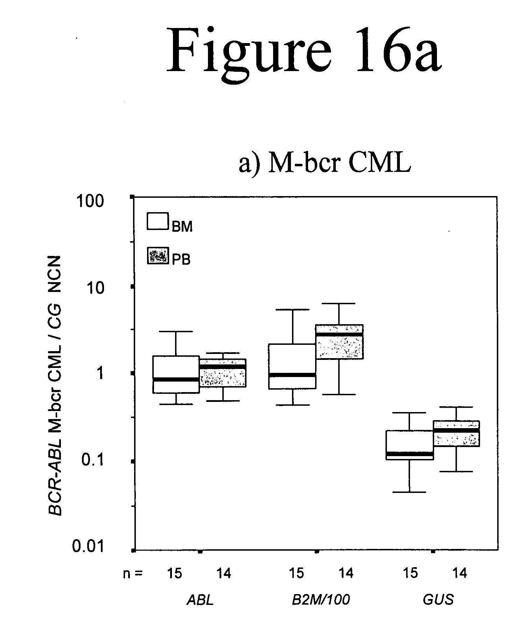

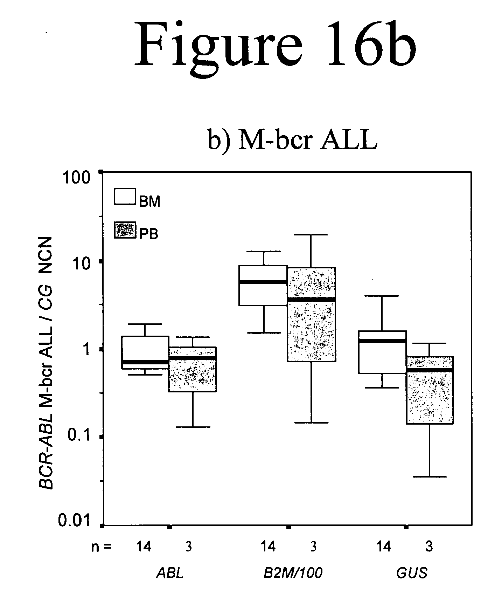

Figure 16 illustrates the comparison of BCR-ABL M-bcr fusion gene transcript expression between BM and PB using either ABL, B2M or GUS as control gene. a) in CML samples, b) in ALL samples. The ratio (NCN) is defined as the ratio between the copy number of BCR-ABL M-bcr per one copy of ABL or GUS gene and 100 copies of B2M gene. The median value corresponds to the black bold line. The box refers to the range defined by the 25th and the 75th percentile. n: number of patient samples ;

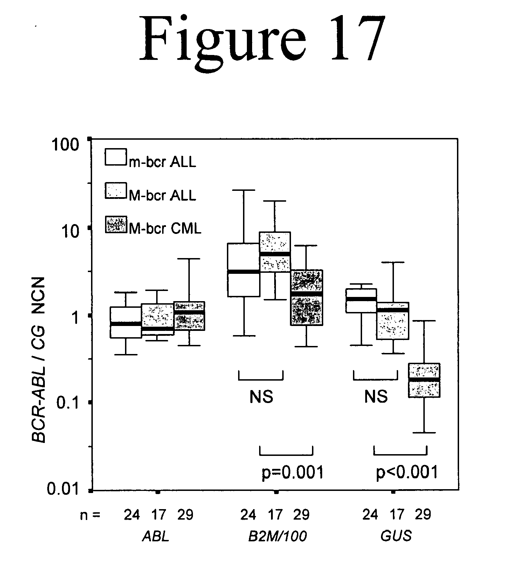

Figure 17 illustrates the comparison of BCR-ABL fusion gene transcript expression between m-bcr ALL, M-bcr ALL and M-bcr CML at diagnosis using either ABL, B2M or GUS as control gene. PB and BM were pooled according to each group to compare BCR-ABL fusion gene expression between m-bcr ALL, M-bcr ALL and M-bcr CML. No significant difference was observed between m-bcr and M-bcr ALL either with B2M or GUS control gene (Mann-Whitney test). A highly significant difference was found between BCR-ABL positive ALL (n=41) and CML (n=29) samples either with B2M or GUS control gene (Mann-Whitney test). n: number of patient samples ;

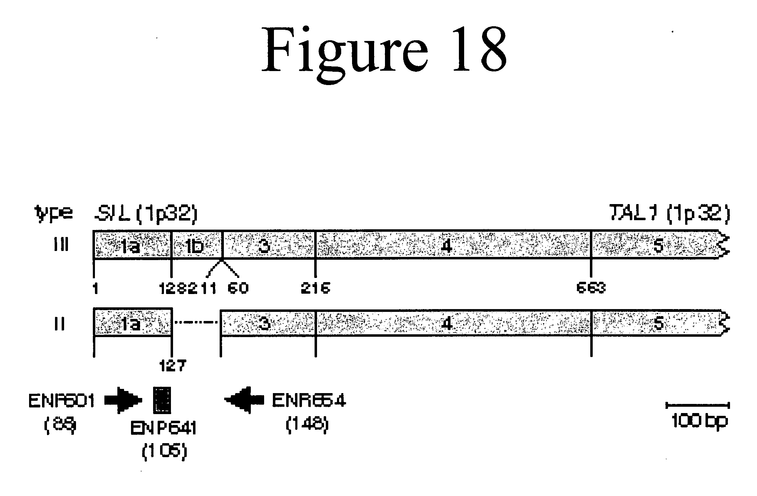

Figure 18 is a schematic diagram of the SIL-TAL1 fusion gene transcript covered by the RQ-PCR primer and probe set (ENF601 - ENP641 - ENR664). The number under the primers and probe refers to their 5' nucleotide position in the normal gene transcript (see Table 21) ;

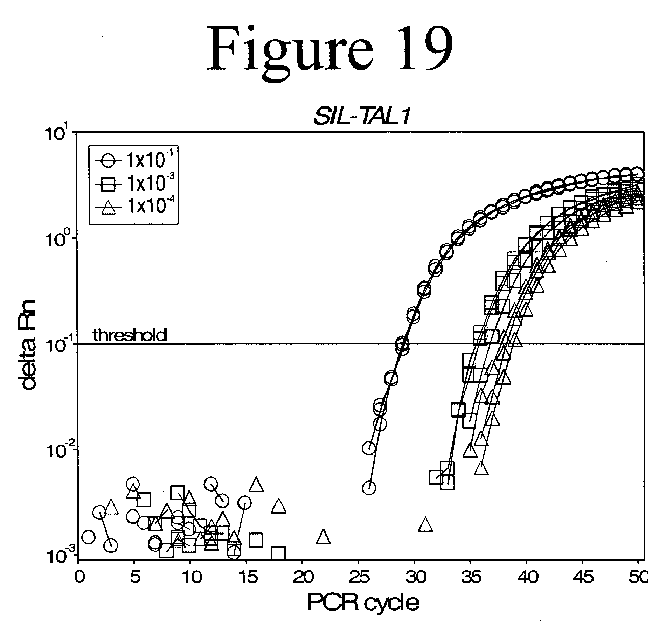

Figure 19 illustrates the amplification plots of 10⁻¹, 10⁻³ and 10⁻⁴ dilutions of a SIL-TAL1 positive RNA sample in a negative RNA sample ;

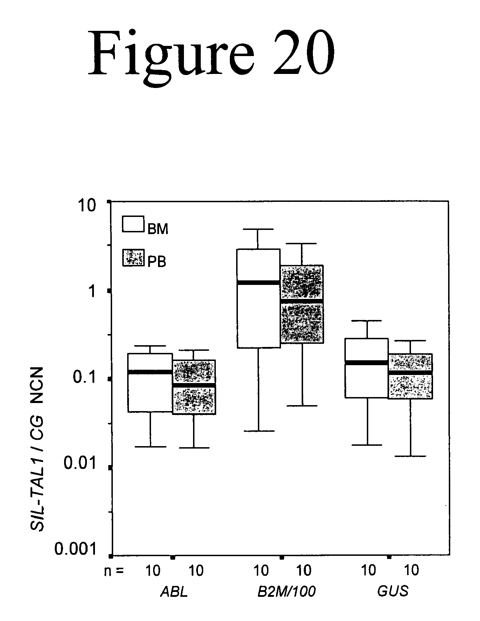

Figure 20 illustrates the comparison of SIL-TAL1 fusion gene transcript expression between BM and PB at diagnosis using either ABL, B2M or GUS as control gene. The ratio (NCN) is defined as the ratio between the copy number of SIL-TAL1 per one copy of ABL or GUS gene and 100 copies of B2M gene. The median value corresponds to the black bold line. The box refers to the range defined by the 25th and the 75th percentile. All differences between BM and PB per control gene are not significant on paired samples (n=5, Wilcoxon test). n: number of patient samples ;

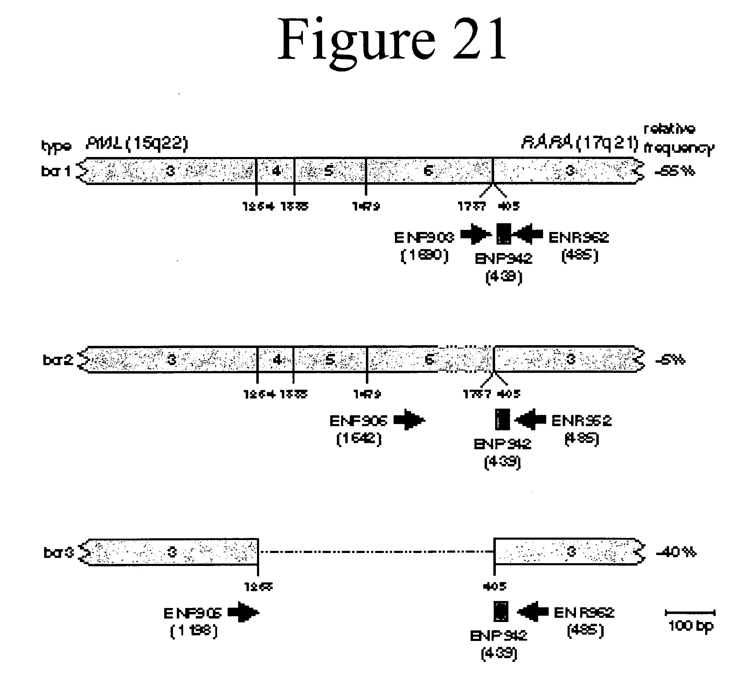

Figure 21 is a schematic diagram of the PML-RARA fusion gene transcript covered by the RQ-PCR primer and probe set. Three sets are available to cover all the PML-RARA transcript variants. A common probe (ENP942) and a common reverse primer (ENR962) on RARA gene exon 3 are used. BCR1, BCR2 and BCR3 variants are respectively amplified with ENF903, ENF906 and ENF905 forward primers. The relative frequency of each particular transcript appears on the right side of the diagram. The number under the primers and probe refers to their 5' nucleotide position in the normal gene transcript (see Table 24) ;

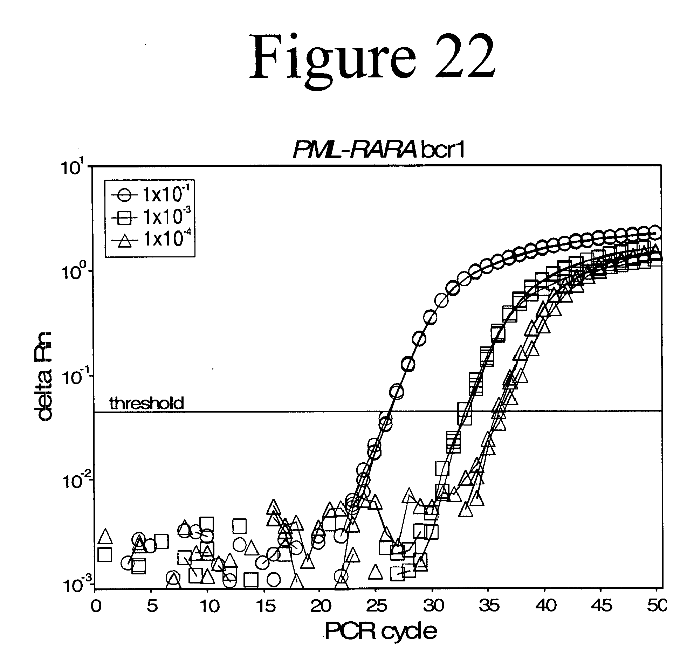

Figure 22 illustrates the amplification plots of 10⁻¹, 10⁻³ and 10⁻⁴ dilutions of a PML-RARA BCR1 positive RNA sample in a negative RNA sample ;

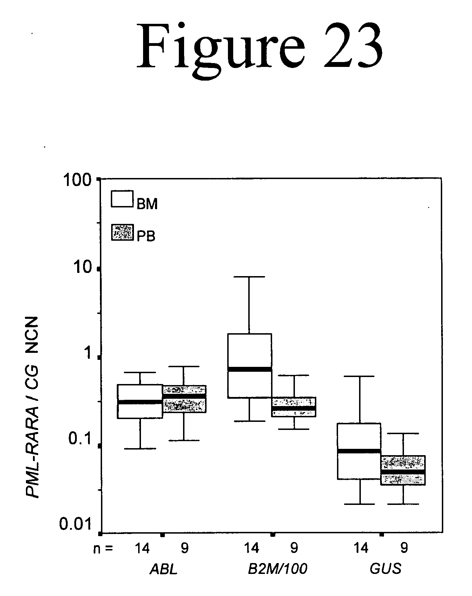

Figure 23 illustrates the comparison of PML-RARA fusion gene transcript expression between BM and PB at diagnosis using either ABL, B2M or GUS as control gene. The ratio (NCN) is defined as the ratio between the copy number of PML-RARA per one copy of ABL or GUS gene and 100 copies of B2M gene. The median value corresponds to the black bold line. The box refers to the range defined by the 25th and the 75th percentile. All differences between BM and PB per control gene are not significant on paired samples (n=6, Wilcoxon test) except for B2M NCN (p=0.03). n: number of patient samples ;

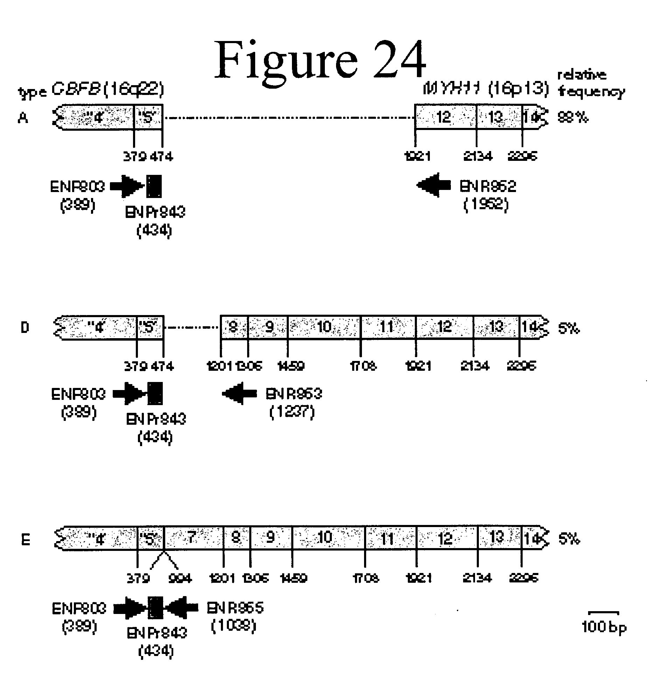

Figure 24 is a schematic diagram of the CBFB-MYH11 fusion gene transcripts covered by the RQ-PCR primer and probe set. a) Type A: set ENF803 - ENPr843 - ENR862, b) Type D: set ENF803 - ENPr843 - ENR863, c) Type E: set ENF803 - ENPr843 - ENR865. The relative frequency of each particular transcript appears on the right side of the diagram. The number under the primers and probe refers to their 5' nucleotide position in the normal gene transcript (see Table 27) ;

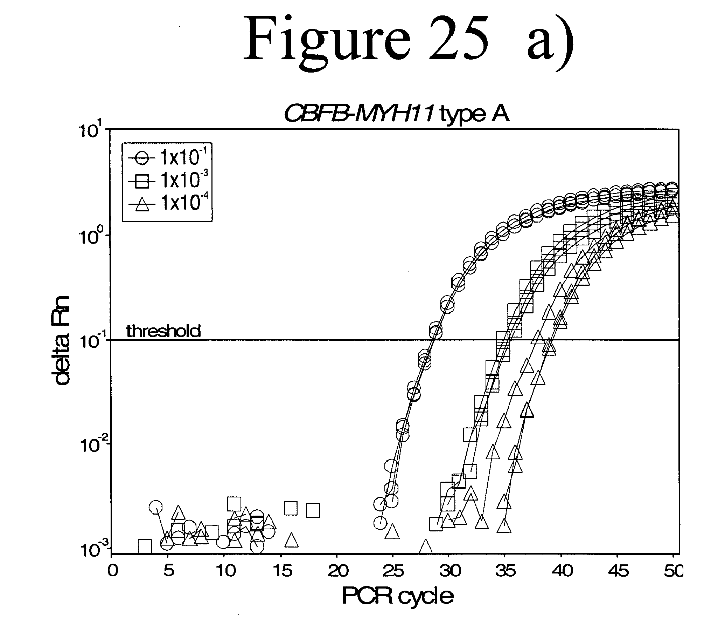

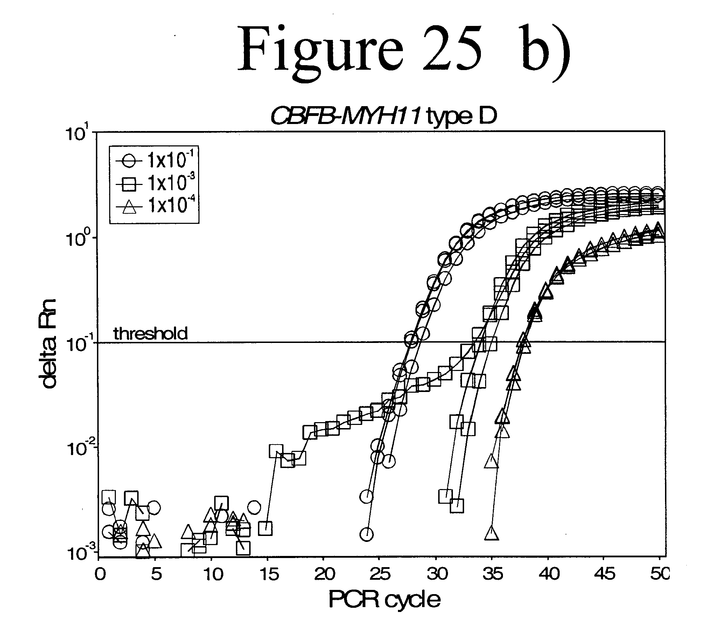

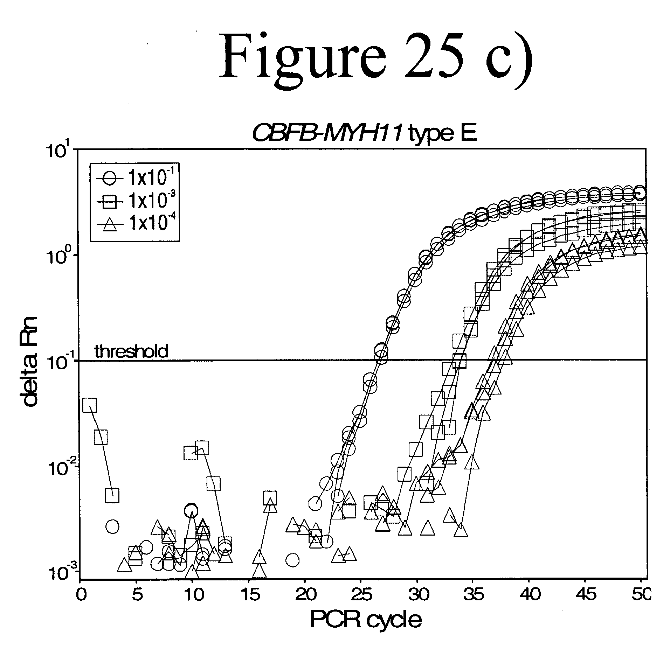

Figure 25 illustrates the amplification plots of 10⁻¹, 10⁻³ and 10⁻⁴ dilutions of a CBFB-MYH11 positive RNA sample in a negative RNA sample: a) Set type A on ME-1 cell line, b) Set type D on patient sample, c) Set type E on patient sample;

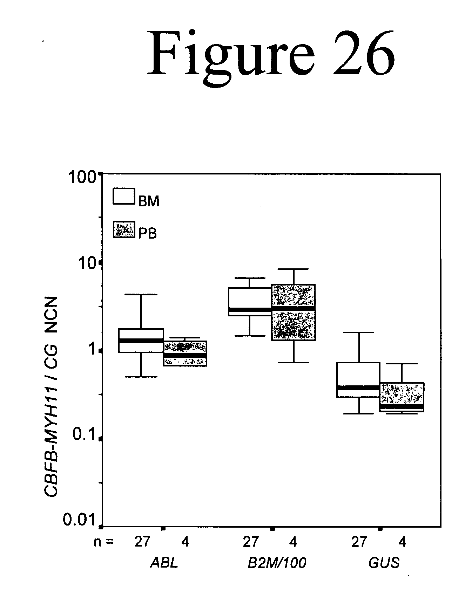

Figure 26 illustrates the comparison of CBFB-MYH11 fusion gene transcript expression between BM and PB at diagnosis using either ABL, B2M or GUS as control gene. Samples have been pooled according to their origin (BM or PB), independently of the transcript type. The ratio (NCN) is defined as the ratio between the copy number of CBFB-MYH11 per one copy of ABL or GUS gene and 100 copies of B2M gene. The median value corresponds to the black bold line. The box refers to the range defined by the 25th and the 75th percentile. n: number of patient samples ;

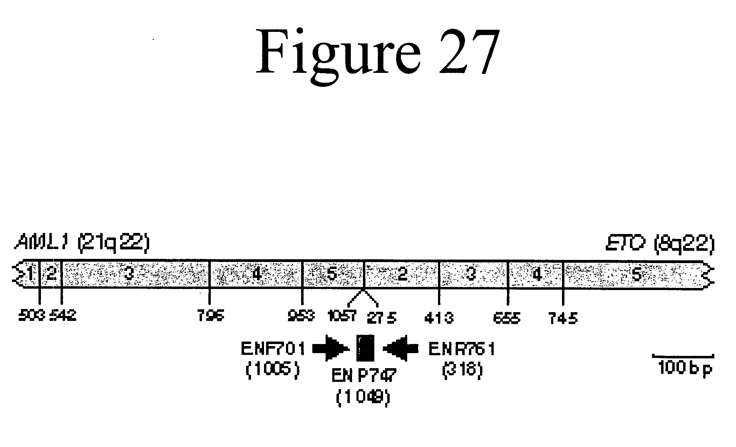

Figure 27 is a schematic diagram of the AML1-ETO fusion gene transcript covered by the RQ-PCR primer and probe set. EAC codes (ENF701, ENP747, ENR761). The number under the primers and probe refers to their 5' nucleotide position in the normal gene transcript (see Table 30). The probe is located on the breakpoint ;

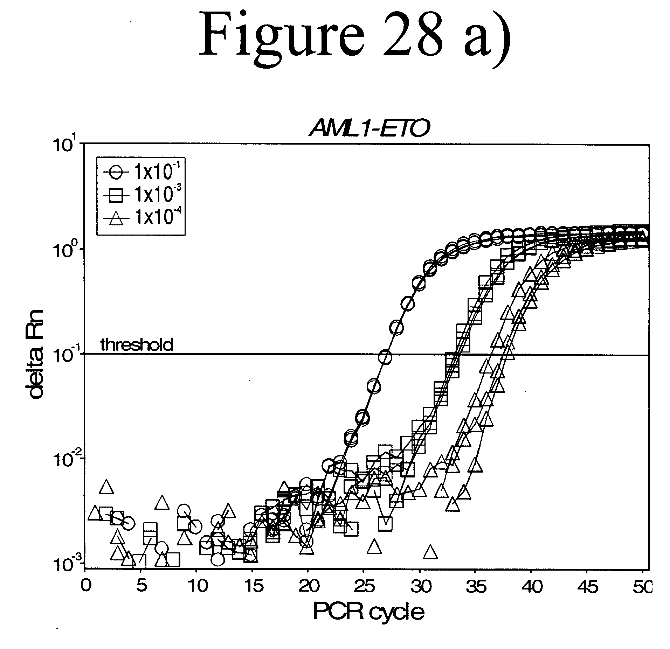

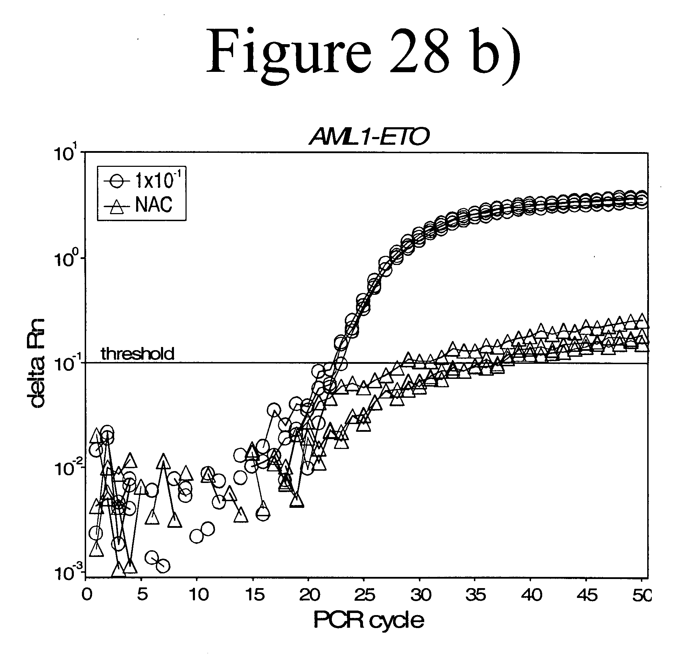

Figure 28 illustrates the amplification plots of 10⁻¹, 10⁻³ and 10⁻⁴ dilutions of a AML1-ETO positive RNA sample in a negative RNA sample. a) Classical amplification plot. b) Creeping curve phenomenon observed on NAC samples ;

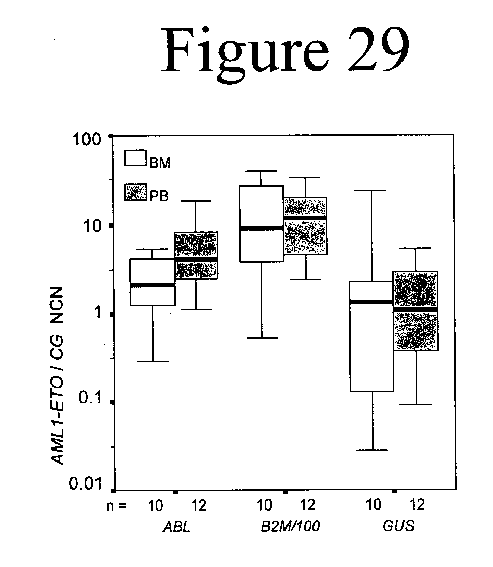

Figure 29 illustrates the comparison of AML1-ETO fusion gene transcript expression between BM and PB at diagnosis using either ABL, B2M or GUS as control gene. The ratio (NCN) is defined as the ratio between the copy number of AML1-ETO per one copy of ABL or GUS gene and 100 copies of B2M gene. The median value corresponds to the black bold line. The box refers to the range defined by the 25th and the 75th percentile. All differences between BM and PB per control gene are not significant on paired samples (n=10, Wilcoxon test). However, the use of GUS gene is not recommended due to the large range of values observed for the ratio AML1-ETO / GUS. n: number of patient samples ;

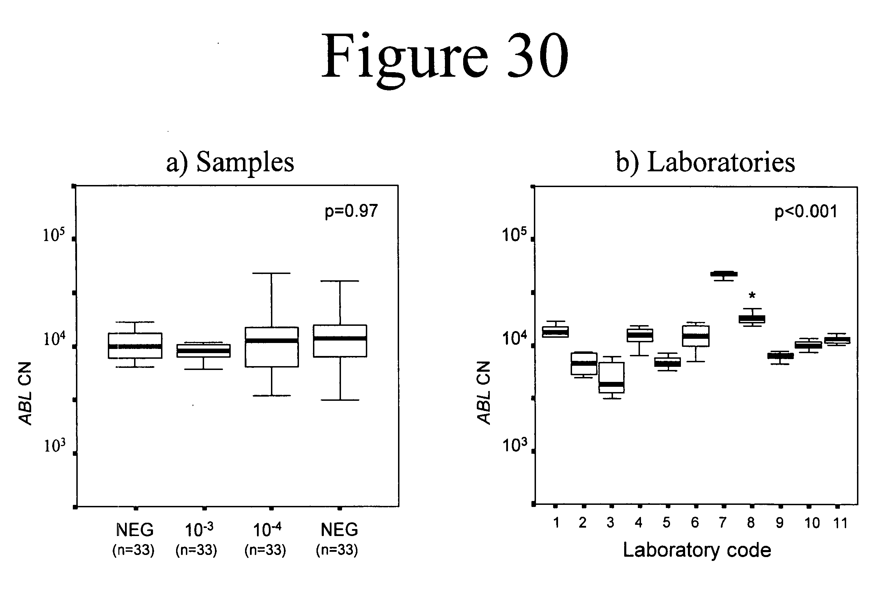

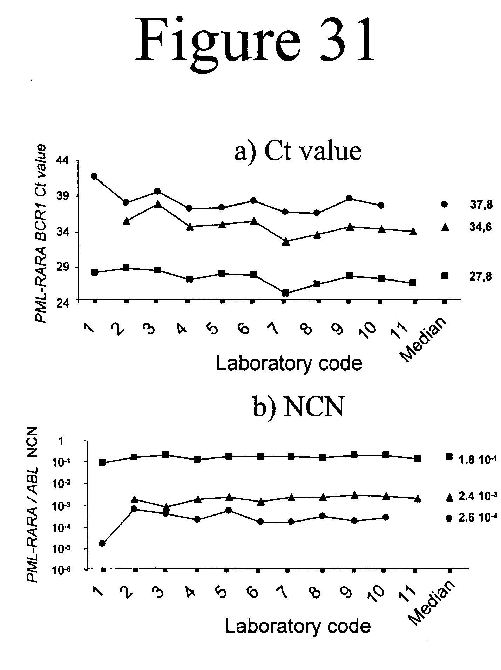

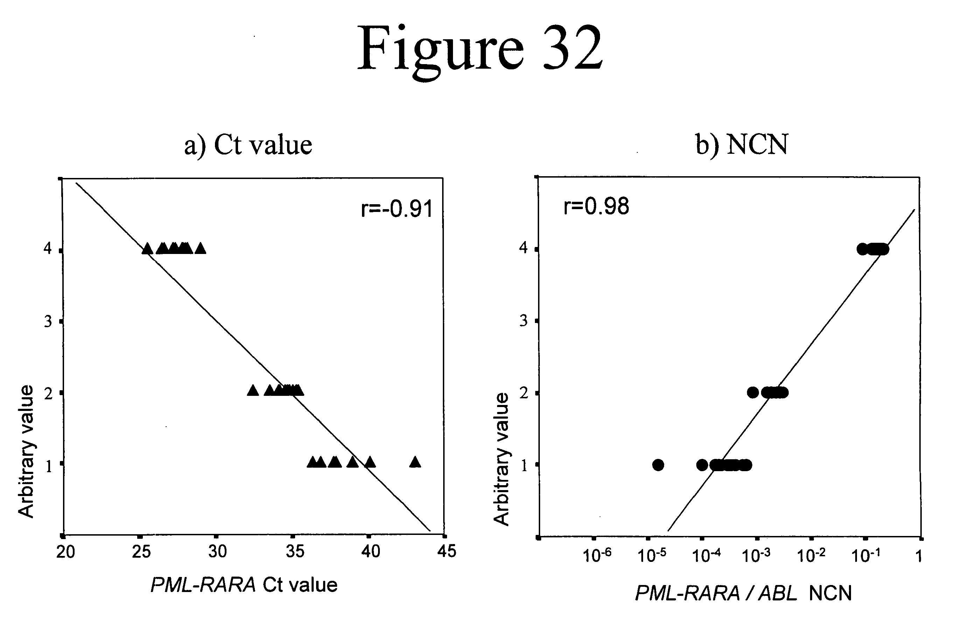

Figure 30 illustrates the influence of the pre-PCR steps on ABL gene transcript quantification of four coded samples tested in 11 randomly assigned laboratories during the balanced randomized assay (phase IIIb, PML-RARA network). a) No significant difference between coded samples using a global linear model, b) Highly significant difference between laboratories for the same samples. Network leader who sent out the coded samples to other laboratories. The 10⁻¹ dilution was excluded from the analysis because it could not be considered as identical to the four others. The 10⁻³ and 10⁻⁴ diluted samples also appear in the figures 31 and 32 ;
Figure 31 illustrates the results per laboratory of the amplification of PML-RARA fusion gene transcript in three coded samples during the balanced randomized assay (phase IIIb). a) Ct value of the FG transcript. b) ABL NCN of the FG transcript of the 3 coded samples. Dilutions were 10⁻¹ (■), 10⁻³ (▲) and 10⁻⁴ (●) dilution of NB-4 RNA into a negative RNA sample. The CG improved greatly the linearity of the results (see also Figure 32). 10⁻³ and 10⁻⁴ diluted samples also appear in the figure 30.
Figure 32 is a schematic diagram of the results obtained during the balanced randomized assay (phase IIIb) within the PML-RARA network. Three coded PML-RARA positive samples: 10⁻¹ (Y-axis = 4), 10⁻³ (Y-axis = 2) and 10⁻⁴ (Y-axis = 1) were analyzed in 11 different laboratories. Results show on the X-axis: a) the Ct value or b) the ratio PML-RARA / ABL of each coded sample according to dilution. These samples are identical to those appearing in the figures 30 and 31. Correlation coefficient r and regression curve calculated from these samples appear in the figure. In order to compare both figures, an identical scale was used for the X axis. The same analysis has been performed for each FG network during phase IIIb for Ct and delta Ct values and CN and NCN. Results appear in the Table 33 ;

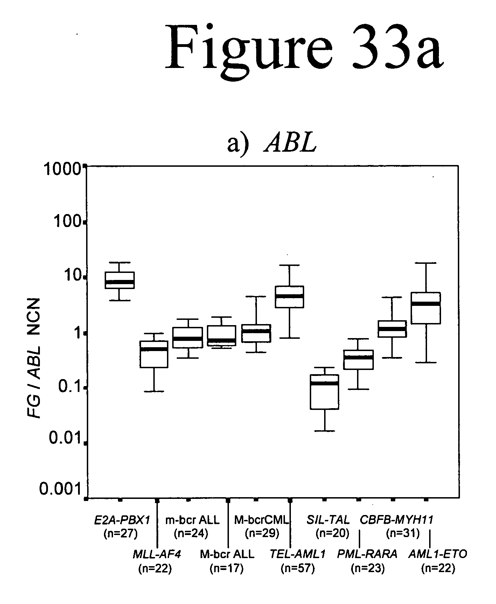

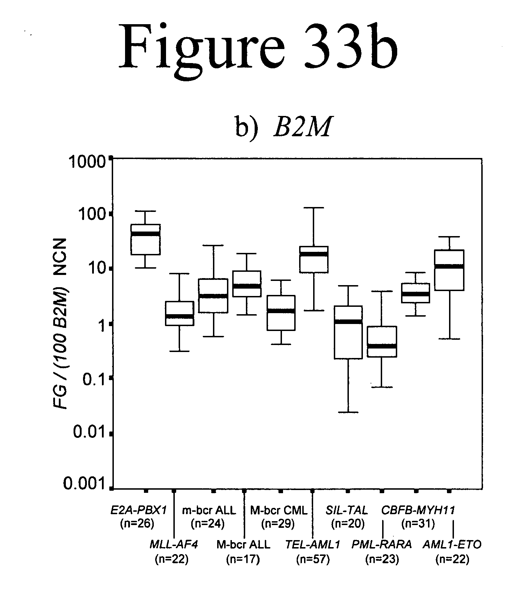

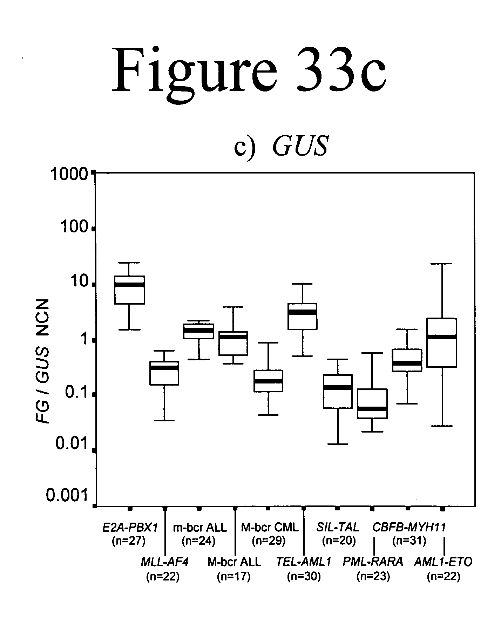

Figure 33 illustrates the comparison of FG transcript expression in leukemic samples at diagnosis. a) Using ABL as control gene, b) Using B2M as control gene, c) Using GUS as control gene. The ratio (NCN) is defined as the ratio between the copy number of the FG transcript per one copy of ABL or GUS gene transcript or 100 copies of B2M gene transcript. The median value corresponds to the black bold line. The box refers to the range defined by the 25th and the 75th percentile. BM and PB samples are pooled per transcript. n: number of patient samples for each particular transcript ;

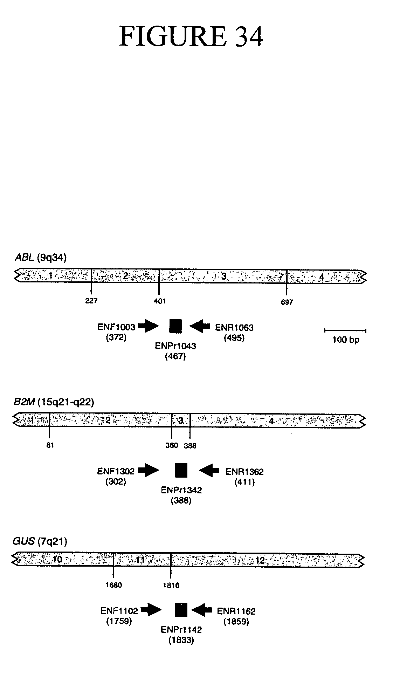

Figure 34 illustrates a schematic diagram of the three selected CG transcripts covered by the EAC RQ-PCR sets. ABL EAC set: ENF1003, ENPr1043, ENR1063, B2M EAC set: ENF1302, ENPr1342, ENR1362 set and GUS EAC set: ENF1102, ENPr1142, ENR1162. The number under the primers and probes refers to their 5' nucleotide position in the gene transcript (see Table 37). ENF = forward primer, ENPr = TaqMan reverse probe, ENR = reverse primer ;

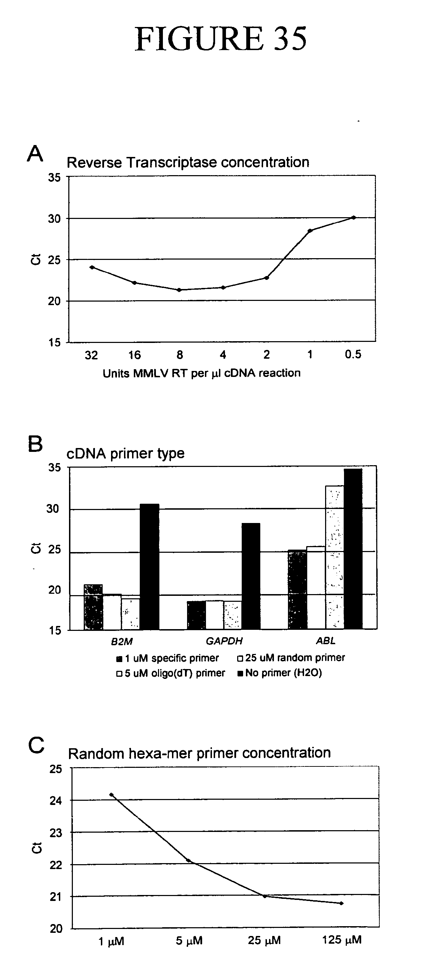

Figure 35 illustrates the optimization of cDNA reaction for RQ-PCR. a) Effect of reverse transcriptase (RT) concentration on RT-RQ-PCR. Seven aliquots of K562 cell line RNA were reverse transcribed to cDNA using 25 mM random primer and two-fold dilutions from 0.5-32 U of MMLV RT per microliter of cDNA reaction. TaqMan PCR was performed using primer sets for B2M, GAPDH, ABL and BCR-ABL on each cDNA and the average Ct values of the four target genes in each cDNA plotted against the RT concentration. b) Comparison of different cDNA primer types in K562 RNA: Mixture of target gene specific primers (1 mM each), random hexa-mer primers (25 mM), an oligo(dT) primer (5 mM) and no primer. c) Effect of primer concentration on cDNA yield. RNA aliquots from the cell lines K562 and REH were reverse transcribed using 5 U of MMLV RT per microliter of cDNA reaction and 1-125 mM random hexa-mer primer. The cDNA's yields were monitored by TaqMan PCR using B2M, GAPDH and ABL. The average Cts of the three target genes in each cDNA were plotted against primer concentration ;

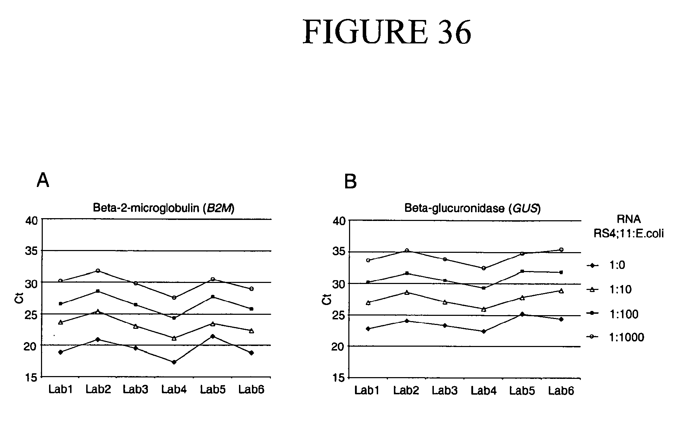

Figure 36 Illustrates the inter-laboratory reproducibility of cDNA synthesis assessed by B2M gene transcript amplification. Ten-fold dilutions of RS4;11 cell line RNA into E.coli RNA were prepared centrally (laboratory #1) and aliquots distributed on dry-ice to the CG group member laboratories. Each laboratory performed cDNA synthesis and TaqMan analysis. Similar results were observed for ABL and GUS gene transcripts ;

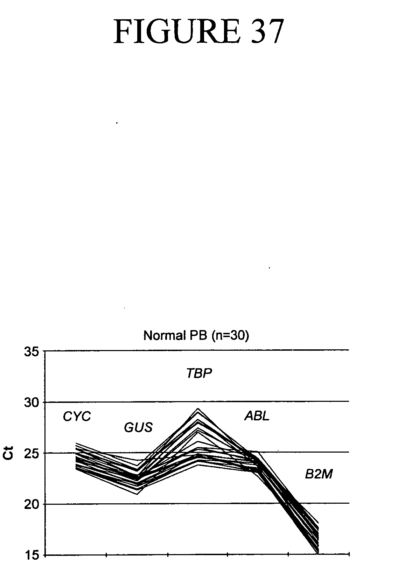

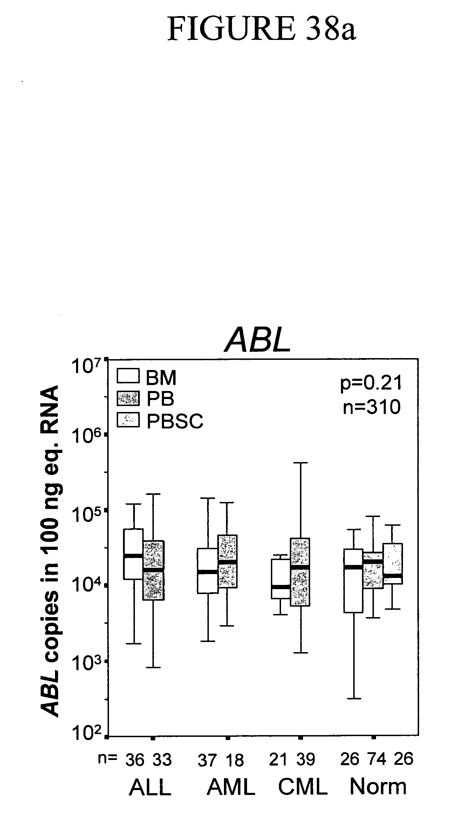

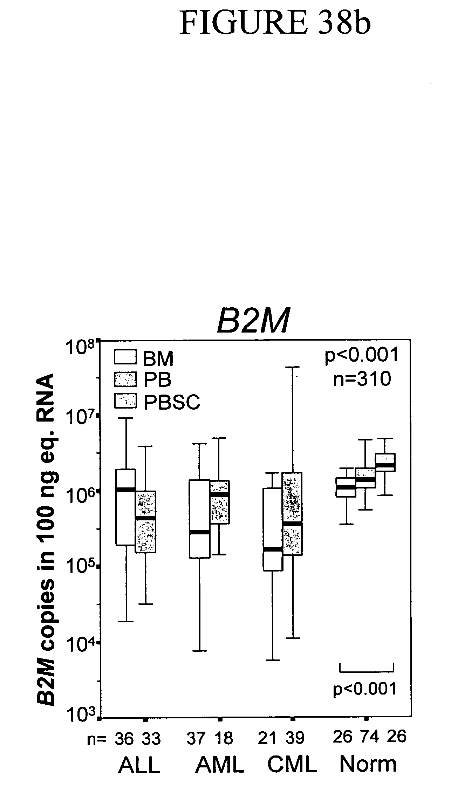

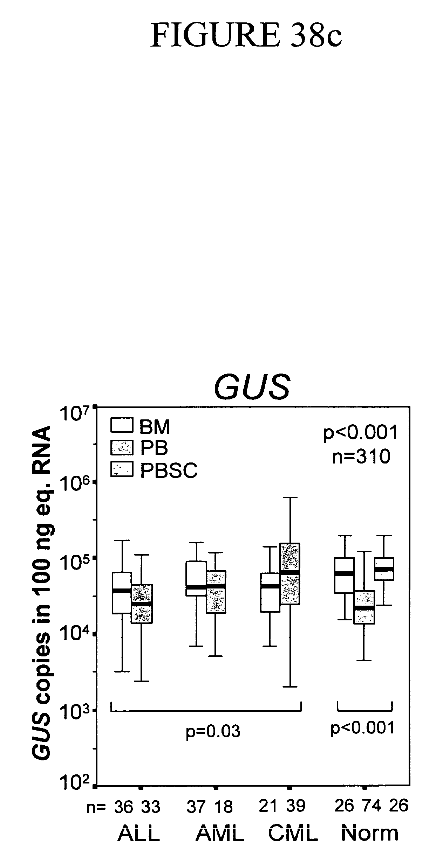

Figure 37 illustrates the variation in CG expression in normal PB samples. PBMNC from 30 normal donors were analyzed with the CYC, GUS, TBP, ABL and B2M gene specific RQ-PCR sets. Each line represents the data from one sample ; Figure 38 illustrates the comparison of the three CG transcripts expression in 310 fresh normal and leukemic samples: a) ABL, b) B2M and c) GUS. For normal samples, analysis performed according to the tissue (BM, PB or PBSC); comparable expression levels were found between PB and PBSC for B2M gene transcript and between BM and PBSC for GUS gene transcript after post-hoc analysis. In the upper right corner, result of the global linear model performed according to the sample type (normal, ALL, AML or CML) and the tissue. The black bold line corresponds to the median value. The box refers to the range defined by the 25th and the 75th percentile. A similar analysis was performed on archived leukemic samples (see results) ;

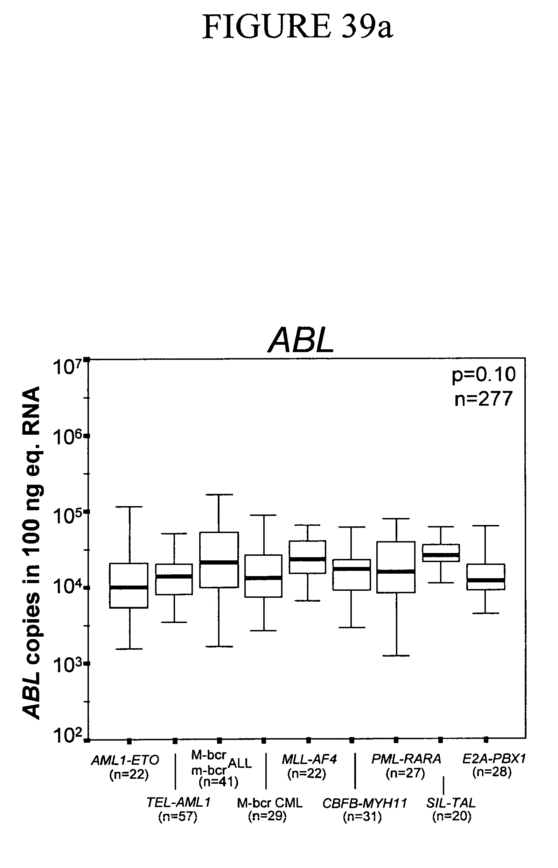

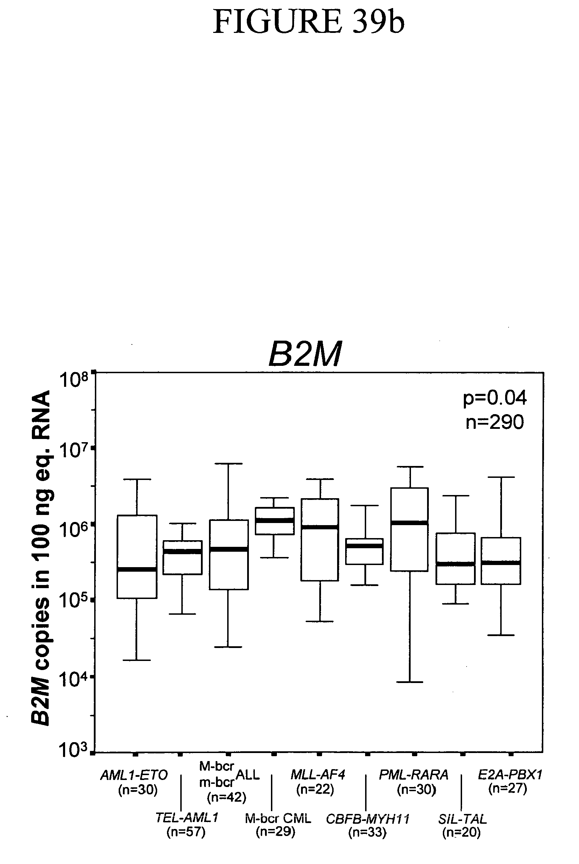

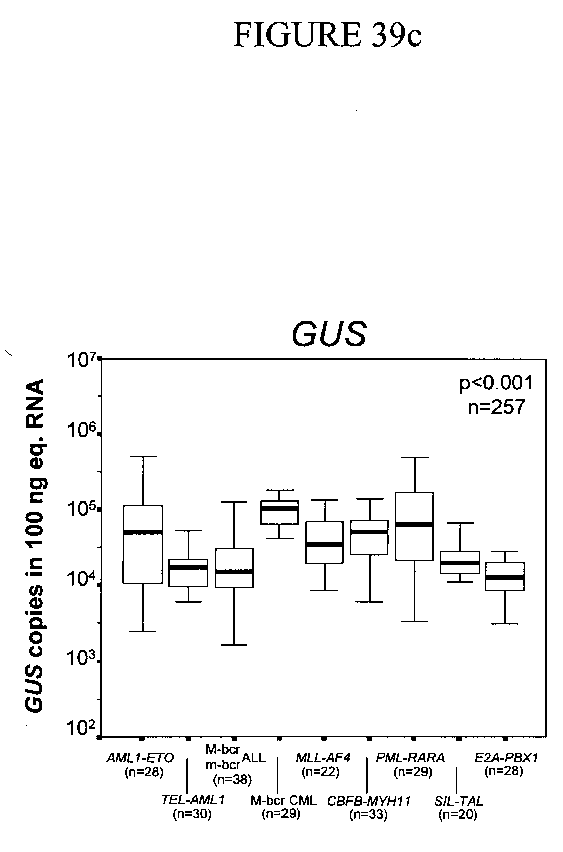

Figure 39 illustrates the comparison of the three CG transcripts expression in archived leukemic samples at diagnosis according to the FG. a) ABL, b) B2M, c) GUS. Data from BM and PB samples were merged per FG transcript. In the upper right corner, result of the global linear model performed according to the FG transcript. The black bold line corresponds to the median value. The box refers to the range defined by the 25th and the 75th percentile. In TEL-AML1 network, only 30 out of 57 samples were tested for GUS transcript expression ;

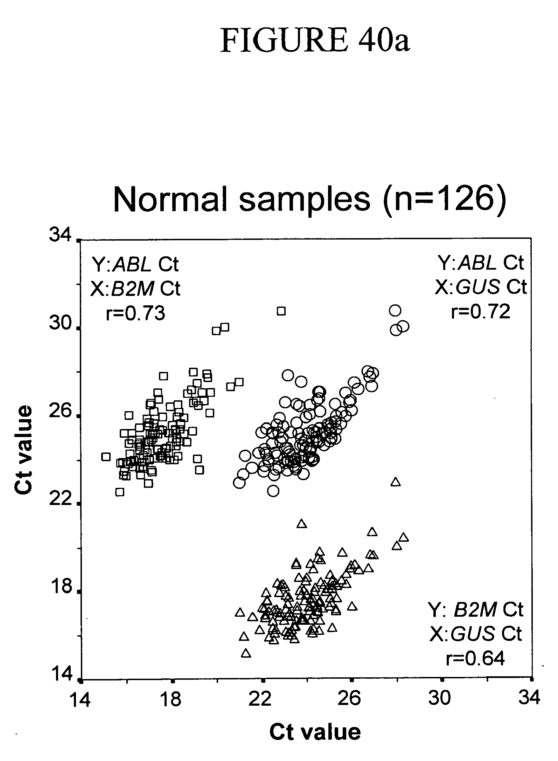

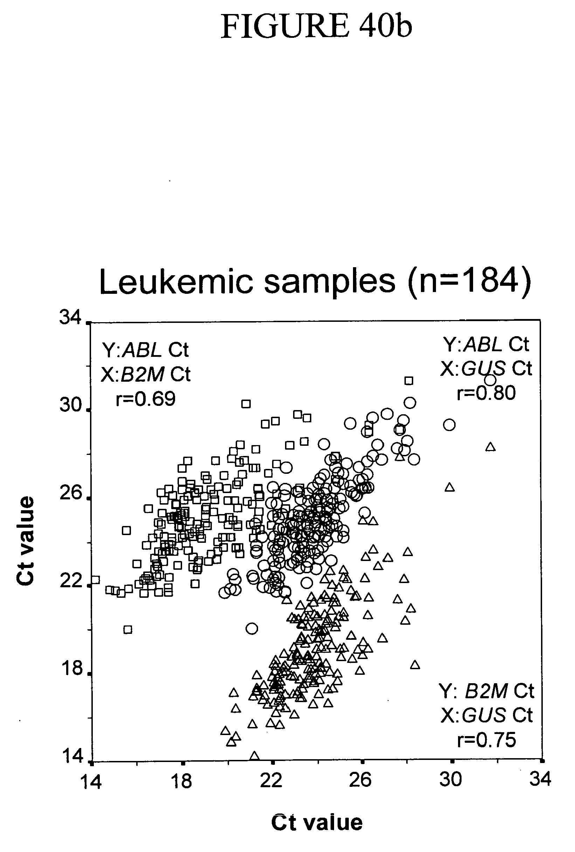

Figure 40 illustrates the correlation between the three EAC selected CGs. a) in fresh normal, b) leukemia samples. Results appeared globally more widespread in leukemia samples compared to normal samples. This observation could be related to the increased number of samples (184 versus 126) and laboratories (14 versus 6) included, and to an effective heterogeneity of CGs expression ; and

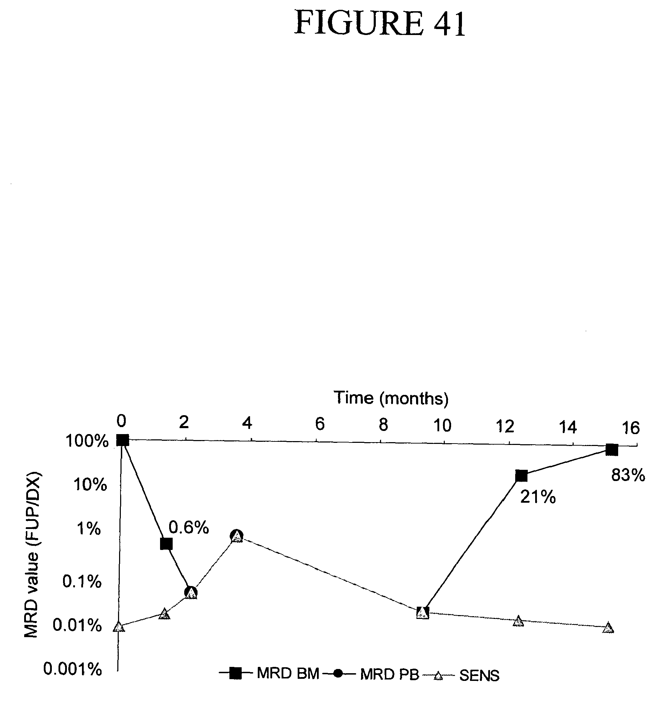

Figure 41 illustrates the longitudinal MRD monitoring of a MLL-AF4 positive patient using EAC MLL-AF4 and ABL RQ-PCR sets. Time 0 was diagnosis, Time 12 was cytological relapse. Value at diagnosis is 1 (100%) and subsequent FG positive results are expressed relatively to the diagnostic sample. Four months after diagnosis, the PB sample was clearly degraded (ABL Ct value: 32.3, ABL CN: 257). Although the result appears in the figure, care should be taken in interpreting this negative result. Calculation was performed according to ΔΔCt and NCN methods found in Table 40. Both methods of calculation lead to similar results both for the MRD value and for sensitivity, consequently only one graphic is shown.

**Table 1 Optimization of the detection of the main fusion gene transcripts in leukemias**

| **Chromosome Aberration** | **RQ-PCR target** | Positive controls | |
|---|---|---|---|
| | | **Cell lines ^{a}** | **Patients ^{b}** |
| t(1;19)(q23;p13) | *E2A-PBX1* | 697 | + |
| t(4;11)(q21;q23) | *MLL-AF4* | RS4;11, MV4-11, ALL-PO | + |
| T(12;21)(p13;q22) | *TEL-AML1* | REH | + |
| t(9;22)(q34;q11) | *BCR-ABL* m-bcr | TOM-1 | + |
| t(9;22)(q34;q 11) | *BCR-ABL* M-bcr | K-562 | + |
| del(1)(p32p32) | *SIL-TAL1* | CEM, RPMI8402^{a} | + |
| T(15;17)(q22;q21) | *PML-RARA* | NB-4 | +^{c} |
| inv(16)(p13q22) | *CBFB-MYH11* | ME-1 | +^{c} |
| t(8;21)(q22;q22) | *AML1-ETO* | KASUMI-1 | + |
| *-* | *Control genes* | HL-60, RS4;11 | + |

| | | | |
|---|---|---|---|
| ^{a} The cell lines used during phase I to IVa are listed; for *SIL-TAL* target, four other cell lines have been tested during phase IVb only (Table 22), ^{b} Analyzed diagnostic samples during phase IVb, ^{c}For *PML-RARA* bcr2, bcr3 and *CBFB-MYH11* type D and type E, patient samples have also been analyzed in the previous phases. | | | |

**Table 2 Phases with work tasks**

| **Period** | **Work task(s)** | **Distributed material** |
|---|---|---|
| **Phase I** | *Training* | RNA dilutions |
| **Phase II** | *Optimization* | Plasmid dilutions RNA dilutions |
| **Phase IIIa** | *Final validation* | Plasmid dilutions RNA & cDNA dilutions |
| | *QC1* | 3 coded RNA samples (negative, 10⁻³,10⁻⁴) |
| **Phase IIIb** | *randomized assay* | Plasmid dilutions |
| | *QC2* | 5 coded RNA samples (2 negative, 10⁻¹,10⁻³, 10⁻⁴) |
| **Phase Iva** | *E. Coli Experiments* | Plasmid dilutions |
| | *QC3* | 5 coded RNA samples (2 negative, pure, 10⁻⁴, random) |
| **Phase IVb** | *Patient testing* | Plasmid dilutions |

**Table 3 Standardized EAC RT and RQ-PCR protocols**

| **1. RT reaction ^{a}** |
|---|
| ■ 1 µg of total RNA in 10 µl of H₂O |
| ■ Incubate at 70°C for 10 min. |
| ■ Cool on ice and add other reagents to final volume of 20 µl |
| Reverse Transcriptase (either MMLV or Superscript I or II): 100 U |
| RT Buffer (according to the RTase used) |
| dNTP: 1 mM |
| DTT: 10 mM |
| Random Hexamers: 25 µM |
| RNAse inhibitor: 20 U |
| ■ Incubate subsequently at: |
| room temperature for 10 min. |
| 42°C for 45 min. |
| 99°C for 3 min. |
| ■ Place the sample at 4°C after RT step |
| ■ Dilute the final cDNA with 30 µl of H₂O |

| **2. RQ-PCR reaction ^{a}** |
|---|
| ■ Final volume: 25 µl |
| ■ 5 µl of final cDNA (100 ng RNA equivalent) |
| ■ Primers: 300 nM each |
| Probe: 200 nM except for the *AMLl-ETO* probe (100 nM) |
| Master Mix: 12.5 µl (1X) |
| ■ Incubate the sample: |
| At 50°C for 2 min |
| At 95°C for 10 min |
| Followed by 50 cycles: |
| 95°C for 15 sec. |
| 60°C for I min. |

| **3. Standardized procedure for data analysis** |
|---|
| ■ Set threshold at 0.1 except for *PML-RARA* (0.05) |
| ■ Set baseline between cycle 3 and 15 except for *B2M*(3-10). |

| |
|---|
| ^{a}All mentioned concentrations are for the final volume of the reaction |

**Table 4 Results observed for selected EAC primer and probe sets**

| **Fusion gene targets (n = 15)** | **RNA dilution (log)** | **Plasmid dilution (copy number)** |
|---|---|---|
| **Sensitivity** | -4 / -5 | 10 molecules |
| **Reproducibility** | -3 / -4 CV(Ct) < 6% | 10 molecules^{a} CV(Ct) < 4%^{b} |

| **Robustness** | | |
|---|---|---|
| **ΔRn** | >1 | >1 |
| Slope | 3.45 ± 0.3 | 3.3 ± 0.3 |

| | | |
|---|---|---|
| EAC criteria are defined in the Material and Methods section. ^{a} 100 molecules only for FR variants (tested only during phase IIIa): *PML-RARA* bcr2 and bcr3, *CBFB-MYH11* Type D and E, b CV of the 10 copies plasmid. CV was below 2.5% for the other dilutions. | | |

**Table 5 Sequences and positions of the E2A-PBX1 primers and probe**

| **EAC code^{a}** | **Primer/probe localisation 5'-3' position (size)** | | **Sequence 5' - 3'** |
|---|---|---|---|
| **ENF101** | *E2A* | 1436-1454 (19) | CCAGCCTCATGCACAACCA |
| **ENP141** | *E2A* | 1481-1502 (22) | CCCTCCCTGACCTGTCTCGGCC |
| **ENR161** | *PBX1* | 409-389 (21) | GGGCTCCTCGGATACTCAAAA |

| | | | |
|---|---|---|---|
| ^{a} ENF = forward primer, ENP = TaqMan probe, the underlined sequence was used as MGB probe, ENR = reverse primer, ^{b} Positions according to accession numbers M31222 (*E2A*) and M86546 (*PBX1*). | | | |

**Table 6 Expression values of the E2A-PBX1 FG transcript in cell line 697 and patients at diagnosis (phase IV)**

| **Samples** | **Ct values** | | | | ***E2A-PBX1* NCN** | | |
|---|---|---|---|---|---|---|---|
| | ***E2A-PBX1*** | ***ABL*** | ***B2M*** | ***GUS*** | ***perABL*** | ***per 100 B2M*** | ***per GUS*** |
| **Cell line** | | | | | | | |
| **697** | 20.6 | 24.3 | 19.4 | 25.4 | 5.0 | 33.9 | 9.0 |

| **Patients** | | | | | | | |
|---|---|---|---|---|---|---|---|
| **BM (n=14)** | 20.7 [18.4-22.6] | 24.4 [22.6-28.2] | 20.2^{a} [16.7-22.5] | 24.9 [22.3-27.7] | 7.6 [2.1-19.5] | 44.6^{a} [10.5-101.0] | 8.9 [1.6-17.8] |
| | | | | | | | |
| **PB (n=13)** | 21.0 [18.0-22.6] | 25.2 [22.9-28.5] | 19.5 [16.1-22.6] | 25.8 [22.8-26.3] | 9.4 [3.8-72.4] | 42.7 [2.2-115.9] | 13.6 [2.5-67.6] |
| **Cor Coef** | | | | | 0.83 | 0.62 | 0.66 |

| | | | | | | | |
|---|---|---|---|---|---|---|---|
| ^{a}one value was not available in one BM sample out of 14 for *B2M.* For patient samples and cell lines, median values [95% range] are indicated. | | | | | | | |

**Table 7 Results of the QC rounds for E2A-PBX1 FG transcript detection (phases III and IV)**

| **QC phase** | **False negativity** | | **False positivity** | |
|---|---|---|---|---|
| | **10⁻³** | **10⁻⁴** | **FG neg. control** | **NAC / NTC** |
| **IIIa** | 0% (0/8) | 0% (0/8) | 0% (0/8) | 0%(0/16) |
| **IIIb** | 0%(0/10) | 0% (0/10) | 0% (0/20) | 0% (0/20)^{b} |
| **Iva** | ND | 0%(0/8)^{a} | 0% (0/16) | 0%(0/16) |
| **All phases** | 0% (0/18) | 0%(0/26) | 0% (0/44) | 0%(0/52) |

| | | | | |
|---|---|---|---|---|
| Results are proportions of false positive or negative samples. 10⁻³ and 10⁻⁴ are RNA dilutions of ACC42 cell line RNA in HL60 RNA. ^{a} 23 out of 24 wells were positive, ^{b} 57 out of 60 wells were negative. | | | | |

**Table 8 Sequences and positions of the MLL-AF4 primers and probe**

| **EAC code^{a}** | **Primer/probe localisation ^{b} 5'-3' position (size)** | | **Sequence 5' - 3'** |
|---|---|---|---|
| **ENF207** | ***MLL*** | 4050-4074 (25) | CCCAAGTATCCCTGTAAAACAAAAA |
| **ENF208** | ***MLL*** | 4186-4208 (23) | GATGGAGTCCACAGGATCAGAGT |
| **ENP242** | ***AF4*** | 1517-1538 (22) | CATGGCCGCCTCCTTTGACAGC |
| **ENR262** | ***AF4*** | 1581-1560 (22) | GAAAGGAAACTTGGATGGCTCA |

| | | | |
|---|---|---|---|
| ^{a} ENF = forward primer, ENP = TaqMan probe, the underlined sequence was used as MGB probe, ENR = reverse primer, ^{b} Positions according to accession numbers L04284 (*MLL*) and L13773 (*AF4*). | | | |

**Table 9 Expression values of the MLL-AF4 FG transcript in cell lines and patients at diagnosis (phase IV)**

| **Samples** | **Ct values** | | | | ***MLL-AF4* NCN values** | | |
|---|---|---|---|---|---|---|---|
| | ***MLL-AF4*** | ***ABL*** | ***B2M*** | ***GUS*** | ***per ABL*** | ***per 100 B2M*** | ***per GUS*** |
| **Cell lines** | | | | | | | |
| **RS4;11** | 2.4.4 | 23.1 | 19.5 | 22.8 | 0.35 | 4.4 | 0.29 |
| **MV4-11** | 25.4 | 23.9 | 20.6 | 24.2 | 0.56 | 9.3 | 0.63 |
| **ALL-PO** | 24.7 | 23.1 | 19.7 | 23.0 | 0.62 | 9.3 | 0.59 |

| **Patients** | | | | | | | |
|---|---|---|---|---|---|---|---|
| **BM (n=14)** | 26.1 [24.7-29.6] | 24.6 [23.1-26.1] | 19.1 [17.2-22.0] | 23.5 [21.3-25.7] | 0.46 [0.09-0.98] | 1.2 [0.07-8.3] | 0.32 [0.04-0.65] |
| | | | | | | | |
| ***PB* (n=8)** | 27.1 [23.8-31.0] | 25.0 [22.9-29.4] | 18.6 [18.9-22.6] | 23.4 [21.6-27.9] | 0.60 [0.16-0.91] | 1.4 [0.32-2.9] | 0.27 [0.09-0.58] |
| **Cor Coef** | | | | | 0.79 | 0.67 | 0.76 |

| | | | | | | | |
|---|---|---|---|---|---|---|---|
| For patient samples and cell lines, median values [95% range] are indicated. | | | | | | | |

**Table 10 Results of the QC rounds for MLL-AF4 FG transcript detection (phases III and IV)**

| QC phase | False negativity | | False positivity | |
|---|---|---|---|---|
| | 10⁻³ | 10⁻⁴ | FG neg. control | NAC / NTC |
| IIIa | 5%(1/21) | 10%(2/21) | 0%(0/21) | 5% (2/42)^{b} |
| IIIb^{a} | 9%(1/11) | 9%(1/11) | 0%(0/22) | 0%(0/22) |
| IVa | 0%(0/23) | 9% (2/23) | 2%(1/46) | 9% (2/23)^{b} |
| All phases | 3.6%(2/55) | 9% (5/55) | 1.1%(1/89) | 4.6%(4/87) |

| | | | | |
|---|---|---|---|---|
| Results are proportions of false positive or negative samples. 10⁻³ and 10⁻⁴ are the RNA dilutions of a positive *MLL-AF4* cell line RNA in a negative RNA (PB MNC). ^{a} Only RS4;11 was tested during phase IIIb, ^{b} For NAC and NTC samples, only one well out of 2 was positive in each case. | | | | |

**Table 11 Sequences and positions of the TEL-AML1 primers and probe**

| **EAC code^{a}** | **Primer/probe localisation^{b} 5'-3' position (size)** | | **Sequence 5' - 3'** |
|---|---|---|---|
| **ENF301** | *TEL* | 984-1002 (19) | CTCTGTCTCCCCGCCTGAA |
| **ENPr341** | *TEL* | 1024-1005 (20) | TCCCAATGGGCATGGCGTGC |
| **ENR361** | *AML1* | 557-542 (16) | CGGCTCGTGCTGGCAT |

| | | | |
|---|---|---|---|
| ^{a} ENF = forward primer, ENPr = TaqMan reverse probe (in order to select the C-rich strand), the underlined sequence was used as MGB probe, ENR = reverse primer, ^{b} Positions according to accession numbers U11732 *(TEL)* and D43969 (*AML1*). | | | |

**Table 12 Expression values of the TEL-AML1 FG transcript in cell line and patients at diagnosis (phase IV)**

| **Samples** | **Ct values** | | | | ***TEL-AML1* NCN ^{a}** | | |
|---|---|---|---|---|---|---|---|
| | ***TEL-AML1*** | ***ABL*** | ***B2M*** | ***GUS^{b}*** | ***per ABL*** | ***per 100 B2M*** | ***per GUS^{b}*** |
| **Cell line** | | | | | | | |
| **REH** | 2.1.7 | 23.4 | 18.7 | 23.5 | 3.2 | 27 | 2.5 |

| **Patients** | | | | | | | |
|---|---|---|---|---|---|---|---|
| **BM (n=30)** | 22.6 [20.0-27.9] | 25.5 [23.5-27.7] | 19.2 [18.0-23.0] | 25.2 [23.7-27.3] | 4.45 [0.46-32.7] | 23 [0.63-132] | 3.19 [0.27-10.3] |
| | | | | | | | |
| **PB (n=27)** | 2.3.9 [21.6-27.9] | 25.4 [24.4-28.3] | 19.1 [17.9-22.7] | 25.2 [24.5-26.3] | 4.71 [0.20-15.2] | 12 [0.39-99] | 2.63 [0.50-9.0] |
| **Cor Coef** | | | | | 0.66 | 0.42 | 0.66 |

| | | | | | | | |
|---|---|---|---|---|---|---|---|
| ^{a} Corrected according to the blast percentage in the sample, ^{b} Only 13 peripheral blood and 17 bone marrow samples were tested for *GUS.* For patient samples and cell lines, median values [95% range] are indicated. | | | | | | | |

**Table 13 Results of the QC rounds for TEL-AML1 FG transcript detection (phases III and IV)**

| Phase | False negativity | | | False positivity | |
|---|---|---|---|---|---|
| | 10⁻³ | 10⁻⁴ | 5.10⁻⁵ | FG neg. control | NAC / NTC |
| IIIa | 0% (0/7) | 0% (0/7) | ND | 0% (0/14) | 0% (0/14) |
| IIIb | 0% (0/11) | 0% (0/11) | ND | 9% (2/22) | 0% (0/22) |
| IVa | ND | 0% (0/7) | 0% (0/7) | 7% (1/14) | 0% (0/7) |
| All phases | 0% (0/18) | 0% (0/25) | 0% (0/7) | 6% (3/50) | 0% (0/43) |

| | | | | | |
|---|---|---|---|---|---|
| Results are proportions of false positive or negative samples. 10⁻³, 10⁻⁴, and 5.10⁻⁵ are the RNA dilutions of REH cell line RNA in a negative RNA. | | | | | |

**Table 14 Sequences and positions of the BCR-ABL m-bcr primers and probe**

| **EAC code^{a}** | **Primer/probe localisation^{b} 5'-3' position (size)** | | **Sequence 5' - 3'** |
|---|---|---|---|
| **ENF402** | *BCR* | 1727-1744 (18) | CTGGCCCAACGATGGCGA |
| **ENP541** | *ABL* | 230-254 (25) | CCCTTCAGCGGCCAGTAGCATCTGA |
| **ENR561** | *ABL* | 277-257 (21) | CACTCAGACCCTGAGGCTCAA |

| | | | |
|---|---|---|---|
| ^{a} ENF = forward primer, ENP = TaqMan probe, the underlined sequence was used as MGB probe, ENR = reverse primer, ^{b} Positions according to accession numbers X02596 (BCR) and X16416 (*ABL*). | | | |

**Table 15 Expression values of the BCR-ABL m-bcr FG transcript in cell line and ALL patients at diagnosis (phase IV)**

| **Samples** | **Ct values** | | | | ***BCR-ABL* NCN values** | | |
|---|---|---|---|---|---|---|---|
| | ***BCR-ABL* m-bcr** | ***ABL^{a}*** | ***B2M*** | ***GUS*** | ***per ABL^{a}*** | ***per 100 B2M*** | ***Per GUS*** |
| **Cell line** | | | | | | | |
| **TOM-1** | 22.8 | 21.8 | 18.1 | 23.8 | 0.44 | 4.69 | 1.59 |

| **Patients** | | | | | | | |
|---|---|---|---|---|---|---|---|
| **BM (n=17)** | 24.7 [21.3-27.1] | 24.5 [21.7-27.1] | 20.4 [16.6-22.1] | 25.8 [22.1-28.9] | 0.80 [0.35-1.38] | 2.70 [0.58-26.3] | 1.48 [0.24-16.6] |
| | | | | | | | |
| **PB (n=7)** | 23.6 [21.1-29.1] | 22.7 [21.0-27.0] | 18.2 [15.3-19.5] | 24.5 [21.5-25.5] | 0.68 [0.39-1.81] | 3.36 [0.03-13.75] | 1.63 [0.18-9.34] |
| **Cor Coef** | | | | | 0.96 | 0.82 | 0.80 |

| | | | | | | | |
|---|---|---|---|---|---|---|---|
| ^{a} The *ABL* EAC RQ-PCR set can amplify both *BCR-ABL* and *ABL* transcripts. Thus the values are impaired by the presence of the FG. For patient samples and cell lines, median values [95% range] are indicated. | | | | | | | |

**Table 16 Results of the QC rounds for BCR-ABL m-bcr FG transcript detection (phases III and IV)**

| QC phase | False negativity | | False positivity | |
|---|---|---|---|---|
| | 10⁻³ | 10⁻⁴ | FG neg. control | NAC / NTC |
| IIIa | 0% (0/24) | 0% (0/24) | 13% (3/24) | 8% (5/64) |
| IIIb | 0% (0/30) | 0% (0/30) | 5% (3/60) | 5% (2/42) |
| IVa | ND | 14% (3/21) | 0% (0/42) | 0% (0/14) |
| All phases | 0% (0/54) | 4% (3/75) | 4.8% (6/126) | 5.8% (7/120) |

| | | | | |
|---|---|---|---|---|
| Results are proportions of false positive or negative samples. 10⁻³ and 10⁻⁴ are the RNA dilutions of TOM-1 *BCR-ABL* m-bcr positive cell line in HL-60 RNA. | | | | |

**Table 17 Sequences and positions of the BCR-ABL M-bcr primers and probe**

| **EAC code^{a}** | **Primer/probe localisation^{b} 5'-3' position (size)** | | **Sequence 5' - 3'^{c}** |
|---|---|---|---|
| **ENF501** | *BCR* | 3173-3193 (21) | TCCGCTGACCATCAAYAAGGA |
| **ENP541** | *ABL* | 230-254 (25) | CCCTTCAGCGGCCAGTAGCATCTGA |
| **ENR561** | *ABL* | 277-257 (21) | CACTCAGACCCTGAGGCTCAA |

| | | | |
|---|---|---|---|
| ^{a}ENF = forward primer, ENP = TaqMan probe, the underlined sequence was used as MGB probe,ENR = reverse primer, ^{b} Positions according to accession numbers X02596 (BCR) and X16416 *(ABL).* ^{c} Y (Cytidine or Thymidine) appears on the *BCR* primer according to the polymorphism recently described on the *BCR* gene. ⁵² | | | |

**Table 18 Expression values of the BCR-ABL M-bcr FG transcript in cell line and CML patients at diagnosis (phase IV)**

| **Samples** | **Ct values** | | | | ***BCR-ABL* NCN** | | |
|---|---|---|---|---|---|---|---|
| | ***BCR-ABL M-bcr*** | ***ABL ^{a}*** | ***B2M*** | ***GUS*** | ***per ABL ^{a}*** | ***per 100 B2M*** | ***per GUS*** |
| **Cell line** | | | | | | | |
| **K-562** | 20.5 | 20.7^{b} | 20.6 | 21.5 | 1.0 | 155 ^{b} | 0.79 |

| **Patients** | | | | | | | |
|---|---|---|---|---|---|---|---|
| **BM (n=15)** | 25.1 [21.5-27.0] | 25.2 [20.7-26.8] | 18.1 [15.9-21.5] | 22.4 [21.6-27.9] | 0.86 [0.44-3.0] | 0.95 [0.43-5.3] | 0.12 [0.04-0.87] |
| | | | | | | | |
| **PB (n=14)** | 23.1 [21.9-25.8] | 23.7 [22.6-26.7] | 17.6 [16.8-21-5] | 21.7 [20.7-24.8] | 1.17 [0.48-4.4] | 2.8 [0.56-6.2] | 0.22 [0.08-0.41] |
| **Cor Coef** | | | | | 0.81 | 0.63 | 0.76 |

| | | | | | | | |
|---|---|---|---|---|---|---|---|
| ^{a} The *ABL* EAC RQ-PCR set can amplify both *BCR-ABL* and *ABL* transcripts. Thus the values are impaired by the presence of the FG. We found values up to 3.0 in BM and up to 4.4 in PB samples of CML patients at diagnosis. These unexpected results were obtained with plasmid standard curve and without standard. ^{b} K-562 cell line was tested on two independent cultures to confirm the results. For patient samples and cell lines, median values [95% range] are indicated. | | | | | | | |

**Table 19 Expression values of the BCR-ABL M-bcr FG transcript in ALL patients at diagnosis (phase IV)**

| **Samples** | **Ct values** | | | | ***BCR-ABL* NCN** | | |
|---|---|---|---|---|---|---|---|
| | ***BCR-ABL M-bcr*** | **ABL^{a}** | **B2M** | **GUS** | ***per ABL^{a}*** | ***per 100 B2M*** | ***per GUS*** |
| **Patients** | | | | | | | |
| **BM and PB^{a} (n=17)** | 24.1 [21.5-29.9] | 24.0 [21.6-26.4] | 19.0 [16.3-20.5] | 24.8 [20.8-27.1] | 0.71 [0.13-1.93] | 4.90 [0.14-19.5] | 1.15 [0.03-3.89] |
| **Cor Coef** | | | | | 0.90 | 0.56 | 0.60 |

| | | | | | | | |
|---|---|---|---|---|---|---|---|
| ^{a} The *ABL* EAC RQ-PCR set can amplify both *BCR-ABL* and *ABL* transcripts. Thus the values are impaired by the presence of the FG. For patient samples, median values [95% range] are indicated. | | | | | | | |

**Table 20 Results of the QC rounds for BCR-ABL M-bcr FG transcript detection (phases III and IV)**

| QC phase | False negativity | | False positivity | |
|---|---|---|---|---|
| | 10⁻³ | 10⁻⁴ | FG neg. control | NAC / NTC |
| IIIa | 0% (0/11) | 9%(1/11) | 9%(1/11) | 4%(5/120) |
| IIIb | 0%(0/11) | 9%(1/11) | 5.5% (1/22) | 5%(7/132)^{a} |
| IVa | 0% (0/11) | 0% (0/11) | 18% (4/22) | 2% (2/88) |
| All phases | 0% (0/33) | 6.1% (2/33) | 10.9% (6/55) | 4.1% (14/340) |

| | | | | |
|---|---|---|---|---|
| Results are proportions of false positive or negative samples. 10⁻³ and 10⁻⁴ are the RNA dilutions of K-562 cell line RNA in a negative RNA. ^{a} One lab had all their NAC+NTC contaminated thus contributing to 50% of false positive results. | | | | |

**Table 21 Sequences and positions of the SIL-TAL1 primers and probe**

| **EAC code^{a}** | **Primer/probe localisation^{b} 5'-3' position (size)** | | **Sequence 5' - 3'** |
|---|---|---|---|
| **ENF601** | *SIL* | 85-103 (19) | CGCTCCTACCCTGCAAACA |
| **ENP641** | *SIL* | 105-126 (22) | ACCTCAGCTCCGCGGAAGTTGC |
| **ENR664** | *TAL1* | 148-130 (19) | CCGAGGAAGAGGATGCACA |

| | | | |
|---|---|---|---|
| ^{a} ENF = forward primer, ENP = TaqMan probe, the underlined sequence was used as MGB probe, ENR = reverse primer, ^{b} Positions according to accession numbers M74558 (*SIL*) and S53245 (*TAL1*). | | | |

**Table 22 Expression values of the SIL-TAL1 FG transcript in cell lines and patients at diagnosis (phase IV)**

| **Samples** | **Ct values** | | | | ***SIL-TALI* NCN^{a}** | | |
|---|---|---|---|---|---|---|---|
| | ***SIL-TALI*** | ***ABL*** | ***B2M*** | ***GUS*** | ***perABL*** | ***per 100 B2M*** | ***per GUS*** |
| **Cell lines ^{b}** | | | | | | | |
| **CEM** | 26.1 | 23.3 | 20.6 | 22.9 | 0.12 | 1.38 | 0.07 |
| **ALL1** | 25.4 | 21.4 | 16.9 | 21.2 | 0.11 | 0.79 | 0.09 |
| **Molt-15** | 28.3 | 24.2 | 18.0 | 23.2 | 0.10 | 0.27 | 0.06 |
| **Molt-16** | 26.0 | 21.7 | 16.6 | 22.1 | 0.08 | 0.45 | 0.11 |
| **HSB-2** | 25.5 | 24.4 | 19.5 | 24.2 | 0.41 | 3.47 | 0.44 |
| **PF382** | 26.1 | 21.5 | 16.7 | 22.2 | 0.07 | 0.47 | 0.11 |

| **Patients** | | | | | | | |
|---|---|---|---|---|---|---|---|
| **BM (n=10)** | 27.1 [25.2-29.4] | 24.3 [22.2-25.0] | 19.7 [17.4-21.1] | 24.5 [22.9-25.3] | 0.12 [0.02-0.23] | 1.24 [0.03-4.86] | 0.15 [0.02-0.44] |
| **PB (n=10)** | 28.4 [26.5-29.4] | 24.3 [23.6-25.9] | 19.3 [17.1-20.8] | 24.7 [23.8-25.2] | 0.09 [0.02-0.21] | 0.86 [0.05-3.28] | 0.12 [0.01-0.26] |
| **Cor Coef** | | | | | 0.68 | 0.19 ^{c} | 0.57 |

| | | | | | | | |
|---|---|---|---|---|---|---|---|
| ^{a} Corrected according to the blast percentage in the sample, ^{b} The CEM cell line was analyzed in duplicate in 8 laboratories, whereas the other cell lines were analysed in duplicate in single laboratories, ^{c} No significant correlation. For patient samples and cell lines, median values [95% range] are indicated. | | | | | | | |

**Table 23 Results of the QC rounds for SIL-TAL1 FG transcript detection (phases III and IV)**

| QC phase | False negativity | | False positivity | |
|---|---|---|---|---|
| | 10⁻³ | 10⁻⁴ | FG neg. control | NAC / NTC |
| IIIa | 14% (1/7)^{a} | 43% (3/7)^{a} | 0% (0/8) | 0% (0/64) |
| IIIb | 0% (0/10)^{a} | 0% (0/10)^{a} | 5% (1/22) | 0% (0/66) |
| Iva | 0% (0/8) | 0% (0/8) | 6% (1/16) | 0% (0/32) |
| All phases | 4% (1/25) | 12% (3/25) | 4.3% (2/46) | 0% (0/162) |

| | | | | |
|---|---|---|---|---|
| Results are proportions of false positive or negative samples. 10⁻³ and 10⁻⁴ are the RNA dilutions of a positive *SIL-TAL1* cell line (CEM or Molt-15) in a negative RNA (HL-60 or PB MNC) sample. NEG: negative RNA (HL-60 mRNA, U937 mRNA, or PB MNC RNA). ^{a} One sample was excluded due to *ABL* Ct values >29. | | | | |

**Table 24 Sequences and positions of the PML-RARA primers and probe**

| **EAC code^{a}** | **Primer/probe localisation^{b} 5'-3' position (size)** | | **Sequence 5' - 3'** |
|---|---|---|---|
| **ENF903** | *PML* | 1690-1708 (19) | TCTTCCTGCCCAACAGCAA |
| **ENF906** | *PML* | 1642-1660 (19) | ACCTGGATGGACCGCCTAG |
| **ENF905** | *PML* | 1198-1216 (19) | CCGATGGCTTCGACGAGTT |
| **ENP942** | *RARA* | 439-458 (20) | AGTGCCCAGCCCTCCCTCGC |
| **ENR962** | *RARA* | 485-465 (21) | GCTTGTAGATGCGGGGTAGAG |

| | | | |
|---|---|---|---|
| ^{a} ENF = forward primer, ENP = TaqMan probe, the underlined sequence was used as MGB probe, ENR = reverse primer, ^{b} Positions according to accession numbers M73778 (*PML*) and X06538 (*RARA*). | | | |

**Table 25 Expression values of the PML-RARA FG transcript in NB-4 cell line and bcr1 patients at diagnosis (phase IV)**

| **Samples** | **Ct values** | | | | ***PML-RARA* NCN** | | |
|---|---|---|---|---|---|---|---|
| | ***PML-RARA*** | ***ABL*** | ***B2M*** | ***GUS*** | ***perABL*** | ***per 100 B2M*** | ***per GUS*** |
| **Cell line** | | | | | | | |
| **NB-4** | 24.7 | 23.7 | 17.1 | 22.0 | 0.2 | 0.4 | 0.1 |

| **Patients** | | | | | | | |
|---|---|---|---|---|---|---|---|
| **BM (n=14)** | 25.6 [23.1-27.5] | 24.5 [21.7-28.5] | 17.7 [16.0-24.9] | 22.2 [20.3-26.7] | 0.30 [0.09-1.82] | 0.72 [0.19-3.18] | 0.08 [0.02-0.56] |
| | | | | | | | |
| **PB (n=9)** | 25.7 [23.7-29.4] | 24.6 [22.0-27.4] | 17.9 [16.5-20.0] | 23.0 [20.6-25.4] | 0.36 [0.11-0.78] | 0.26 [0.07-0.60] | 0.05 [0.02-0.13] |
| **Cor Coef** | | | | | 0.80 | 0.54 | 0.73 |

| | | | | | | | |
|---|---|---|---|---|---|---|---|
| For patient samples and cell lines, median values [95% range] are indicated. NB-4 cell line was analyzed in triplicate (FG) or duplicate (CG) in 2 laboratories. | | | | | | | |

**Table 26 Results of the QC rounds for PML-RARA bcr1 FG transcript detection (phases III and IV)**

| QC phase | False negativity | | False positivity | |
|---|---|---|---|---|
| | 10⁻³ | 10⁻⁴ | FG neg. control | NAC / NTC |
| IIIa | 0% (0/7) | 0% (0/7) | 29% (2/7) | 7.1% (2/28) |
| IIIb | 0% (0/11) | 0% (0/11) | 4.5% (1/22) | 0% (0/44) |
| IVa | 0% (0/11) | 0% (0/10) | 13% (2/16) | 11% (3/28) |
| All phases | 0% (0/29) | 0% (0/28) | 11%(5/45) | 5% (5/100) |

| | | | | |
|---|---|---|---|---|
| Results are proportions of false positive or negative samples. 10⁻³ and 10⁻⁴ are the dilutions of NB-4 cell line RNA in a negative RNA. | | | | |

**Table 27 Sequences and positions of the CBFB-MYH11 primers and probe**

| **EAC code ^{a}** | **Primer/probe localisation^{b} 5'-3' position (size)** | | **Sequence 5' - 3'** |
|---|---|---|---|
| **ENF803** | *CBFB* | 389-410 (22) | CATTAGCACAACAGGCCTTTGA |
| **ENPr843** | *CBFB* | 434-413 (22) | TCGCGTGTCCTTCTCCGAGCCT |
| **ENR862** | *MYH11* | 1952-1936 (17) | AGGGCCCGCTTGGACTT |
| **ENR863** | *MYH11* | 1237-1217 (21) | CCTCGTTAAGCATCCCTGTGA |
| **ENR865** | *MYH11* | 1038-1016 (23) | CTCTTTCTCCAGCGTCTGCTTAT |

| | | | |
|---|---|---|---|
| ^{a} ENF = forward primer, ENPr = TaqMan reverse probe (in order to select the C-rich strand), the underlined sequence was used as MGB probe, ENR = reverse primer, ^{b} Positions according to accession numbers L20298 (*CBFB*) and D10667 (*MYH11*). | | | |

**Table 28 Expression values of the CBFB-MYH11 FG transcript in cell line and patients at diagnosis (phase IV)**

| **Samples** | **Ct values** | | | | ***CBFB-MYH11* NCN values** | | |
|---|---|---|---|---|---|---|---|
| | ***CBFB-MYH11*** | ***ABL*** | ***B2M*** | ***GUS*** | ***per ABL*** | ***per 100 B2M*** | ***per GUS*** |
| **Cell line** | | | | | | | |
| **ME-1** | 24.7 | 25.1 | 19.4 | 19.4 | 0.90 | 4.64 | 0.40 |

| **Patients** | | | | | | | |
|---|---|---|---|---|---|---|---|
| **Type A^{a} (n=24)** | 24.8 [22.2-27.8] | 25.0 [22.5-27.7] | 18.8 [16.3-24.0] | 23.3 [21.6-26.8] | 1.35 [0.13-14.8] | 3.98 [1.45-447] | 0.42 [0.07-4.57] |
| | | | | | | | |
| **Type D^{b} (n=3)** | 24.4 [24.0-25.7] | 24.2 [24.2-26.0] | 19.2 [18.6-19.8] | 24.2 [22.4-24.6] | 1.0 [0.78-1.07] | 4.0 [3.0-4.7] | 0.32 [0.25-0.55] |
| | | | | | | | |
| **Type E ^{c} (n=4)** | 26.0 [25.2-28.4] | 25.6 [23.7-27.0] | 19.2 [18.2-19.5] | 24.2 [23.5-25.7] | 0.88 [0.62-1.55] | 2.1 [0.72-2.57] | 0.35 [0.21-0.37] |
| **Cor Coef** | | | | | 0.76 | 0.65 | 0.76 |

| | | | | | | | |
|---|---|---|---|---|---|---|---|
| ^{a} Set ENF803 - ENR862 - ENP843, ^{b} ENF803 - ENR863 -ENP843, ^{c}Set ENF803 - ENR865 - ENP843. Due to few PB samples (n=4), data have been analyzed according to the type of transcript. For patient samples and cell lines, median values [95% range] are indicated. | | | | | | | |

**Table 29 Results of the QC rounds for CBFB-MYH11 FG transcript detection (phases III and IV)**

| QC phase | False negativity | | False positivity | |
|---|---|---|---|---|
| | 10⁻³ | 10⁻⁴ | FG neg. control | NAC / NTC |
| IIIa | 0% (0/14) | 7% (1/14) | 0% (0/7) | 0% (0/28) |
| IIIb | 0% (0/11) | 18% (2/11) | 0% (0/22) | 0% (0/44) |
| IVa | ND | 13% (1/8) | 6.3% (1/16) | 0% (0/32) |
| All phases | 0% (0/25) | 12% (4/33) | 2.2% (2/45) | 0% (0/104) |

| | | | | |
|---|---|---|---|---|
| Results are proportions of false positive or negative samples. 10⁻³ and 10⁻⁴ are the dilutions of ME-1 cell line RNA in a negative RNA. | | | | |

**Table 30 Sequences and positions of the AML1-ETO primers and probe**

| **EAC code^{a}** | **Primer/probe localization^{b} 5'-3' position (size)** | | **Sequence 5' - 3'** |
|---|---|---|---|
| **ENF701** | *AML1* | 1005-1026 (22) | CACCTACCACAGAGCCATCAAA |
| **ENP747** | *AML1* | 1049-295 (30) | AACCTCGAAATCGTACTGAGAAGCACTCCA^{c} |
| **ENR761** | *ETO* | 318-297 (22) | ATCCACAGGTGAGTCTGGCATT |

| | | | |
|---|---|---|---|
| ^{a} ENF = forward primer, ENP = TaqMan probe, the underlined sequence was used as MGB probe, ENR = reverse primer, ^{b} Positions according to accession numbers D43969 (*AML1*) and D14289 (*ETO*), ^{c}': location of the breakpoint. | | | |

**Table 31 Expression values of the AML1-ETO FG transcript in cell line and patients at diagnosis (phase IV)**

| **Samples** | **Ct values** | | | | ***AML1-ETO* NCN** | | |
|---|---|---|---|---|---|---|---|
| | ***AMLI-ETO*** | ***ABL*** | ***B2M*** | ***GUS*** | ***per ABL*** | ***per 100 B2M*** | ***per GUS*** |
| **Cell line** | | | | | | | |
| **KASUMI-1** | 22.2 | 26.2 | 23.3 | 24.1 | 5.46 | 80 | 1.42 |

| **Patients** | | | | | | | |
|---|---|---|---|---|---|---|---|
| **BM (n=10)** | 24.9 [20.1-28.0] | 26.4 [20.7-27.7] | 18.8 [16.8-21.9] | 23.8 [20.8-26.7] | 2.1 [0.29-5.3] | 10.0 [0.5-38.9] | 1.32 [0.03-25.1] |
| | | | | | | | |
| **PB (n=12)** | 23.9 [18.6-27.6] | 26.1 [22.5-28.6] | 19.6 [16.5-23.6] | 23.6 [21.4-27.4] | 4.2 [1.1-20.0] | 12.0 [2.5-33.1] | 1.1 [0.01-159] |
| **Cor Coef** | | | | | 0.81 | 0.80 | 0.44 |

| | | | | | | | |
|---|---|---|---|---|---|---|---|
| For patient samples and cell lines, median values [95% range] are indicated. | | | | | | | |

**Table 32 Results of the QC rounds for AML1-ETO FG transcript detection (phases III and IV)**

| QC phase | False negativity | | False positivity | |
|---|---|---|---|---|
| | 10⁻³ | 10⁻⁴ | FG neg. control | NAC / NTC |
| IIIa | 0% (0/21) | 0% (0/21) | 13 % (1/8)^{a} | 8% (3/32) |
| IIIb | 0% (0/11) | 0% (0/11) | 14% (3/22) ^{b} | 0% (0/44) |
| Iva | 0% (0/24) | 0% (0/24) | 25% (5/16)^{c} | 8% (3/32) |
| All phases | 0% (0/56) | 0% (0/56) | 20% (9/46) | 5.6% (6/108) |

| | | | | |
|---|---|---|---|---|
| Results are proportions of false positive or negative samples. 10⁻³ and 10⁻⁴ are the RNA dilutions of KASUMI-1 cell line RNA in HL-60 RNA. ^{a} False positivity was due to 3 false positive replicates in a single lab. ^{b} False positivity was due to 3 single positive wells in 3 different laboratories. ^{c} Two labs out of twelve make up 4/5 false positive samples, thus contributing to 80% of false positivity. | | | | |

**Table 33 Correlation coefficients obtained during the balanced randomized assay on diluted samples (phase IIIb)**

| **Fusion transcript** Type | ***E2A-PBX1*** | ***MLL-AF4*** Ex10-Ex4 | ***TEL-AML1*** | ***BCR-ABL*** m-bcr | ***BCR-ABL*** M-bcr | ***SIL-TAL1*** | ***PML-RARA*** bcr1 | ***CBFB-MYH11*** Type A | ***AMLI-ETO*** |
|---|---|---|---|---|---|---|---|---|---|
| **FG Ct** | **-0.99** | -0.78 | -0.97 | -0.98 | -0.85 | -0.84 | -0.91 | -0.86 | -0.91 |
| **FG CN** | **0.99** | 0.72 | 0.95 | 0.98 | 0.80 | 0.80 | 0.86 | 0.82 | 0.91 |
| **Delta Ct** | -0.96 | -0.74 | **-0.98** | **-0.99** | -0.92 | -0.86 | -0.90 | -0.88 | **-0.94** |
| ***ABL* NCN** | 0.96 | **0.91** | **0.98** | **0.98** | **0.98** | **0.99** | **0.98** | **0.97** | 0.93 |

| | | | | | | | | | |
|---|---|---|---|---|---|---|---|---|---|
| Correlation coefficients were calculated on three diluted FG positive RNA samples. Details appear in Figures 31 and 32. In bold, highest absolute value(s) observed. | | | | | | | | | |

**Table 34 Overall results of the QC rounds on coded RNA samples within the EAC network (phases III and IV)**

| QC phase ^{a} | False negativity | | False positivity | |
|---|---|---|---|---|
| | 10⁻³ | 10⁻⁴ | FG neg. control | NAC/NTC |
| IIIa | 1.7% (2/120) | 4.6% (6/130) | 6.5% (7/108) | 4.2% (17/408) |
| IIIb | 0.8% (1/119) | 1.7% (2/116) | 4.7% (11/234) | 2.1% (9/436) |
| Iva | 0% (0/77) | 5% (6/120) | 7.6% (15/198) | 3.7% (10/272) |
| All phases | 1.0% (3/316) | 3.8% (14/366) | 6.0% (33/546) ^{b} | 3.2% (36/1116) |

| | | | | |
|---|---|---|---|---|
| Results are proportions of false positive or negative samples. ^{a}During phase IIIb, coded samples were tested in randomly assigned laboratories. False positive results were observed in all the three QC rounds for *BCR-ABL* M-bcr, *PML-RARA* and *AML1-ETO* networks. | | | | |

**Table 35 False positivity per FG network during the QC rounds (phases III and IV)**

| Fusion transcript | FG neg. control | NAC/NTC |
|---|---|---|
| *E2A-PBX1* | 0% (0/44) | 0%(0/52) |
| *MLL-AF4* | 1.1% (1/89) | 4.6%(4/87) |
| *TEL-AML1* | 6%(3/50) | 0% (0/43) |
| *BCR-ABL,* m-bcr | 4.8% (6/126) | 5.8% (7/120) |
| *BCR-ABL,* M-bcr | 10.9% (6/55) | 4.1% (14/340) |
| *SIL-TAL1* | 4.3% (2/46) | 0%(0/162) |
| *PML-RARA* | 11% (5/45) | 5% (5/100) |
| *CBFB-MYH11* | 2.2%(1/45) | 0% (0/104) |
| *AML1-ETO* | 20%(9/46) | 5.6% (6/108) |
| All FG | 6.0% (33/546)^{a} | 3.2% (36/1116) |

| | | |
|---|---|---|
| Results are proportions of false negative samples. ^{a} When excluding *AML1-ETO* network, proportion decreases to 4.8% (24/500). | | |

**Table 36: List of the 14 housekeeping genes ⁴³analyzed in the first round**

| **Candidates CGs** | | | | **Reason(s) for exclusion** |
|---|---|---|---|---|
| **Code** | **Name** | **Chromosome** | **Median Ct value** | |
| 18S rRNA^{a} | 18S Rrna | 12p12 | 14.5 | Very high expression |
| *ABL*^{b,c} | **Abelson** | **9q34** | 29.0^{b} | |
| | | | **27.7^{c}** | |
| *PO* ^{a} | Acidic ribosomal protein | 11p15 | 22.3 | Pseudogenes |
| *B2M*^{a,b} | **Beta-2-microglobulin** | **15q21** | 20.7^{a} | |
| | | | **21.6^{b}** | |
| *ACTB*^{a} | Beta-actin | 7p15 | 20.5 | Pseudogenes |
| *GUS*^{a} | Beta-glucuronidase | 7q21 | 25.9 | |
| *CYC*^{a} | Cyclophilin | 7p13 | 27.6 | Pseudogenes |
| *GAPDH*^{a} | Glyceraldehyde 3 phosphate dehydrogenase | 12q13 | 21.1 | Pseudogenes |
| *HPRT*^{a} | Hypoxanthine phosphoribosyltransferase | Xq26 | 26.8 | Ch(X) |
| *PGK*^{a} | Phosphoglycerokinase | Xq13 | 24.2 | Ch(X) |
| *PBGD*^{b} | Porphobilinogen Deaminase | 11q23 | 27.1 | alternative transcriptional start sites |
| *PBGD2*^{b} | Porphobilinogen deaminase 2 | 11q23 | 27.0 | |
| *TBP*^{a,b} | Transcription Factor IID | 6q27 | 30.0^{a} | Low expression ^{a} |
| | | | 25.7 ^{b} | Variable in PBMNC ^{b} |
| *TFRC*^{a} | Transferrin Receptor | 3q26 | 26.6 | Variable expression in hematopoietic cells |

| | | | | |
|---|---|---|---|---|
| ^{a} Pre-developed human endogenous control plate from Applied Biosystems (65 samples tested). ^{b} In-house primer/probe set tested (36 samples tested). ^{c} EAC primer/probe set tested (20 samples tested). Samples have been tested before having optimized RT and RQ-PCR conditions. Thus median Ct value per CG might differ from the subsequent extensive analysis. In bold, selected genes for the final validation. | | | | |

**Table 37 : Sequences of the three finally EAC selected CG RQ-PCR sets**

| **Gene Acc Num** | **EAC Code^{a}** | **Sequence (5'-3')** | **5' Position (size)** |
|---|---|---|---|
| Abelson | ENF1003 | TGGAGATAACACTCTAAGCATAACTAAAGGT | 589 (31) |
| (*ABL*) | ENPr1043 | Fam-CCATTTTTGGTTTGGGCTTCACACCATT-Tamra | **684 (28)** |
| M14752 | ENR1063 | GATGTAGTTGCTTGGGACCCA | 712(21) |
| **beta-2-microglobulin** | ENF1302 | GAGTATGCCTGCCGTGTG | 302 (18) |
| (*B2M*) | ENPr1342 | Fam-CCTCCATGATGCTGCTTACATGTCTC-Tamra | 388 (26) |
| NM_004048 | ENR1362 | AATCCAAATGCGGCATCT | 411 (18) |
| **Beta-glucuronidase** | ENF1102 | GAAAATATGTGGTTGGAGAGCTCATT | 1759 (26) |
| *(GUS)* | ENPr1142 | Fam-CCAGCACTCTCGTCGGTGACTGTTCA-Tamra | 1833 (26) |
| M15182 | ENR1162 | CCGAGTGAAGATCCCCTTTTTA | 1859 (22) |

| | | | |
|---|---|---|---|
| ^{a} ENF = forward primer, ENPr = TaqMan reverse probe (in order to select the C-rich strand), ENR = reverse primer | | | |

**Table 38: Origin and type of fresh normal donor and leukemia patient samples during phase IVb**

| **Laboratory Reference N°** | **Normal Donor** | | | **Diagnostic Leukemic Samples** | | | | | | **Nb. samples** |
|---|---|---|---|---|---|---|---|---|---|---|
| | **PB** | **BM** | **PBSC** | **AML PB** | **AML BM** | **ALL PB** | **ALL BM** | **CML PB** | **CML BM** | |
| 1 | 11 | 5 | 7 | 6 | 9 | 6 | 7 | 8 | 6 | 65 |
| 2 | 11 | | 4 | | 3 | | | 1 | 1 | 20 |
| 3 | 11 | 1 | 1 | | 3 | 9 | 4 | 5 | 3 | 37 |
| 4 | 11 | | | 3 | 5 | 2 | 2 | | | 23 |
| 5 | 18 | 9 | | | | | | | | 27 |
| 6 | 11 | 2 | 4 | 2 | 3 | 5 | 3 | | | 30 |
| 7 | | 9 | 9 | 4 | 2 | | 2 | 1 | 2 | 29 |
| 8 | | | | | | 10 | 10 | | | 20 |
| 9 | | | | | 1 | | | 14 | 4 | 19 |
| 10 | | | | | 11 | | 1 | | 6 | 18 |
| 11 | 1 | | | | | | 7 | | | 8 |
| 12 | | | | 1 | 1 | | 1 | 3 | 1 | 7 |
| 13 | | | 1 | | | | | 5 | 1 | 7 |
| 14 | | | | 2 | 1 | 1 | | 2 | | 6 |
| **Nb. Samples** | **74** | **26** | **26** | **18** | **39** | **33** | **37** | **39** | **24** | **316** |

| | | | | | | | | | | |
|---|---|---|---|---|---|---|---|---|---|---|
| Among 316 included samples, six were excluded due to a poor *B2M* plasmid amplification (see Materials and Methods section). | | | | | | | | | | |

**Table 39: Reference values of transcript expression level in normal and leukemia samples for the three selected CGs**

| Genes | Samples | n | **Ct ^{a} Median** [3^{rd}-97^{th} perc.] | **CN^{a} Median** [3^{rd}-97^{th} perc.] |
|---|---|---|---|---|
| ***ABL*** | **Leukemia and Normal^{a}** | 310 | 24.9 [21.9-29.3] | 18 000 [1.3x10³-1.3x10⁵] |
| ***B2M*** | **Leukemia** | 184 | 18.7 [15.6-24.9] | 550 000 [9.6x10³-4.3x10⁶] |
| | **Normal PB and PBSC^{a}** | 100 | 17.2 [15.7-20.0] | 1 600 000 [2.5x10⁵-6.8x10⁶] |
| | **Normal BM** | 26 | 17.8 [16.7-22.9] | 1 100 000 [2.9x10⁴-2.0x10⁶] |
| ***GUS*** | **Leukemia** | 184 | 23.8 [20.8-28.0] | 41 000 [2.8x10³-3.2x10⁵] |
| | **Normal BM and PBSC^{a}** | 52 | 23.0 [21.1-26.3] | 66 000 [7.6x10³-2.0x10⁵] |
| | **Normal PB** | 74 | 24.5 [22.6-27.7] | 22 000 [2.6x10³-7.1x10⁴] |

| | | | | |
|---|---|---|---|---|
| ^{a} Data have been merged according to the results of the post-hoc analysis. | | | | |

**Table 40: EAC Formula for calculation of MRD value and theoretical sensitivity based on EAC selected FG and CG RQ-PCR sets**

| **Parameters** | **ΔΔCt method** *No standard curve needed* | **NCN method** *FG and CG standard curves* |
|---|---|---|
| MRD value (MRDv) | ₁₀((ΔCt_{FUP}-ΔCt_{DX})/-3.4) | **(FG_{CN} / CG_{CN})_{FUP}** |
| | | **(FG_{CN} / CG_{CN})_{DX}** |
| **Sensitivity (SENSv)** | (40-Ct_{CG,FUP}-ΔCt_{DX})/-3.4) | CG_{CN,DX} |
| | | CG_{CN,FUP} x FG_{CN,DX} |

| | | |
|---|---|---|
| DX: diagnostic; FUP: follow-up; NCN: normalized copy number; CT_{CG}: Ct value of the CG; CT_{FG}: Ct value of the FG; FG_{CN}: fusion gene copy number, CG_{CN}: control gene copy number. ΔCt_{FUP}: (Ct_{FG} - Ct_{CG}) during follow-up, ΔCt_{DX}: (Ct_{FG} - Ct_{CG}) at diagnosis. Values 40 and -3.4 are respectively experimental results for the intercept value and mean slope observed in our EAC program for plasmid standard curves. | | |

### DETAILED DESCRIPTION OF THE PRESENT INVENTION

### Organization

The aim of phases I and II was the initial selection of primers and probes in addition to training of the members.

During Phase IIIa, different standard curves were compared (i.e. generated using RNA, cDNA or DNA plasmids) and the final validation of selected primer/probe sets within specific networks was made. In parallel, inventors performed their first quality control round (QC1).

During Phase IIIb, testing of selected FG primer/probe sets was undertaken by all the laboratories involved, on centrally prepared coded quality control samples (QC2). This testing of the FG transcript targets was performed by randomly selected laboratories outside the original FG transcript networks.

During phase IVa, the third quality control round (QC3) was performed including undiluted cell line RNA samples.

During Phase IVb, the reference values of normalized FG transcript levels in leukemic cell lines and patient samples was determined using the EAC primer/probe sets according to the EAC standardized protocol.

In addition, reference ranges were established for the control genes in normal peripheral blood (PB), bone marrow (BM) and PB stem cells in fresh samples.

### Material & Methods

### Principle

Currently available RQ-PCR technologies allow detection of fluorescence emission during the PCR reaction from one (TaqMan™) ot two (Light Cycler) internal oligonucleotide probes, or a fluorescent dye; the detected fluorescence being proportional to the amount of target present in the sample 7.

It was decided to run their EAC protocol using the ABI 7700 platform with TaqMan probes since this was the first robust RQ-PCR technology available permitting analysis of a large number of samples in a single run (96-well plate format). The 5' nuclease assay (TaqMan technology) uses a single internal oligonucleotide probe bearing a 5' reporter fluorophore (e.g. FAM) and 3' quencher fluorophore (e.g. TAMRA). During the extension phase, the TaqMan probe is hydrolysed by the nuclease activity of the Taq polymerase, resulting in separation of the reporter and quencher fluorochromes and consequently in an increase in fluorescence (Figure 1). In the TaqMan technology, the number of PCR cycles necessary to detect a signal above the threshold is called the Cycle threshold (Ct) and is directly proportional to the amount of target present at the beginning of the reaction (Figure 2a). The ΔRn corresponds to the increase in fluorescence intensity when the plateau phase is reached. Using standards or calibrators with a known number of molecules, one can establish a standard curve and determine the precise amount of target present in the test sample (Figure 2b). The theoretical slope of the standard curve is - 3.32 for a PCR reaction with maximum efficiency. Using a known amount of DNA molecules for the standard curve, the intercept point on the Y-axis defines the number of cycles theoretically needed to detect one molecule.

### Molecular targets

Assays were designed to detect nine leukemia-associated fusion genes, including their more common breakpoint variants giving rise to 15 RNA targets: E2A-PBX1, MLL-AF4 (variants exon 9 - exon 5, exon 10 - exon 4 and exon 11 - exon 5), TEL-AML1, BCR-ABL (M-bcr and m-bcr), SIL-TAL1, PML-RARA(bcr1, bcr2 and bcr3), CBFB-MYH11 (type A, D and E) and AML 1-ETO.

In addition, 14 housekeeping genes were evaluated for their suitability to serve as control genes for sample to sample quality variations and gene expression quantification. Three control genes were ultimately selected (ABL, B2M and GUS); inventors analyzed the expression of the ABL gene during phases II-III and all three selected control genes (ABL, B2M and GUS) during phase IV.

### Primer and probe design

Primers and probes were designed using Primer Express software (Applied Biosystems) based on their location on two separated exons and on the sequence of the amplicon generated by the primer sets described in the BIOMED-1 program (5). They are depicted in the schematic diagram of the exon/intron structure of the corresponding FG (see Examples 1-9). During the first two meetings an initial selection of primers and probes was made; newly designed sets for each molecular target as well as already available "in house" sets from experienced laboratories were evaluated. The set selection was based on: 1) the absence of non-specific amplification artifacts ; 2) a good efficiency with a slope close to - 3.32 (100% theoretical efficiency) ; 3) a good sensitivity (at least 10⁻⁴ RNA dilution or 100 copies for plasmid dilution) ; 4) the robustness of the reaction with a ΔRn value >1.0 at the plateau phase for the highest dilutions (Table 4). Such results had to be reached in at least 80 % of the participating laboratories for a particular primer/probe set to be selected. Potential primer/probe sets were tested in parallel on serial dilutions of cell lines and plasmids, and the set with the best performance profile, particularly in terms of sensitivity, was selected. If none of the primer/probe sets satisfied the selection criteria, new sets were designed and evaluated. Overall, starting from 47 primer sets and 44 probes which were tested during the first phases, 12 primer sets and nine probes were finally selected for the 15 targets (see diagrams in each example section). In each case, the sensitivity of the TaqMan-based RQ-PCR analysis appeared to be comparable to previously standardized nested RT-PCR analysis (5). On the BCR gene, Y (Cytidine or Thymidine) appears on the BCR primer at position 3188, according to the polymorphism recently described.

In the initial CG screening, a pre-developed human endogenous control plate (kindly provided by Applied Biosystems) containing primer and probe sets for 11 CGs was used as recommended by the manufacturer (Table 36). Six additional CGs primer and probe sets, designed by EAC laboratories, were analyzed: one EAC and one in-house set for Abelson (ABL), one in-house set for beta-2-microglobulin (B2M), two EAC sets for Porphobilinogen deaminase (PBGD and PBGD2) and one in-house set for Transcription Factor IID (TBP) (Table 36). After selection, the number of CGs was ultimately reduced to three, which were subjected to further analysis (Figure 34 and Table 37). Oligonucleotide primers and probes were designed employing the Primer Express 1.0 program (Applied Biosystems) and the Oligo 6 program (Molecular Biology Insights Inc., Cascade, CO, USA). Primer and probe sets were also tested using 100 ng genomic DNA per PCR to confirm that assays were cDNA specific.

### RNA and cDNA from cell lines and leukemia samples at diagnosis

RNA and/or cDNA samples were prepared centrally by the FG network leaders (Table 1) and distributed on dry ice to members of their respective networks during phases I to IVa. FG transcript - positive cell line RNA was commonly used (see Table 1 for specific cell lines), except for rare FG transcripts (PML-RARA bcr2 and bcr3 and CBFB-MYH11 type D and E) for which patient RNA was provided by the network leader. These RNA samples were diluted in PB lymphocytes (PBL) RNA or FG transcript-negative cell line RNA. Cell lines were purchased from the DSMZ (Braunschweig, Germany), ATCC (Manassas, VA, USA) or directly provided by academical laboratories (TOM-1, ME-1 and PF382) and cultured according to the supplier's instructions. During phase III, network leader laboratories prepared equivalent dilution series of cDNA. During Phase IVb, patient sample RNAs, positive for the relevant FG transcript and that had been stored for less than 18 months, were analyzed locally undiluted and/or diluted in a solution of 1 µg/µl E. Coli 16S & 23S rRNA (Roche, Meylan, France) in duplicate.

### Plasmids calibrators

PCR products of ABL, B2M and GUS gene transcripts for preparation of plasmids were amplified with respectively ABL-F (5'-CCT TCA GCG GCC AGT AGC-3') & ABL-R (5'-GGA CAC AGG CCC ATG GTA C-3'), B2M-F (5'-CCT TGA GGC TAT CCA GCG T-3') & B2M-R (5'-CCT GCT CAG ATA CAT CAA ACA TG-3') and GUS-F (5'-CCT GTG ACC TTT GTG AGC AA-3') & GUS-R (5'-GTC TGC CGT GAA CAG TCC A-3'). PCR conditions of the BIOMED-1 Concerted Action were used.(5) The protocol for cloning and preparation of plasmid dilutions has already been disclosed. For ABL and GUS plasmids, three dilutions (105, 104, and 103 copies in 5 µl) were used to calculate the standard curve. For the B2M plasmid, three different dilutions (107, 106, and 105 copies in 5 µl) were used. Corresponding coefficients of variation for Ct values were below 4% (ABL), 5% (B2M) or 3% (GUS) for all dilutions (data from all phases).

### Biological material and preparation of RNA

From heparinized peripheral blood (PB), bone marrow (BM), and PB stem cells (PBSC) mononuclear cells (MNC) were obtained by Ficoll-Hypaque density centrifugation. All patient sampling was performed according to protocols approved by the local ethical committees of the given institutions and/or geographical areas. The RS4;11 cell line was grown in medium RPMI1640 with 10% fetal calf serum and harvested in the exponential growth phase. RNA was extracted by routine methods in the participating laboratories employing either a TRIzol reagent (Invitrogen, Cergy, France), a RNAzol reagent (Biotech Italia, Rome, Italy) or a column based system (Qiagen, Hilden, Germany) according to the manufacturer's recommendations. After extraction and isolation, the RNA concentration was determined by measurement of the optical density at 260 nm and the RNA was stored at -80°C until use.

### Plasmid DNA calibrators containing the target gene sequences

PCR products of the 15 different FG transcripts were generated from cell line or patient RNA by RT-PCR using BIOMED-1 A and B primers, as previously described. (5) PCR products were cloned into the PCR II TOPO vector (Invitrogen, Groningen, Netherlands). The selected plasmid clones were sequenced for confirmation of their insert (Genome Express, Grenoble, France). After subsequent bulk production, the plasmids were extracted using QIAFILTER Plasmid MIDI kit (Qiagen, Courtaboeuf, France) and quantified spectrophotometrically. The copy number for 1 µg was estimated according to the molecular weight of the vector and the insert. Then 20 µg of plasmid were linearized with BamHI or HindIII restriction enzymes for 1 h at 37°C under agitation. The digested plasmid was serially diluted in a solution of Tris 10 mM, EDTA 1 mM pH8, containing 20 ng/µl of E. Coli 16S & 23S rRNA (Roche). Five successive dilutions (200 000, 20 000, 200, 20 and 2 copies per microliter) were prepared. The corresponding standard curve generated a mean slope of - 3.45 and intercept of 39.8±1 Ct. A mean Ct value of 22.5±1 was obtained for the 20 000 copies/µl dilution.

### Standardized RT-PCR protocol

A common EAC protocol was established for all molecular targets (FG and CG transcripts) during phases I and II and then used by each laboratory throughout phases III and IV (Table 4).

### RT step

This reaction was adapted from the BIOMED-1 protocol.24 Starting from one µg of total RNA, the main modifications involved alteration of the concentrations of random hexamers (25 µM) and of reverse transcriptase (100U) either MMLV or Superscript (Invitrogen; Roche), which significantly enhanced the sensitivity of the assay.

### RQ-PCR step

All the RQ-PCR reactions were performed on a 7700 ABI platform (Applied Biosystems, Foster City, USA) using primers and TaqMan probes kindly provided by Applied Biosystems in conjunction with the TaqMan Universal Master Mix purchased from the same manufacturer. The number of amplification cycles was 50 (detailed protocol is described in Table 3).

### Optimization of RQ-PCR assay (phase II)

In order to optimize the results and save costs, Phase II involved assessment of the influence of primer and probe concentrations (900 and 300 nM for primers and 200 and 100 nM for probes) and the reaction volume (50 µl versus 25 µl) on the sensitivity of the assay.

After extensive testing within FG networks using serial dilutions of FG positive cell line RNA in FG negative RNA (10⁻¹ to 10⁻⁵) and plasmid (106 to 10 copies), optimal results were obtained using a 25 µl volume, with 300 nM of primers and a 200 nM concentration of probe, except for AML1-ETO for which a 100 nM probe was chosen (see Example 9).

For comparative data analysis, a common threshold set at 0.1 was selected in order to be in the exponential phase. Such a threshold value typically lays above so-called "creeping curves" that were rarely observed in some negative controls; "creeping curves" being defined as the amplification curve from a negative sample rising slowly during the PCR reaction. Whilst the mechanism underlying the latter phenomenon is not entirely clear, examination of the "multi-component" view reveals that it is not indicative of specific amplification (see below). However, for PML-RARA the threshold was fixed at 0.05 because of the relatively short exponential phase of the PCR amplification (low ΔRn). The base line was calculated from cycles 3 to 15 (for ABL and GUS) except for high expression control genes where cycles 3 to 10 were used (for B2M). Additionally inventors used dilutions of positive RNA (patient or cell line) into normal PBL or a FG negative cell line RNA (10⁻¹ to 10⁻⁶) during phases I to IIIa and identical dilution series using cDNA during phase IIIa.

### Standard curve comparison (phase IIIa)

A comparison between RNA, cDNA and plasmid standard curves was performed in order to determine the influence of the RT step on the results (standard curve slope, sensitivity and reproducibility) for each laboratory. The FG network leaders sent to the different laboratories within their target network (4 to 12 members, see Table 1) centrally prepared serial dilutions of the FG transcript-positive cell line RNA and the corresponding cDNAs, in addition to serial dilutions of the corresponding FG control plasmid (200 000, 20 000, 200, 20 and 2 copies per µL).

### Quality Control (QC) rounds on coded samples

### General organization

QC1 (phase IIIa) and QC3 (phase IVa) were performed by specific laboratories (Table 1) whereas QC2 (phase IIIb) involved randomly chosen laboratories (see balanced randomized assay section).

### Control samples and definition of (false-) posivitity and (false-) negativity

The positive controls in all experiments and QC rounds concerned well-defined cell lines and patient samples (Tables 1 and 2). Two types of negative controls were used: 1) coded FG negative RNA samples and 2) known negative controls for checking contamination of PCR products. These later contamination controls concerned no-amplification controls (NAC), which contained E. coli RNA instead of human cDNA, and no-template controls (NTC), which contained water instead of human cDNA. Particularly the NAC and NTC negative controls were regarded to be of utmost importance for identification of cross-contamination of PCR products, because this problem is frequently underestimated.

A positive well was defined as a sigmoïdic amplification (log scale) with a Ct value below the Y-intercept Ct value of the plasmid standard curve + one Ct. Amplification on RNA samples of the FG was performed in triplicate and in duplicates for the CG expression. A false negative sample was defined as a positive RNA sample with less than 50% of positive wells (0/2, 0/3 or 1/3). A false positive result was defined as a negative sample, with at least 50 % of positive wells (1/2, 2/3 or 3/3).

### Sensitivity and reproducibility of the experiments

The criteria for sensitivity and reproducibility were defined during QC experiments via coded samples for each FG transcript (Table 4). An experiment was assumed to be reproducible for a particular dilution if more than 80 % of the laboratories detected at least two positive wells with a Ct difference less than 1.5 Ct. The sensitivity of the experiment was defined as the last dilution showing at least 50 % of positive wells in more than 80 % of the laboratories whatever the Ct values were.

### Balanced randomized assay (phase IIIb)

This assay was designed by the Department of Medical Information (Marseille, France). The aim was to evaluate to what extent RQ-PCR results were comparable between laboratories for MRD quantification of FG transcripts in a clinical setting according to the EAC protocol. The study focused on two main points: 1) comparison of the results between laboratories for a particular FG transcript which is crucial for multicenter studies and 2) the linearity of the RQ-PCR methodology in dilution experiments which is important to assess the potential tumor load during treatment. The balanced randomized assay appeared to be an appropriate statistical study to assess these two points without performing all the RQ-PCR analyses (n=15) in each participating laboratory (n=25).

The statisticians randomly assigned the 25 participating laboratories to nine networks. Each of the nine main FG transcripts were tested in 11 laboratories (except for CBFB-MYH11 network, n=12), including the involved FG transcript network leader making a total of 100 RQ-PCR experiments. Each laboratory tested five coded samples for four different targets making a total of 500 coded samples analyzed in this QC2 study (Table 2). FG transcript-positive control RNAs were diluted (10⁻¹, 10⁻³ and 10⁻⁴ dilutions) in a negative RNA sample (HL60 or PBL).

A common plate design was used; the control gene (ABL), the FG transcript and plasmid dilutions (ABL n=3, FG n=5) were amplified in triplicate whereas four negative controls (NAC and NTC for ABL and FG targets) were run in duplicate. The raw data were collected and analyzed by each FG network leader. Common Excel worksheets were designed to collect the results within each FG network and were then forwarded to Marseille for subsequent statistical analysis (see below). The only exclusion criterion for coded RNA samples was an ABL Ct value outside the normal range [22-29.3] as defined by assays conducted within the CG network during phase IIIa.

### Collection of data

For testing of the 14 candidate CGs (17 primer and probe sets), data were obtained from i) ABI endogenous control plate, ii) in-house and iii) newly designed EAC primer and probe sets. For the ABI endogenous control plate, five laboratories tested a total of 65 different archived samples: 22 normal PBMNC, 15 ALL (4 PB / 11 BM), 15 CML (10 PB / 5 BM), and 13 AML (5 PB / 8 BM) samples obtained at diagnosis. This number was reduced to 53 for TFRC (data partially missing for one laboratory) and 21 for 18S rRNA (data available from one laboratory only). A minimum of four normal PB and four leukemic samples was expected per laboratory. For in-house ABL, B2M and TBP primer and probe sets, 20 samples were tested in one laboratory: 4 normal PBMNC, 3 ALL, 7 AML and 6 CML. For EAC ABL, PBGD and PBGD2 sets, 36 samples were tested: 4 PBMNC, 11 ALL, 7 AML and 14 CML in two laboratories. Paired results obtained with two different sets of primer for the same CG (one laboratory only) were available for three CGs: ABL (n=20), B2M (n=12) and TBP (n=12). All these experiments were performed before having optimized the RT step and before establishing a common threshold for data analysis.

In the prospective study, fresh normal (PB, BM or PBSC), ALL, AML or CML (either PB or BM) samples were tested in individual laboratories for the three EAC selected CGs (Table 37). MNC were isolated and cells were lysed (initial step of the RNA extraction procedure) on the same day as the samples were obtained. Initially, 316 samples were collected (Table 38). Six leukemia samples were excluded because CG analysis was impossible due to a poor B2M plasmid amplification. Consequently, only 310 samples were analyzed to define reference values on the same set of sample. Information on the presence of a putative FG transcript was not available.

For the retrospective study (phase IVb), 311 archived ALL, AML or CML samples (either PB or BM) with an identified FG transcript were tested in individual laboratories for the three EAC selected CGs, even if these laboratories were not included in the corresponding FG network. Results were collected and tabulated by the FG network leaders. Only samples with a CG Ct value within the reference range defined below were selected for the analysis. In the TEL-AML1 network only 30 out of 57 samples were tested for GUS transcript expression. In the E2A-PBX1 network, data on B2M expression was not available for one sample.

### Statistical analysis

### General methodology

The CG and the FG RQ-PCR data were collected and analyzed by each FG network leader. The mean Ct or mean ΔCt (mean Ct [FG] - mean Ct [CG]) values and the mean value of the log10 of the copy number (CN) for each gene were used. The normalized copy number (NCN) was defined as the CN of the FG per one copy of the CG transcript (mean value of log 10 [FG CN] - mean value of log 10 [CG CN]) except for normalization to B2M gene transcript level for which the results were expressed per 100 copies. Since CN did not show a normal distribution, the logarithmic value of the CN was used for statistical analysis. For an easier comprehension, results obtained in a logarithmic way were subsequently converted into decimal values. The level of significance was set at p<0.05. In Tables and Figures, when the p value was between 0.05 and 0.1, even if not statistically significant, the numbers are noted. When the p value was > 0.1, the result appears as not significant (NS). The correlations between the expression level of different genes were measured by the Pearson correlation coefficient (r). The box-plots used for presenting data show the median value (dark line) within a box containing 50 % of the samples (25th to 75th percentile). The statistical analysis was performed in Marseille, France, using the SPSS 10.1 Software (SPSS Inc., Chicago, USA).

### Balanced randomized assay (phase IIIb)

### - Detection of significant differences in transcript quantification between laboratories

Four parameters per sample (Ct, ΔCt, CN and NCN) were tested using a global linear model. The laboratory number was set as a random effect and data were analyzed. When the results were not comparable between laboratories (p<0.05) for the selected parameter and transcript, a post-hoc analysis (Tukey method) was used to evaluate the number of laboratories reproducible with others (p≥0.05) defining a subgroup. For this, two criteria were chosen to estimate the reproducibility of the results : the number of subgroups (Sn) and the number of laboratories (Ln) reproducible with at least two other laboratories as defined by a non-significant difference between the mean (p≥0.05) according to the Tukey method. When a particular laboratory was present in two or more subgroups, it was counted only once. The best parameter to compare results between laboratories was the one with the highest Ln value and the lowest Sn value. Ideally, only one subgroup (Sn=1) containing all the laboratories (Ln = 11 or 12 per network or Ln=25 for the whole assay) was expected.

### - Evaluation of the linearity

The Pearson correlation coefficient was chosen to measure the linearity of the quantification of the FG transcripts using the three coded positive samples. For each FG transcript, the best parameter (ΔΔCt or NCN) to assess the results was the one with the highest correlation coefficient.

### Reference values at diagnosis (Phase IVb)

Leukemia samples were excluded from the analysis if the CG amplification was not within the normal range for fresh samples defined as follows ABL Ct [21.8-29.4], B2M Ct [15.6-24.9] and GUS Ct [20.8-28.0]. The comparison between PB and BM was performed with non-parametric tests (Wilcoxon paired test for at least five paired samples or Mann-Whitney U test for unpaired samples). At least five paired samples were expected per FG transcripts. The 95 % range of expression for NCN refers to the range between the 3rd and the 97th percentile for the selected gene. Correlation coefficients between the FG CN and each CG CN are given before normalization by the CG. For cell line(s), the median value obtained on the same sample in eight different laboratories is shown.

### Results

### Selection of control genes

### Criteria for selection

The control gene group started with a screening of candidate CGs on normal PB and diagnostic leukemic samples using: i) pre-developed human endogenous control plate (ABI) containing primer/probe sets for 11 commonly used CGs and ii) six primer/probe sets (Table 36). Of the 17 primer/probe sets covering 14 CGs, the network aimed to identify at least one with a high median expression (Ct between 16.4 and 23.0) and at least one with a medium median expression (Ct between 23.0 and 29.6). These limits arbitrarily defined covered a range of two logs (6.6 Ct). The lower limit was selected in order to obtain an adequate number of records for the baseline calculations and the higher limit to achieve sufficient sensitivity for sample evaluation.

The selected CGs should fulfill the following major criteria: i) absence of pseudogenes (known or encountered during testing), ii) high or medium expression, excluding very high or low expression, iii) no significantly different CG expression between normal PB samples and leukemic samples and iv) no significantly different CG expression between PB and BM. Minor criteria for exclusion were also identified: i) X-chromosomal location in order to avoid any potential dosage gender effect, ii) variability within one leukemic type (AML, ALL or CML) at diagnosis or normal PB, and iii) cell cycle dependent expression.

Two CGs were excluded according to their expression level: 18SrRNA and TBP tested with ABI primer and probe sets (Table 36). Among the four highly expressed CGs (PO, ACTB, GAPD and B2M), only B2M was not known to have pseudogenes. However, B2M expression analyzed by the ABI set differed significantly between normal and leukemic samples and between PB and BM (p<0.001 in both cases, n=65, Mann-Whitney test). Therefore the ABI B2M set was discarded. Among the five remaining CGs with medium expression on ABI plate, three were discarded due to minor reasons: HPRT and PGK (X-chromosomal location) and TFRC (variable expression in hematopoietic cells).

Among the six additional primer and probe sets, covering four CGs, in-house B2M RQ-PCR set appeared to be suitable since no statistically significant difference between PB and BM was observed. Comparison between normal and leukemic samples was not possible since only four normal samples were tested (see material and methods). Among CGs with medium expression, TBP transcript amplified with the EAC set was of suitable median expression level (Ct = 25.7). PBGD and PBGD2 sets exhibited similar median Ct value (Ct = 27). However, due to the presence of alternative transcriptional start sites, these PBGD sets were finally discarded. Median Ct values of the two ABL sets were not identical (Table 36) although Ct values were correlated (r=0.87, n=20). ABL EAC set was preferred since the median Ct value was lower.

So, after the first selection process, the number of genes was reduced to five: ABL, B2M, CYC, GUS and TBP (Table 36).

### Variability of expression in normal PBMNC

These five initially selected CGs were subjected to further analysis using five locally prepared PB samples from normal donors in each of the six CG laboratories. RQ-PCR analysis was performed using optimized EAC RT and PCR protocols. The variations in Ct values of ABL, B2M, CYC and GUS were comparable and all within three Ct values (Figure 37). Connecting lines of these four CGs were nearly parallel. Thus, variations in Ct values could be ascribed to differences in the efficiency of the cDNA synthesis and/or variation in the initial amount of RNA (see below). In contrast, the TBP gene transcript showed a variation of five Ct values (equal to 32-fold) and non-parallel sample lines, indicating larger variation in the TBP gene expression (Figure 37). The TBP gene was therefore excluded from further analysis.

### cDNA specificity of primers/probe sets

Since primer and probe sequences used for the human endogenous control plate were not available, new primer and probe sets (EAC sets) for the CYC and GUS genes were designed and tested. Three different CYC primer/probe sets were evaluated in two laboratories and each set was found to amplify genomic DNA due to the presence of pseudogenes. Thus, CYC was excluded from further analysis.

To evaluate the risk of false positive results due to pseudogenes or fortuitous genomic homologies, the ABL, B2M and GUS EAC primer/probe sets were tested in five laboratories on 150 genomic DNA samples (30 per laboratory) obtained from normal donors and leukemic patients. All RQ-PCR analyses for B2M and GUS were negative, whereas 7% (10/150) of samples were positive in the ABL RQ-PCR in three out of five testing laboratories (n=2, 3 and 5 positive results, respectively). However, Ct values ranged from 35 to 45 (data not shown) and therefore were far away from the Ct values obtained using good quality RNA samples. Consequently, even if some remaining DNA was present in the RNA sample, this would not significantly affect the CG data. Therefore, inventors decided not to exclude the ABL primer set based on the low level amplification of DNA. In conclusion, ABL, B2M and GUS primer/probe sets shown in Table 37 were selected for further testing as potential candidate CGs.

### CG expression in normal and leukemic samples

After having established the optimal conditions for the cDNA synthesis and RQ-PCR protocols and having defined the best-suited CGs in the initial studies, the EAC network proceeded to establish the biological variation in the expression of the selected CGs in normal and leukemic samples.

### Expression of the selected CGs in fresh samples

This prospective study was performed on normal donors (n=126) as well as on leukemic patients at diagnosis (n=184). Normal PBSC (n=26) were tested since MRD can also be studied in this particular harvest. Contributing laboratories as well as sample type and numbers are shown in Table 38.

In normal samples, ABL gene expression did not differ significantly between PB, BM and PBSC (Figure 38a). On the contrary, B2M gene expression significantly differed between the three different sample types (PB, PBSC and BM) if analyzed using a global linear model (p<0.001, n=126). However, post-hoc analysis revealed that B2M expression level in PB or PBSC samples was comparable (Figure 38b). GUS gene expression was significantly different between the three sample types using a global linear model (p<0.001, n=126). Post-hoc analysis showed that GUS expression was significantly lower in PB but not significantly different between BM and PBSC (Figure 38c). In leukemic samples, CG expression was not significantly different between BM and PB or between ALL, AML and CML, except for GUS expression (p=0.03, n=184) (Figures 38a,b,c).

Comparison between normal and leukemic samples showed that only ABL expression did not differ significantly (p=0.21, n=310, (Figure 38a). In contrast, significant differences (p<0.001, n=310) were found for B2M and GUS expression using a global linear model (Figures 38b et c). When Ct values were analyzed instead of CN on the same samples (n=310), comparable results were obtained.

### Reference values for CG expression in fresh normal and leukemic samples

Inventors decided to establish reference values on fresh samples in order to evaluate the range of CG expression and subsequently to identify poor quality samples (see Material and Method section). Reference values were defined by a target (median value) and two limits (3rd and 97Th percentiles). Samples (6% of the fresh samples) outside this range were considered as unexpected results. Samples with a too high Ct value and consequently a too low CN were presumably degraded samples or samples containing an inhibitor, whereas samples with a too low Ct value and thus a too high CN could be related to an overestimated RNA quantification. The reference values, based on 126 normal samples and 184 leukemic samples at diagnosis are shown in Table 39.

In normal samples (n=126), an approximately 50-fold difference in ABL and GUS expression and up to 70-fold difference in B2M expression was found (Table 39). In leukemia samples (n=184), an approximately 100-fold difference in ABL and GUS gene expression and up to 500-fold difference in B2M expression was observed (Table 39). A parametric approach using the mean value (and not the median) and two standard deviations gave similar results to the methodology based on percentiles (data not shown).

### CG expression in archived samples

Data on archived leukemic samples at diagnosis (n=311) with an identified FG transcript were obtained from the FG groups. Undegraded samples were selected according to the reference values defined above, thus excluding cases with poor or no amplification of CG due to degradation or presence of inhibitors. ABL was a more restrictive CG for including samples compared to GUS or B2M (proportion of excluded samples, respectively 11%, 9% and 6%) resulting in a lower number of samples analyzed for this CG. Because in the TEL-AML1 network only 30 out of 57 samples were tested for GUS transcript expression, result of only 257 samples were finally available for this CG.

An analysis similar to the previous one performed on fresh leukemia samples (see above) was performed. Only ABL gene transcript CN did not differ significantly between tissues and leukemia type (p=0.50, n=277) or between FG transcript groups, when BM and PB samples were merged (p=0.10, n=277, Figure 6a). In contrast, the B2M gene transcript CN was significantly different between tissues and leukemia types (p=0.03, n=290) and between FG transcript groups (p=0.04, n=290, Figure 39b). Also GUS gene expression differed significantly between tissues and leukemia types (p<0.001, n=257) and between FG transcript groups (p<0.001, n=257, Figure 39c).

### Correlation between control genes and with the fusion genes

The expression level of the three CG was always correlated (p<0.01), whatever the Ct or CN values were used. The highest correlation was found between ABL and B2M (r=0.73) or GUS (r=0.72) Ct values in fresh normal samples (Figure 40a) and between ABL and GUS (r=0.80) Ct values in fresh leukemia samples (Figure 40b).

In archived leukemia samples, the highest correlation was found between B2M and GUS gene Ct values (r=0.67), whereas the lowest correlation was observed between ABL and GUS gene Ct values in the same samples (r=0.54), suggesting a differential degradation kinetic of these two genes. Finally, the correlation between the FG transcript expression and ABL gene transcript expression was higher or identical to the two other EAC selected CGs.

### Choice of the control gene

It was previously shown that ABL gene expression did not differ significantly between normal and leukemic samples at diagnosis. Moreover, of the three extensively tested CGs, ABL gene expression had the highest correlation with the FG transcripts in diagnostic samples. In the present inventors study, in a model of MRD detection (phase IIIb), normalization of FG expression to that of ABL as the CG improved the reproducibility of FG transcript results obtained in comparison to raw (not normalized) Ct or CN values. Therefore, inventors propose to use ABL as the CG of first choice for normalization in diagnostic and follow-up samples. As second choice, inventors recommend to use either B2M or GUS depending on their relative correlation with the respectice FG transcript expression and the variability of the NCN at diagnosis. In practice, identification of isolated samples with a low expression level of CG suggests RNA degradation or presence of inhibitors in such samples. On the other hand, observation of reduced or absent CG amplification for all samples tested is indicative of a reagent problem during the RT or PCR reactions. Finally, the CG transcript CN for each patient RNA sample allows normalization of efficiency of the pre-PCR steps.

### Standard curve comparison (phase IIIa)

No significant differences were observed in Ct values for the lowest RNA, cDNA (10⁻³ and 10⁻⁴) and plasmid (10⁶, 10⁵ and 10³ copies) dilutions, even between centrally and locally prepared cDNA samples. For the highest dilutions, the Ct values were more reproducible between laboratories for centrally distributed cDNA (10⁻³ and 10⁻⁴ dilutions) and plasmid (10 and 100 copies) than for RNA dilutions. The respective CV were below 5% for cDNA and plasmids and 11% for RNA at the highest dilution. In two target-networks (AML1-ETO and CBFB-MYH11 type A), this observation even resulted in significantly fewer positive results for the samples for which RT was performed locally compared to the centrally prepared cDNA samples. The slopes established with cDNA or plasmid dilutions were close to the theoretical slope -3.32 (100% efficiency) for the vast majority of the participating laboratories. In contrast, slopes from RNA dilutio4ns were indicative of lower reaction efficiency probably due to RNA degradation during transportation. Finally, the sensitivity levels of RNA dilutions for all targets were comparable to standardized nested PCRs designed in the BIOMED-1 program24 and were even better for bcr2 and bcr3 variants of PML-RARA. Ten plasmid copies could generally be detected by all laboratories (see phase IIIa and b).

### Balanced randomized assay (phase IIIb)

This assay was set up in order to detect differences in transcript quantification between laboratories. The laboratories were randomly chosen to amplify four different FG transcripts, generally outside their original FG networks (see Material and Methods section). This methodology is also of importance as a QC round to detect if false negativity and positivity are proportionally identical to other phases for which only laboratories focusing on a particular FG were performing the experiments.

### ABL amplification

### - On plasmid dilutions

Inventors observed very similar results for the three ABL plasmid dilutions amongst the 25 laboratories. Inventors found for the ABL 105 copies plasmid dilution: 22.30±0.38 (Ct±Sd, n=296) and a corresponding CV of 1.7%. Inventors found a significant laboratory effect (set as a random effect) on ABL Ct measurement for the three ABL plasmid dilution (p<0.001, n=296). But the difference between opposites laboratories was no more than 1.2 Ct. Such a difference might well not be relevant in a clinical point of view. According to the criteria defined in the Material and Methods section, all laboratories had reproducible results for Ct value of each ABL plasmid dilution.

### - On coded RNA samples

No significant difference was found within networks between highly diluted samples (10⁻³ and 10⁻⁴) and negative samples when ABL expression was compared using Ct or CN values (results of PML-RARA network given as example in Figure 30a), except for the CBFB-MYH11 network (p=0.03). On the contrary, inventors found highly significant differences (p<0.001) between laboratories within each network for the same samples (results of PML-RARA network given as example in Figure 30b). Thus Ct and CN values were dependent of the testing laboratory and not of the sample. These data strongly suggest that variations occur during the pre-PCR steps (transportation and RT efficiency).

### Fusion gene transcript amplification

Inventors focused on the best parameter (Ct, CN, ΔCt or NCN) to express RQ-PCR results in a serial dilution model. Inventors calculated the corresponding correlation coefficients on three diluted samples measured in 11 different laboratories (PML-RARA network as an example in Figure 31). The correlation coefficients were higher for NCN method in five FGs, slightly higher with the ΔCt method in two FGs whereas both methods were equivalent for the remaining two FGs (Table 33). Only for quantification of E2A-PBX1 transcripts, ABL appeared not to be helpful as CG to normalize the RQ-PCR results. Overall, use of ABL gene transcripts for normalization was found to greatly improve the linearity of the results (PML-RARA network as an example in Figure 32) and avoided overlap in FG Ct and CN values observed between different dilutions.

Finally, inventors checked the results of this model for MRD quantification. The correlation curve between Ct value (Y-axis) and CN value (log10, X-axis) for wells related to coded FG positive samples (n=824, covering all nine FG targets) showed a mean slope and an intercept of -3.35 and 39.7, respectively. These good results indicated that even with nine different plasmid sets (one per transcript), the quantification of the FG transcripts in the coded RNA samples was similar whatever the plasmid set was.

### Fusion gene expression levels in diagnostic leukemia samples

Inventors compared 55 paired BM and PB samples taken at the time of diagnosis. Two additional pairs in MLL-AF4 network were not analysed due to insufficient number. The statistical analysis revealed that sample source had no significant impact upon the expression level at diagnosis of the relevant FG expressed as NCN, except for PML-RARA using B2M or GUS as the CG. These analyses would suggest that either source of diagnostic material could act as a suitable reference for MRD studies. Overall, median NCN of each FG transcript was variable, ranging from 8.5 copies (E2A-PBX1) down to 0.1 copy (SIL-TAL1) per copy of ABL gene transcript (Figure 33a). Such result was also observed with B2M and GUS CG. Relative expression levels of the different FG were generally consistent irrespective of the CG used for normalization (Figure 33b,c), although some heterogeneity was observed in a few cases (see PML-RARA, AML1-ETO and SIL-TAL1 in the Examples section). In most cases, NCN of the FG transcript in patient samples were comparable to those observed in the corresponding cell line controls (see each example). These results were corrected according to the blast cell proportion in the sample when the information was available.

### RNA dilution series in E. Coli RNA

FG positive cell line or patient RNA was diluted in E. coli RNA, using ten-fold dilution steps (10⁻¹ to 10⁻⁶, see Material and Methods section). A concordance was observed in the limiting dilution experiments between the last positive dilution of the cell line or patient RNA and the sensitivity predicted on the basis of FG and CG levels in the corresponding undiluted RNA. These data indicated that the quantification of the CG and the FG with CN in the pure sample was correct. A linear regression analysis on the same dilutions showed that the FG/CG ratio did not change significantly (less than a factor two) over 3 to 5 logarithmic dilutions depending on the FG expression level in the pure sample. These results indicated equal RQ-PCR amplification efficiencies for the FG and the CG on a large dilution range.

### Expression of RQ-PCR data

MRD monitoring by RQ-PCR analysis is becoming a tool for decision making in multi-center therapeutic trials. For this reason, it is of capital importance for RQ-PCR results to be expressed in an uniform way. The majority of publications used a copy number ratio between the FG and the CG with a standard curve, this ratio being expressed as a decimal value or a percentage. To obtain the standard curve, laboratories used either cell line Cdna or plasmid DNA. So far, few authors used the ΔΔCt method and, to inventors knowledge, a standard curve of diagnostic cDNA has not been used so far.

Four possibilities were discussed : 1) cell line RNA dilutions, 2) percentage of positive cell number relative to the diagnostic sample, 3) copy number ratios and 4) the ΔΔCt method.
1) The cell line RNA dilutions appeared to be very sensitive to degradation during transportation as shown in inventors study. The variability of expression for one cell line can be subject to large variations. Such potential variation depends on the source of the cell line, the timing of cell culture when RNA extraction has been done, and finally the RT efficiency. For multicenter studies the option to overcome such difficulties could be to centrally prepare and distribute the cDNA.
2) Results expressed as frequency of positive cells would have the huge advantage to allow direct comparison with other MRD techniques. Furthermore, it would enable more reliable determination of kinetics of FG transcript reduction within individual patients, given the variability in FG transcript expression levels between patients as observed in this study. However, such an approach is dependent upon availability of diagnostic material against which relative levels of MRD can be judged. Since the precise level of FG expression and its variations during treatment at the single cell level are not entirely clear, inventors ultimately decided to express results in terms of ratios between the target (FG transcript) and the reference (CG transcript) in inventors experiments.
3) The ratio (NCN) was expressed as FG copies per copy of ABL or GUS gene transcript and per 100 copies of B2M gene transcript due to its high expression level. This ratio should be independent of the starting RNA quantity. For this purpose, inventors used plasmid standard curves, which offer the possibility to directly quantify the copy number of the transcripts. Inventors data show that plasmids are suitable calibrators for inter-laboratory or intra-laboratory normalization of RQ-PCR analysis. Plasmid DNA is probably a good option providing stability and robustness which is unlikely to be achieved when using large-scale production of cDNA as a potential QC material.
   The potential drawbacks of this method are: Firstly, the risk of contamination, although inventors used plasmid dilutions containing FG copies within the same range as patient samples. Usual rigorous precautions for PCR analysis are always required for limiting this risk. Secondly, the use of plasmid calibrators reduces the number of wells available for patient samples and slightly increases the cost. Thirdly, the calibrators introduce additional steps/calculations potentially increasing the variability. Finally, DNA plasmids do not directly assess the RT efficiency; but the CG expression level in patient samples clearly represents a control of the pre-PCR steps.
4) The ΔΔCt method (Applied Biosystems User's bulletin #2) does not have these disadvantages but has its own limitations. The method relies on the relative efficiencies of the FG and CG assays being comparable and consistent from plate to plate; therefore it is critical that positive RNA or cDNA standards are routinely included, to enable deterioration in assay performance to be detected by a rise in Ct value as encountered once in inventors study for the analyzis of 70 CML samples, including 17 paired samples. This method can be very efficient in expert laboratories and can be used to determine relative level of MRD in comparison to the diagnostic sample. There are concerns that this approach may not lend itself to assessment of inter-experimental variations in the intra or inter-laboratory setting. This may create difficulties in comparing RQ-PCR data between different groups, particularly when different machines are used (there are at least eight providers today).

### Proposal for assessing the sensitivity level of RQ-PCR experiments based on EAC data

### Background

When one encounters an absence of FG transcript amplification in a patient sample during follow-up, it is necessary to assess the detection limit for the particular assay to determine the reliability and clinical relevance of the result obtained. To address this issue, inventors propose two formulae to calculate the sensitivity level of a given experiment. The use of copy number values will be reported hereinafter.

### Calculation

The formula is based on the results of E. coli dilution experiments and the correlation between FG CN and CG CN with a slope close to 1 in diagnostic samples (see each particular example). In this formula, the sensitivity is directly related to the NCN of the FG at diagnosis and the CG CN of the sample. Ideally, the calculation should be based upon the patient's diagnostic NCN, after correction for blast percentage. If not available, EAC data can be used. In this model, 10 copies of the FG plasmid should be amplified for any particular fusion transcript. If only 100 copies can be amplified, the sensitivity should be reduced by one log₁₀. $SENS = - {log}_{10} \left(NCN\right) - {log}_{10} \left(CG CN\right)$

In this formula, SENS is the sensitivity (log₁₀) of the experiment for the diagnostic sample and should be expressed as 10^{SENS}. NCN is either the ratio of the patient sample at diagnosis or if not available the corresponding median NCN from the EAC data.

The formula is valid for all CG at diagnosis but one should be aware of the bias towards underestimation for BCR-ABL / ABL ratio for samples containing high level of leukemic cells. Although, only the ABL gene did not show any significant difference between BM and PB and between normal samples and leukemic samples at diagnosis. Thus this formula can be used only with ABL as CG without any correction for assessing the sensitivity level of the experiment during the follow-up.

### Three illustrations through real cases

### - At diagnosis

Patient A presents a pediatric T-ALL at diagnosis. The search of SIL-TAL1 FG transcript in its PB sample remains negative. The quantification of ABL gene transcript using RQ-PCR with inventors EAC protocol is 46000 copies. Thus the estimated sensitivity of the experiment based on EAC data for the median SIL-TAL1 FG transcript expression in PB (0.09, Table 22) at diagnosis is: $SENS = - {log}_{10} \left(0.09\right) - {log}_{10} \left(46000\right) = - 3.6 \left(or {10}^{- 3.6}\right)$

### - At relapse

Patient B presents a late relapse of a TEL-AML1 positive precursor-B-ALL. The quantification of TEL-AML1 FG transcript in its PB sample is 419000 copies. The quantification of ABL gene transcript in the same sample is 17000 copies. The TEL-AML1/ABL ratio for this patient is 25 (419000/17000). Thus the estimated sensitivity based on TEL-AML1 FG expression in this patient is: $SENS = - {log}_{10} \left(25\right) - {log}_{10} \left(17000\right) = - 5.6 \left(or {10}^{- 5.6}\right)$

### -During follow up

The same patient B is followed 3 months later by RQ-PCR for the detection of TEL-AML1 FG transcript in PB and BM samples. TEL-AML1 FG transcript is not detected by RQ-PCR in both samples. Results of ABL gene transcript quantification on the PB and BM samples are respectively 7700 and 13700 copies. Based on the observation that TEL-AML1 NCN does not differ significantly between PB and BM (Figure 11), the ratio in this patient at relapse (419000/17000) is used to calculate the sensitivity of this experiment: $SENS \left(BM\right) = - {log}_{10} \left(419/17\right) - {log}_{10} \left(13700\right) = - 5.5 \left(or {10}^{- 5.5}\right)$ $SENS \left(PB\right) = - {log}_{10} \left(419/17\right) - {log}_{10} \left(7700\right) = - 5.3 \left(or {10}^{- 5.3}\right)$

Compared to classical RT-PCR FG transcript follow-up, the sensitivity in this case relies on patient and not on cell lines samples. The sensitivity threshold calculated with this methodology is clearly more accurate than the one based on classical RT-PCR on cell line dilutions (5).

The invention is further illustrated by the following non limiting examples.

### EXAMPLES

### Example 1. t(1;19) (q23;p13) with the E2A-PBX1 fusion gene transcript

### 1.1 Background

The leukemogenic FG transcript E2A-PBX1 results from fusion of the E2A and PBX1 (formerly prl) genes, through the t(1;19)(q23;p13). The t(1;19)(q23;p13) is found in 3-5 % of childhood and in 3 % of adult precursor-B-ALL. In 95 % of cases, E2A-PBX1 transcripts are expressed. This expression is tightly associated with detection of cytoplasmic Ig µ chains. The remaining t(1;19) precursor-B-ALL are E2A-PBX1 negative and show no rearrangement of the E2A gene.

The E2A gene, located on chromosome 19, encodes the helix-loop-helix Ig enhancer binding factors E12 and E47 and the PBX1 gene on chromosome 1 encodes a DNA binding homeobox protein. The genomic organization of E2A is well-defined and breakpoints occur almost exclusively in the 3.5 kb intron between exon 13 and 14. The genomic organization of PBX1 is not yet fully known and the breakpoints are dispersed over an intronic region of about 50 kb between exon 1 and 2. The majority of cases with E2A-PBX1 FG transcripts show a constant junction of E2A exon 13 to PBX1 exon 2 (Figure 3). A variant FG transcript has been described in about 5-10 % of E2A-PBX1 positive patients. It is characterized by insertion of a stretch of 27 nucleotides at the E2A-PBX1 junction. This inserted sequence probably arises from an alternatively spliced exon of PBX1.

The FG encodes a chimeric transcriptional activator containing the N-terminal transcriptional activator domain of E2A joined to the C-terminal DNA-binding homeobox domain of PBX1. The transforming activity of E2A-PBX1 proteins has been demonstrated both in vitro and in vivo.

Several studies using RT-PCR amplification of E2A-PBX1 FG transcripts to assess MRD have been reported. All of them were performed with a qualitative assay showing a detection threshold of up to 10⁻⁴/⁻⁵. In none of these reports does the presence or absence of E2A-PBX1 transcripts during follow-up predict treatment outcome. The largest series including 71 patients, found no difference in event-free survival between PCR-positive and PCR-negative patients analyzed at the end of consolidation treatment. All these studies pinpoint to the limitations of qualitative assessment of MRD in monitoring t(1;19) positive ALL patients, and underline the importance of quantitative approaches such as RQ-PCR methods.

### 1.2 EAC data

### 1.2.1 Primer design and optimization (phases I and II)

Among two primer and probe sets tested, one was chosen: ENF101, ENR 161 and ENP 141. Positions and nucleotide sequences are shown in Figure 3 and in Table 5, respectively. This set was selected on the basis of higher ΔRn values at the plateau phase with high dilutions of the E2A-PBX1 positive cell line 697 (also known as ACC42) RNA in HL60 RNA. During phase II and phase III experiments, this set of primers and probe allowed us to detect a 10⁻⁴ dilution of 697 RNA in HL60 RNA in seven out of seven laboratories; an example of typical amplification plots (10⁻¹, 10⁻³, 10⁻⁴ cell line RNA dilutions) are shown in Figure 4. It was concluded that ENF101, ENR 161 and ENP 141 are suitable for quantification of all E2A-PBX1 fusion transcripts, including the variant form.

### 1.2.2 E2A-PBX1 expression in 697 cell line and diagnostic patient samples (phase IV)

One cell line, 697 and 27 diagnostic samples, (14 BM and 13 PB samples), were analyzed. Blast percentages (defined morphologically) were available in all but two samples (1 PB and 1 BM) and ranged from 74 to 100 % (median = 96 %). Median values and 95 % range for control genes and E2A-PBX1 Ct values, as well as normalized E2A-PBX1 copy number (corrected according to the blast percentage), are reported in Table 6. Ct values detected for E2A-PBX1 and CG in the 697 cell line and patient samples were comparable indicating that they are expressed at similar levels. The highest correlation coefficient was observed between E2A-PBX1 and ABL transcripts. Among PB and BM samples, ten were paired samples, harvested in the same patient at presentation of the disease. No statistically significant difference could be observed between BM and PB, in terms of Ct or NCN in paired samples (Figure 5).

### 1.2.3 QC rounds (phases IIIa to IVa)

During the various QC rounds, 11 negative samples (five negative RNA, three NAC and three NTC) and five positive samples (10⁻³ (2 samples) and 10⁻⁴ (3 samples) dilutions of 697 cell line RNA in HL60 RNA) were tested in eight to 10 labs (Table 7). E2A-PBX1 amplification of the negative samples accounts for 156 wells. Three wells (corresponding to 3 different samples) were found positive (2 %), but none of these samples were considered positive according to the criteria defined in the Material and Methods section. E2A-PBX1 amplification of the positive samples accounts for 78 wells. Only one well was found negative (1 %). According to the criteria defined in the Material and Methods section, all five positive samples were found positive, as expected from the sensitivity threshold defined in previous phases.

### Example 2. t(4;11) (q21;q23) with the MLL-AF4 fusion gene transcript

### 2.1 Background

The t(4;11) (q21;q23) is the most frequent 11q23 translocation in precursor-B-ALL and involves MLL (HRX, Htrx, ALL1) and AF4 (FEL) genes. While MLL-AF4 positivity is observed in 5% of pediatric and adult ALL cases, this subgroup accounts for 40 to 60% of infant and therapy induced ALL. The function of mammalian MLL is still largely unknown, but it seems to play a central role in segmentation during development. The recent mouse AF4 knock out suggested that this gene encodes a putative transcription factor which is involved in the ontogeny of the lymphoid lineage. Increased resistance of MLL-AF4 positive leukemia to stress induced cell death shown in vitro has been suggested to contribute to their poor prognosis.

At the molecular level, breakpoints in MLL and AF4 genes are spread within introns, between exon 8 and 12 (MLL) and exon 3 and 7 (AF4), some transcripts being more frequent in either adult or infant ALL.(5) It was reported that using a nested RT-PCR technique with 10⁻⁴ sensitivity, low expression levels of MLL-AF4 transcripts in up to 13 % of pediatric ALL at diagnosis, some of them being negative for the MLL-AF4 rearrangement by Southern analysis, and in around 25 % of fresh normal BM or fetal liver samples. Based on these data, the authors suggested that RT-PCR assays for the MLL-AF4 FG transcripts were not suitable for MRD monitoring. However, these remarkable findings have not been confirmed so far by other groups or by other techniques.

Nevertheless, in the same period, the first prospective MRD study, using nested RT-PCR, on 25 MLL-AF4 positive patients showed a significant correlation between PCR positivity, relapse and survival. The heterogeneity of the MLL-AF4 FG transcripts, their relatively low incidence in childhood and adult ALL and their poor prognosis with classical therapy explain the scarce number of reported MRD studies for this RT-PCR target.

### 2.2 EAC data

### 2.2.1 Primer design and optimization (phases I and II)

From the three probes and six primer sets tested, a common probe ENP242 and a common reverse primer ENR262, both located on AF4 exon 5, were adopted (Figure 6 and Table 8). Due to the large variety of MLL-AF4 FG transcripts, a common primer/probe set for all MLL-AF4 variants could not be designed. Two forward primers on the MLL gene were used in order to amplify at least 90% of non-infant and 60 % of infant MLL-AF4 FG transcripts: ENF207 (exon 9) and ENF208 (exon 10) (5). To inventors knowledge, no primer/probe sets for RQ-PCR have been published to date for MLL-AF4 or any other MLL FG transcripts.

Three cell lines were available for testing: RS4;11 (MLL exon 10-AF4 exon 4), MV4-11 (MLL exon 9-AF4 exon 5) and ALL-PO expressing two alternative transcripts (MLL exon 10 and 11-AF4 exon 5). Three plasmids were constructed: MLL-AF4 exon 9-exon 5, exon 10-exon 4 and exon 11-exon 5. An example of typical amplification plots (10⁻¹, 10⁻³, 10⁻⁴ cell line RNA dilutions) are shown in Figure 7; inventors sensitivity data on cell line material were comparable to those previously observed for detection of MLL-AF4 FG transcripts using nested PCR assay (5). The "creeping-curve" phenomenon was a rare event within the negative samples and mostly laboratory related (see Material and Methods section and Example 9).

### 2.2.2 MLL-AF4 expression in cell lines and diagnostic patient samples (phase IV)

Among the 22 samples included (14 BM and eight PB, including two paired samples), most of them (n=19) were recruited from the French therapeutic protocols LALA-FRALLE for ALL and tested in Marseille. The blast cell percentage in the samples was not known for all the samples; so results were not corrected according this proportion. The normalized MLL-AF4 FG transcript expression (NCN) appeared to be similar between cell lines (n=3) and patients (Table 9). No differences in MLL-AF4 NCN were observed between PB and BM samples (Figure 8). The 95 % range of expression was reduced when using either ABL or GUS as CG (Table 9) compared to the results obtained with B2M or without any CG. Furthermore, the correlation between MLL-AF4 and ABL transcript levels was the highest observed among the three control genes tested in diagnostic samples (Table 9).

### 2.2.3 QC rounds (phases IIIa to IVa)

Few false negative samples (6 %, 7/110) were observed for 10⁻³ and 10⁻⁴ dilutions. The amplification of MLL-AF4 cDNA within negative samples (FG negative samples, NAC and NTC) also called false positivity was limited to 3 % (5/176) and restricted to individual laboratories (Table 10). The Ct value in the false positive wells was always more than 30 and most of the time higher than 37.

### Example 3. t(12;21) (p13;q22) with the TEL-AML1 fusion gene transcript

### 3.1 Background

TEL(ETV6)-AML1(CBFA2,RUNX1) FG transcripts results from the cryptic t(12;21)(p13;q22) and is found in about 25 % of childhood precursor-B-ALL. Both TEL and AML1 encode nuclear transcription factors, which are critical for normal hematopoiesis. Their fusion lead to leukemogenesis by disrupting the normal function of TEL and/or creating a transcriptional repressor that impairs AML1 target gene expression. Two recurrent translocation breakpoints have been described. The major one breaks within TEL intron 5 and AML1 intron 1, generating TEL exon 5-AML1 exon 2 FG transcripts. The minor one is found in about 10 % of TEL-AML1 positive ALL and breaks within AML1 intron 2, generating TEL exon 5-AML1 exon 3 FG transcripts which are 39 bp shorter (Figure 9). This latter FG transcript, together with transcripts lacking AML1 exon 3 are also detected in cases with AML1 exon 2 fusion, as a result of alternative splicing. Cases with breakpoints in TEL intron 4 have been described but remain exceptional.

The prognosis of TEL-AML1 positive ALL is still controversial. There is agreement that presence of the TEL-AML1 FG transcripts is associated with a high probability of 4 year-event free survival. However, some authors, but not others, reported relapse rates similar to those of ALL in general, with a majority of relapses occuring off-therapy. Whether these variations from one study to another are due to methodological bias or differences in efficacy of chemotherapy regimens is still unclear.

Few studies have reported MRD results for patients with TEL-AML1 positive ALL so far. Most of these studies relied on a qualitative or semi-quantitative evaluation of the transcript level. The high frequency of TEL-AML1 transcript positivity in precursor-B-ALL prompted several groups to develop quantitative RT-PCR strategies targeted on this transcript to follow MRD. Preliminary data show that MRD is still detectable after induction therapy in 40 to 50 % of patients, and that high MRD levels are found in some patients. However, despite the relatively high frequency of the TEL-AML1 fusion, only small series of patients have been analyzed so far (always less than 30 patients), and the rarity of relapses in addition to their possible late occurrence made it difficult to make any clinical correlations so far.

### 3.2 EAC data

### 3.2.1 Primer design and optimization (phases I and II)

The cell line used for testing was REH,93 which displays a TEL exon 5-AML1 exon 2 fusion. Two plasmid constructs were also used, one containing the "long transcript" (TEL exon 5-AML1 exon 2), and the other containing the "short transcript" (TEL exon 5-AML1 exon 3) (See Materials and Methods section).

At the end of the optimization phase, one primer /probe set (ENF 301 on TEL exon 5, ENR361 on AML1 exon 3 and ENPr341 on TEL exon 5) was selected from the five sets that were initially tested (Figure 9 & Table 11). An example of typical amplification plots (10⁻¹, 10⁻³, 10⁻⁴ cell line RNA dilutions) are shown in Figure 10; the optimized RT and PCR conditions permitted us to detect a 10⁻⁴ dilution of the REH cell line in PBL RNA and 10 copies of plasmids in 100 % of cases, which is consistent with results obtained previously in the BIOMED-1 Concerted Action (5). It has to be noticed that molecular variants such as transcripts with a TEL-exon 4 fusion remain undetected using this set of primers.

### 3.2.2 TEL-AML1 expression in REH cell line and diagnostic patient samples (phase IV)

TEL-AML1 expression was studied in the REH cell line and in 57 TEL-AML1 positive precursor-B-ALL, 30 BM and 27 PB samples, including 23 paired samples (Table 12). Samples contained 18% to 100 % leukemic blasts. A majority of these samples were obtained from patients of Hôpital Saint-Louis (Paris, FR). NCN were calculated and adjusted for the percentage of blasts present in each sample.

TEL-AML1 expression in the REH cell line was within the range detected in primary leukemia samples.

No significant difference for ABL NCN was observed in TEL-AML1 expression when comparing PB and BM samples collected at diagnosis in the same patients (Figure 11).

### 3.2.3 QC rounds (phases IIIa to IVa)

As expected from the sensitivity threshold defined in previous phases, 10⁻³ and 10⁻⁴ RNA dilutions of the REH cell line were always found to be positive (Table 13) according to the criteria defined in the Metrial and Methods section. A 5.10⁻⁵ dilution was found to be positive in 7/7 laboratories. During the various QC rounds, 11 negative samples (six negative RNA and five NAC or NTC samples) were tested in 7 to 11 labs, corresponding to a total of 279 amplification wells (Table 13). Nine of these wells (3 %) were found falsely positive, corresponding to three false positive samples out of 93 (3 %) according to the criteria mentioned above. These false positive wells were observed in 6 different labs. No case of false positivity with 3 positive wells was observed and Ct values were always higher than 39. All false positive wells corresponded to negative RNA, while NAC and NTC were always negative.This observation suggests that these false positive results could be due to contaminations achieved prior to amplification, such as the RT step.

### Example 4. t(9;22) (q34;q11) with the BCR-ABL m-bcr fusion gene transcript

### 4.1 Background

The BCR-ABL FG is associated with formation of the Philadelphia chromosome (Ph) and is one of the most common genetic abnormalities detected in leukemias. In ALL, Ph is detected in 25-30 % of adult and 2-5 % of childhood cases. Less frequently, it is associated with acute myeloid leukemia. In the ALL subset, this genetic lesion is known to confer a very poor prognosis, and, consequently, its detection is important in planning aggressive therapies, including allogeneic bone marrow transplant. In addition, the Ph chromosome is found in more than 95 % of CML cases and is the hallmark of this disease. At the molecular level, the Ph chromosome or t(9;22) results in the juxtaposition of the 5' part of the BCR gene (chromosome 22) to the 3' part of the ABL gene (chromosome 9). In the vast majority of patients, the breakpoints in the BCR gene are clustered within three well defined regions : i) a 55 Kb sequence of the first intron, called the minor breakpoint cluster region (m-bcr), ii) a 5.8 Kb region spanning exons 12 to 16, called the major breakpoint cluster region (M-bcr), and iii) finally intron 19, called µ-bcr. Analysis of µ-bcr breakpoints will not be discussed further due to their extreme rarety. In the case of m-bcr breakpoints, the first exon of the BCR gene (e1) is juxtaposed to the second exon of the ABL gene (a2). The resultant fusion transcript (e1-a2) encodes a 190 Kda chimaeric protein (p190). This type of BCR-ABL FG is found in 65 % of adults and 80% of children with Ph positive ALL. Only in sporadic cases is the p190 encoding BCR-ABL gene found in CML.

Despite huge efforts, the molecular mechanisms by which the hybrid BCR-ABL protein gains transforming capability are still not fully understood. However, the BCR-ABL protein shows an increased and deregulated tyrosine kinase activity and it seems to deregulate the normal cytokine-dependent signal transduction pathways leading to the inhibition of apoptosis and by growth factor independent growth.

All the studies about the clinical value of MRD in Ph+ ALL patients indicate that BCR-ABL positive cells cannot be eradicated even by intensive chemotherapy. In a series of 36 Ph+ ALL patients treated by SCT, it was reported that RT-PCR assessment of MRD was, by multivariate analysis, the best prognostic indicator for continuous complete remission. Recently, it was reported the follow up by RQ PCR analysis of 13 m-bcr positive ALL patients. All these data suggest that, in Ph+ ALL patients, quantitative monitoring of residual leukemic cells could prove more valuable than their qualitative detection to assist in clinical decision-making.

### 4.2 EAC data

### 4.2.1 Primer design and optimization (phase I and II)

The efficiency of four different primer/probe sets, designed using the "Primer Express™" software, was tested in 1:10 serial dilution experiments of RNA from the TOM-1 cell line (e1-a2 junction or m-bcr) into RNA from HL60 cells. All primer/probe sets were free from non-specific amplification artifacts, but two sets were superior in terms of sensitivity and ΔRn value at plateau phase. Both sets had comparable amplification efficiencies and reached 10⁻⁵ sensitivity and ΔRn >3.0 at the 10⁻¹ cell line dilution. After extensive testing, the set that included ENF402 (located in BCR exon 1) ENR561 and probe ENP541, both located in ABL exon 2, were selected. Both the reverse primer and probe are common to the set used for the RQ-PCR detection of BCR-ABL (M-bcr) FG transcripts (Figure 12, Table 14). An example of typical amplification plots (10⁻¹, 10⁻³, 10⁻⁴ cell line RNA dilutions) are shown in Figure 13.

### 4.2.2 BCR-ABL m-bcr FG expression in TOM-1 cell line and diagnostic patient samples (phase IV)

To establish reference intervals of m-bcr transcripts, inventors determined FG expression in 17 BM samples and seven PB samples from sequential ALL patients at diagnosis as well as centrally prepared and distributed RNA from the TOM-1 cell line (Table 15). For the FG and the three CG transcripts assayed, separate series of plasmid dilutions were amplified in each experiment to calculate transcript copy numbers. Although samples with ABL Cts >29.3 were excluded from the analysis because they were not suitable for amplification, the Ct values of all transcripts were generally lower in cell lines than in patient material (both BM and PB), most probably due to low quality of some of the patient RNAs. However, after normalization to control gene expression, m-bcr transcript levels were comparable in patients and the TOM-1 cell line, and very similar in four paired BM and PB samples (Figure 14).

The primers and probe used to amplify ABL mRNA are also able to amplify BCR-ABL FG mRNA, and hence, the assay of ABL gene expression used to normalize data may be affected by the levels of the m-bcr transcript in the samples.

### 4.2.3 Quality control rounds (Phase IIIa to IVa)

Only 3/129 wells gave false negative results (see Materials and Methods for details) in the three QC rounds and in all cases the false negativity was obtained for 10⁻⁴ TOM-1 dilutions. Furthermore, false-positive results were detected in 5.3 % of PCR tests (13/246, Table 16).

### Example 5. t(9;22) (q34;q11) with the BCR-ABL M-bcr fusion gene transcript

### 5.1 Background

Most cases of CML are associated with the presence of t(9;22) resulting in a small derivative chromosome 22 known as the Philadelphia chromosome. As a consequence the ABL protooncogene on chromosome 9 is fused to the BCR gene on chromosome 22. In CML patients and approximately 35 % of Philadelphia-positive adult ALL patients the breakpoint on chromosome 22 is located between exons 12 to 16 of the BCR gene, in the so-called major breakpoint cluster region (M-bcr). The breakpoint on chromosome 9 is located in most cases between exons 1 and 2 in the ABL gene. The transcription product of this BCR-ABL FG is an 8.5-kb aberrant fusion RNA with two junction variants b2a2 and/or b3a2 that gives rise to the BCR-ABL chimeric protein (p210), a tyrosine kinase with deregulated activity. Rare cases with b2a3 and b3a3 BCR-ABL transcripts can be observed.

Because of its high sensitivity, qualitative RT-PCR has been extensively used to monitor residual disease in CML, yielding partially contradictory results. Sequential analysis of patients who received allogeneic BM transplantation (BMT) showed that repeated PCR positivity correlated with an increased risk of relapse. On the contrary, other studies did not find any correlation between PCR positivity and subsequent relapse and showed that long-term survivors of allogeneic BMT could be PCR positive even years after transplant without ever relapsing.

A competitive RT-PCR method to quantify the level of BCR-ABL FG transcripts was developed by several groups in an effort to improve the predictive value of BCR-ABL mRNA detection. The sequential analysis of patients that had undergone BMT showed that monitoring of BCR-ABL FG transcripts levels can be useful to predict an impending relapse while the patient is still in hematological and cytogenetic remission. Based on quantitative RT-PCR studies two groups have proposed to define a "molecular relapse" parameter. In addition, quantification of BCR-ABL FG transcripts has also proven useful to monitor response to α-interferon and Imatinib treated patients.

Despite many encouraging reports, monitoring of BCR-ABL FG transcripts with competitive RT-PCR has had limited clinical impact, partly due to the fact that this approach is difficult to standardize. Since the advent of real-time PCR several groups have published reports that describe the feasibility of monitoring CML patients with this technique. However, most RQ-PCR studies included too few patients or patients from different therapeutic protocols that together with methodological differences make it difficult to evaluate the clinical impact and to define general guidelines to monitor CML patients with RQ-PCR. The availability of a standardized protocol for RQ-PCR will facilitate data comparison among different centers, making it possible to define a threshold where a patient is likely to relapse and ultimately to assess the impact of an early therapeutic intervention based on the kinetics of BCR-ABL FG transcripts.

### 5.2 EAC data

### 5.2.1 Primer design and optimization (phase I and II)

Two alternative forward BCR primers, one located on BCR exon 13 (exon b2) and the second on BCR exon 14 (exon b3) and a reverse ABL primer and probe, both on the second exon of the ABL gene, were designed (Figure 12). This set, together with five comparable sets already available within the network, was evaluated for sensitivity as well as for robustness of the PCR reaction (see Material and Methods section). Based on these parameters, the set designed by the EAC group was chosen. Since dilution series of the K-562 cell line was amplified with equal efficiency with either of the two forward primers, one common forward primer ENF501, located on BCR exon 13 (exon b2), was selected to amplify both fusion variants BCR-ABL b3-a2 and b2-a2 (Figure 12, Table 17). An example of typical amplification plots (10⁻¹, 10⁻³, 10⁻⁴ cell line RNA dilutions) are shown in Figure 15.

### 5.2.2 BCR-ABL M-bcr expression in K-562 cell line and diagnostic patient samples (phase IV)

### - For CML, in K-562 and patients at diagnosis

The expression of BCR-ABL M-bcr transcripts was quantified in 29 CML patients in order to establish the range of FG expression levels in diagnostic samples (Table 18, Figure 16a). These samples included 15 BM and 14 PB samples. The expression of the BCR-ABL FG relative to the CG was very similar between BM and PB. No large variations in BCR-ABL M-bcr expression levels were found between individual patients. Expression of BCR-ABL mRNA in the K-562 cell line was found to be higher than CML diagnostic samples by two logs relative to B2M and one log relative to GUS gene (see Table 18).

### - In BCR-ABL M-bcr positive ALL

In addition to diagnostic samples from CML patients in chronic phase, BCR-ABL M-bcr expression was quantified in diagnostic BCR-ABL M-bcr positive ALL samples (Table 19, Figure 16b). Expression of BCR-ABL relative to B2M and GUS was found to be higher in ALL samples compared to CML samples (Tables 18 and 19). Inventors observed a statistical difference in the FG level of expression between M-bcr ALL and CML patients either with B2M or GUS as CG (p=0.001), while no difference was observed between m-bcr and M-bcr in ALL (Figure 17).

The primer set designed to amplify the ABL control gene is located on exon 2 and also amplifies the BCR-ABL FG. For this reason, the use of ABL as a control gene could introduce a bias for quantifying BCR-ABL in CML and Ph+ ALL samples when a large proportion of the cells express BCR-ABL. Using the ratio (BCR-ABL/ABL) would theoritically lead to an underestimation of the tumor load in these samples since the maximum ratio is one. However, this bias had a minor impact on relative quantification of FG transcripts at diagnosis (see below). Inventors found values up to 3.0 in BM and up to 4.4 in PB samples of CML patients at diagnosis (Table 18), although all median BCR-ABL/ABL ratios were below 1, except for PB CML patients at diagnosis (Tables 15 and 18). These unexpected results obtained with plasmid calibrators were confirmed without calibrators (ΔCt method) and clearly illustrate the limits of accuracy of gene transcript quantification by RQ-PCR. Similar results were observed in a oligocenter context.

### 5.2.3 Quality control rounds (Phase IIIa to IVa)

The percentage of false negatives was 4.9% (14/285) for the first and second quality control rounds (Table 20). The laboratories that showed the false negatives had a consistent reduction in sensitivity for all the targets in a particular phase, which indicated that the cause for the lower sensitivity was a lower RT efficiency rather than a PCR-related problem.

In the third quality control round (phase IVa), a rate of false positivity (5.5%, 6/110) similar to the previous phases was observed despite a particularly high frequency (18%, 4/22) of false positive results within FG negative samples (Table 20). This observation is possibly explained by the inclusion of the undiluted K-562 RNA among the coded samples instead of the 10⁻¹ dilution and thereby increasing the risk of accidentally contaminating neighbouring wells when pipeting the cDNA onto the PCR plate. It should be noted that the majority of the false positive samples (NAC/NTC) were concentrated in individual laboratories while the rest of the laboratories (one laboratory per QC round) showed only occasionally single false positive wells or no false positives.

### Example 6. Intra-chromosomal microdeletion on 1p32 with the SIL-TAL1 fusion gene transcript

### 6.1 Background

The microdeletion on 1p32 is the most frequent chromosome aberration found in childhood T-ALL. The microdeletion involves the TAL1 gene (T cell acute leukemia 1 gene, also known as stem cell leukemia (SCL) or T cell leukemia gene 5 (TCL5)) and the SIL gene (SCL interrupting locus), which is located approximately 90 kb upstream. As a result, the TAL1 coding sequences are placed under the control of the SIL promotor, which is expressed in T cells, and consequently the TAL1 gene becomes ectopically expressed in the involved T-ALL.

The TAL1 gene, in particular exon 4, 5, and 6, encode a 42 kDa protein, which is a basic helix-loop-helix (bHLH) transcription factor. The TAL1 protein can heterodimerise with other bHLH transcription factors, including members of the E2A family, and is an essential factor for the development of all hematopoietic lineages. The SIL gene is a member of the immediate-early gene family, but its function in hematopoietic cells is not yet well defined.

Although both the SIL and TAL1 genes contain several conserved deletion breakpoints, most cases (≥ 95 %) involve the sildb1 breakpoint in combination with the taldb1 or taldb2 breakpoint. By alternative splicing, three different SIL-TAL1 transcripts can be formed, of which the type II transcript is the most predominant one. There is no apparent relationship between the occurrence of SIL-TAL1 transcripts and prognosis or outcome.

SIL-TAL1 transcripts are exclusively found in T-ALL, in which they are present in 5-25 % of the patients. The frequency is related to the immunophenotype of the T-ALL (the presence of the SIL-TAL1 FG is restricted to CD3- and TCRαβ+ T-ALL) and the occurrence of TCRD gene deletions. The SIL-TAL1 FG transcripts seem to be more frequent in children as compared to adults.

Detection of TAL1 deletions at the DNA level has already been described. A recent report described a TaqMan-based RQ-PCR method for the detection of TAL1 deletions at the DNA level in T-ALL patients. In that report, the forward primer and probe were positioned in SIL exon 1b (and part of the following intron) and the reverse primer was located in TAL1 exon 1b. Using the CEM cell line, a sensitivity of 10⁻⁵ could be obtained, which is equivalent to a single leukemic genome. To inventors knowledge, no RQ-PCR primers/probe sets for SIL-TAL1 FG transcripts have been published so far.

### 6.2 EAC data

### 6.2.1 Primer design and optimization (phases I and II)

Initially, three TaqMan probes (located in SIL exon 1a, TAL1 exon 4, and TAL1 exon 5), two forward primers (both in SIL exon 1a) and five reverse primers (two in TAL1 exon 3, two in TAL1 exon 4, and one in TAL1 exon 6) were tested for specificity and efficiency.

A single forward primer (ENF601 ; located in SIL exon 1a), reverse primer (ENR664 ; located in TAL1 exon 3), and probe (ENP641 ; located in SIL exon 1a) were selected (Figure 18 and Table 21). An example of typical amplification plots (10⁻¹, 10⁻³, 10⁻⁴ cell line RNA dilutions) are shown in Figure 19.

The selected primer/probe set will detect virtually all SIL-TAL1 transcripts (the most common type II as well as type III), but will not detect TAL1 translocations or "aberrant" expression of the TAL1 gene without apparent rearrangements.

### 6.2.2 SIL-TAL1 expression in cell lines and diagnostic patient samples (phase IV)

Undiluted RNA of six different SIL-TAL1-positive cell lines were tested in duplicate for CG expression (ABL, B2M, and GUS) and in triplicate for SIL-TAL1 FG expression (Table 22). In three laboratories a total of sixteen SIL-TAL1-positive patients at diagnosis were also included (10 BM and 10 PB samples, including five pairs).

Ct values for the CG transcripts were significantly lower in the cell lines as compared to the patient samples (Table 22), which was may be due to the fact that stored patient samples were used or alternatively that their expression is higher in cell lines than in primary patient samples. SIL-TAL1 transcript expression (Ct, CN and NCN) was comparable between the six cell lines tested, but in patients a slightly larger variation in SIL-TAL1 FG transcript expression was found (Table 22). Nevertheless, the normalized SIL-TAL1 FG transcript expression did not differ between cell lines and patients. Furthermore, comparison between BM and PB samples (including five pairs) showed that SIL-TAL1 transcript expression was similar in both compartments (Figure 20).

### 6.2.3 Quality Control rounds with blind samples (phases IIIa to IVa)

False positivity was observed in two out of 46 FG negative samples (4.3 %) and in none out of 162 NAC/NTC wells (0 %) resulting in a total false positivity of 1.0% (2/208). False-negativity was only observed in the first QC round (phase IIIa), but was absent in the next two QC rounds (Table 23). Therefore, false-negativity does not seem to be a problem, although this may be dependent on the level of SIL-TAL1 FG transcripts in the leukemic cell sample.

### Example 7. t(15;17) (q22;q21) with the PML-RARA fusion gene transcript

### 7.1 Background

The PML-RARA FG transcripts, which are the molecular result of the t(15;17)(q22;q21) translocation, are associated with the majority of APL cases, a distinct AML subset with M3 cytomorphology.

APL accounts for 10-15 % of de novo AML in younger adults in Southern Europe. Among pediatric patients the incidence of APL is usually considered to be lower and accounting for 3-9 %, although published data from Italian cooperative studies indicate that APL occurs in Italian children with the same incidence as observed in adults. Moreover, several small series from different countries in Central and South America have noted a higher-than expected frequency of pediatric APL.

The two genes fused in the t(15;17) are PML, located on chromosome 15 and the retinoic acid receptorα (RARA) gene on chromosome 17. Other genes have been shown to be fused to RARA in rare instances of morphological APL cases negative for the t(15;17), such as PLZF on chromosome 11q23, NPM on 5q35, NUMA on 11q13 and STAT5B on 17q21.

The chimeric PML-RARA protein is a transcriptional repressor. In the absence of ligand (retinoic acid, RA), it binds DNA together with co-repressors such as SMRT (silencing mediator for RAR and TR) and N-CoR (nuclear receptor co-repressor) and renders chromatin inaccessible to transcriptional activators or basal transcription machinery.

RARA breakpoints always occur in intron 2 which is 17 Kb in length (Figure 21). By contrast, three regions of the PML locus are involved in the t(15;17) translocation breakpoints : intron 6 (bcr1 ; 55 % of cases), exon 6 (bcr2 ; 5 %), and intron 3 (bcr3 ; 40 %) (Figure 21). As a consequence, there are three possible PML-RARA isoforms, referred to as long (L, or bcr1), variant (V, or bcr2) and short (S, or bcr3). It should be noted that the size of the PCR products varies in bcr2 positive cases, because of the variable breakpoint positions in exon 6 of the PML gene and inclusion of a variable number of RARA intron 2-derived nucleotides in the FG transcript. Chimeric PML-RARA and RARA-PML transcripts are formed as a consequence of the reciprocal translocation between the PML and RARA loci. However, the observation that RARA-PML FG transcripts are present in most but not all APL cases, has favored the use of PML-RARA FG transcripts as PCR target for detection of APL cells at diagnosis and during monitoring. Standardized conditions for RT-PCR analysis of PML-RARA FG transcripts have been developed by the BIOMED-1 Concerted Action (5). Primer sets have been designed that allow the detection of the various PML-RARA FG transcripts, generated by the existence of different PML breakpoint regions as well as the presence of alternative splicing between central exons of PML.

In the last decade, the availability of differentiation therapy with all-trans retinoic acid (ATRA) has produced a remarkable improvement in the outcome of patients with APL. The challenge is how to identify the relatively small subgroup of patients at particular risk of relapse who cannot be reliably distinguished on the basis of pre-treatment characteristics and who could potentially benefit from more intensive treatment in first remission. Overall, there is general agreement that a positive PML-RARA test after consolidation is a strong predictor of subsequent hematological relapse, whereas repeatedly negative results are associated with long-term survival in the majority of patients. One group reported that recurrence of PCR positivity, detected by 3-monthly BM surveillance marrows performed after completion of therapy, was highly predictive of relapse. Using such a strategy, approximately 70 % of relapses were successfully predicted. A different perspective in the application of MRD to identify APL patients at higher risk of relapse has been used by the MRC ATRA trial, where the kinetics of achieving a molecular remission was evaluated. Finally, the benefit of early treatment at the time of molecular relapse has still to be proven, but preliminary evidence supports such a strategy.

Among the different methods (conventional karyotyping, FISH and PML immunostaining with specific antibodies), RT-PCR detection of the PML-RARA FG transcripts appears to be the only approach suitable for MRD detection (5). Moreover, quantitative PCR could provide information on the correlation between different levels of disease at early phases of therapy and clinical outcome. However, there have been relatively few studies reporting the use of RQ-PCR in APL patients.

### 7.2 EAC data

### 7.2.1 Primer design and optimization (phases I and II)

One probe and two reverse primers on RARA gene exon 3 in combination with seven forward primers on the PML gene were evaluated. Five forward primers were designed in PML exon 6, two and three specific primers for bcr1 and bcr2 breakpoints respectively, while two primers for bcr3 were designed in PML exon 3. Based on published data on the localization of bcr2 PML breakpoints, the respective forward primers on PML exon 6 were designed in order to cover at least 80 % of bcr2 cases. The only cell line available for testing was NB-4,165 which has a bcr1 PML breakpoint; for the evaluation of bcr2 and bcr3 primer/probe sets, diagnostic patient BM RNA was used. Plasmid constructs for all three PML-RARA breakpoint variants were made (See Materials and Methods section).

After extensive testing on cell line and patient RNA and plasmid dilutions, three specific primer/probe sets were selected, based on the maximum sensitivity: the probe RARA ENP942, the common reverse primer RARA ENR962, and three PML forward primers ENF903 (for bcr1), ENF906 (for bcr2) and ENF905 (for bcr3), respectively (Figure 21). Sequences are listed in Table 24. Although ENF906 can potentially be used to amplify bcr1 as well as bcr2 cases, direct comparison of ENF906 and ENF903 for amplification of PML-RARA in serial dilutions of the NB-4 cell line revealed the latter primer to provide a more sensitive assay for amplification of bcr1 cases. An example of typical amplification plots (10⁻¹, 10⁻³, 10⁻⁴ NB-4 RNA (bcr1) in PBL RNA) are shown in Figure 22.

### 7.2.2 PML-RARA expression in cell lines and diagnostic patient samples (phase IV)

PML-RARA expression was studied in the NB-4 cell line and in 16 positive AML-M3 bcr1 patients. (Table 25, Figure 23). Patient samples consisted of 14 BM and 9 PB samples, including six paired BM/PB samples, obtained at diagnosis. Samples contained 10 % to 100 % of leukemia blasts. Normalized copy numbers were calculated and adjusted for the percentage of blasts present in each sample. PML-RARA expression in the NB-4 cell line was within the range of FG expression detected in primary leukemia samples.

PML-RARA expression was studied in six bcr3-positive patients at diagnosis, consisting of six BM and four PB samples (including four paired BM/PB samples). Although the number was very limited, no significant difference was observed in PML-RARA bcr3 expression when comparing PB and BM on paired samples except for B2M normalized results (see Figure 23).

### 7.2.3 QC rounds (phases IIIa to IVa)

During the various QC rounds, 145 negative samples were tested in 7 to 11 labs during the three phases (Table 26). Five out of 100 NAC/NTC samples (5%) and five out of 45 FG negative samples (11%) were falsely positive for bcr1 amplification (Table 26). Overall, the frequency of false positivity was 6.9% (10/145). The so called false-positivity was limited to individual laboratories and the Ct value in the false positive well was always more than 30 and most of the time higher than 35.

By contrast, according to the criteria mentioned above, none false negative samples (n=96) for 10⁻³ and 10⁻⁴ dilutions were observed, neither for bcr1, bcr2 nor bcr3. None of the 42 wells tested independently for bcr2 and bcr3 at 10⁻⁴ dilution falsely resulted as negative. Only 8 out of 180 wells (4.4 %) tested for bcr1 at 10⁻⁴ dilution falsely resulted as negative.

### Example 8. Inv(16) (p13q22) with the CBFB-MYH11 fusion gene transcript

### 8.1 Background

Pericentric inversion of chromosome 16, inv(16)(p13q22), is found in about 8-9 % of newly diagnosed AML cases. The inv(16) positive AMLs are included with those with t(8;21) translocation in a group generally referred to as "Core Binding Factor" (CBF) leukemias, as both are characterized by rearrangements of genes that code for components of the heterodimeric transcription factor CBF, which plays an essential role in hematopoiesis. Inv(16) or the rarer t(16;16)(p13;q22) lead to fusion of the CBFB chain gene with the smooth muscle myosin heavy chain gene MYH11. The resulting FG mRNA can be detected by RT-PCR and represents a suitable molecular marker for both diagnostic and monitoring studies. So far, ten different CBFB-MYH11 FG transcripts have been reported. More than 85 % of positive patients have the type A transcript; type D and E transcripts each represent nearly 5 %, whereas all other types occur in sporadic cases. CBFB-MYH11-positive AML are usually considered to have a favorable prognosis, with more than 50 % of patients obtaining long-term CR. Such favorable results with conventional chemotherapy led some authors to consider that allo-BMT is not indicated to consolidate first CR in these patients, even when a suitable donor is available. Nevertheless, the relapse rate is still high indicating that reliable methods to detect MRD during hematologic CR are needed in order to better adapt the intensity of post remission therapy to specific cohorts of patients. So far, the use of qualitative RT-PCR-based methods employed to detect CBFB-MYH11 FG transcripts did not allow consistent discrimination of prognostic subgroups of patients in CR. In fact, the use of standard nested RT-PCR has produced conflicting MRD results: while in most reports the vast majority of patients in prolonged CR were found to be PCR-negative, a few long-term survivors never converted to RT-PCR negativity. Moreover, 10-20 % of PCR-negative patients eventually relapsed, suggesting that the achievement of PCR negativity is not synonymous with cure. Some of the difficulties in interpreting the above results may derive from lack of standardization of methodologies involved. Quantitative RT-PCR studies using competitive PCR or RQ-PCR enabled monitoring of the decrease in CBFB-MYH11 FG transcripts during early phases of induction and consolidation therapies. However, due to the low number of patients so far examined, it was not possible to define a kinetic or a cut off level for predicting relapse.

### 8.2 EAC data

### 8.2.1 Primer design and optimization (phases I and II)

During phase I, inventors tested six primer/probe sets: three for the A, two for D and one for the E form. As CBFB-MYH11 transcripts type A, D and E represent approximately 95 % of all cases, in order to amplify these transcripts inventors decided to use a common forward primer located on CBFB exon 5 (ENF803) and a common probe located on CBFB exon 5 (ENPr843). Three different reverse primers located respectively on MYH11 exon 12 for type A (ENR862), MYH11 exon 8 for type D (ENR863) and MYH11 exon 7 for type E (ENR865) (Figure 24 and Table 27) were chosen. An example of typical amplification plots (10⁻¹, 10⁻³, 10⁻⁴ RNA dilutions) are shown in Figure 25 for all three types of CBFB-MYH11 transcripts.

### 8.2.2 CBFB-MYH11 expression in the ME-1 cell line and diagnostic patient samples (phase IV)

Pure RNA of the ME-1 cell line (type A) was tested in eight different laboratories (Table 28). In addition, diagnostic BM or PB samples of 24 type A patients, three type D and four type E were analyzed (Figure 26). The values of FG transcripts at diagnosis were similar in all three categories of patients, but show a range of variation from patient to patient of one log.

### 8.2.3 QC rounds (phase IIIa to IVa)

No false negative results for the 10⁻³ dilution were observed, whereas at the 10⁻⁴ dilution a maximum of 12 % of false negative results were observed (Table 29). False positivity was absent during all phases in NAC and NTC wells (0%, n=104), whereas a single case (2 wells out of 16) of false positivity was observed during phase IVa in a coded FG negative sample and was due to contamination (Table 29).

### Example 9. t(8;21) (q22;q22) with the AML1-ETO fusion gene transcript

### 9.1 Background

The AML1(CBFA2, RUNX1)-ETO (MTG8) gene fusion results from the t(8;21)(q22;q22) which is the commonest chromosomal rearrangements associated with AML, being detected in approximately 8 % of AML cases in children and young adults. The AML1 gene encodes the α2 subunit of the heterodimeric transcription factor CBF (core binding factor) which is critical for hemopoietic development and whose β subunit is disrupted by the inv(16)/t(16;16) which leads to the CBFB-MYH11 FG (see Example 8).

As shown in Figure 27, AML1 breakpoints are located within intron 5, whilst ETO breakpoints occur upstream of exon 2. This gives rise to a single type of AML1-ETO FG transcript in which AML1 exon 5 is fused to ETO exon 2 (5), thereby simplifying molecular screening strategies and MRD monitoring as compared to FG with multiple breakpoint regions, such as t(4;11) with MLL-AF4, t(15;17) with PML-RARA and inv(16) with CBFB-MYH11.

AML1-ETO is an important PCR target for MRD detection in view of the generally favorable outcome of patients with the t(8;21), such that routine use of BMT in first CR has been shown to confer no overall survival benefit. Therefore, it is of paramount importance to identify the relatively small subgroup of patients at high risk of relapse who could benefit from additional therapy. However, the role of MRD detection in AML1-ETO positive AML has been somewhat controversial in view of the detection of FG transcripts in patients in long-term remission following chemotherapy, autologous BMT/PBSCT and even alloBMT. The detection of residual transcripts in patients who are cured of their disease has been seen as providing evidence that AML1-ETO alone is insufficient to mediate AML and has recently been shown to relate to a fraction of stem cells, monocytes and B cells present in remission marrow. Hence, the relatively frequent reports of PCR positivity in patients considered to be cured of t(8;21) positive AML is likely to reflect the higher levels of sensitivity commonly achieved for AML1-ETO RT-PCR assays (typically 1 in 105 to 1 in 106), as compared to those for other AML FG targets (typically 1 in 104-105). Despite the fact that a recent study has shown that conventional qualitative RT-PCR has the potential to provide an independent prognostic factor in AML1-ETO positive AML, there has been some concern regarding the suitability of sensitive "end-point" assays for MRD detection as a means of determining treatment approach in this subgroup of patients.

Over the last few years quantitative RT-PCR methods have been investigated to determine whether they can more reliably identify the relatively small subgroup of patients destined to relapse. Competitive RT-PCR assays have revealed variation in AML1-ETO expression relative to ABL between cases at diagnosis (10-fold in BM, 32-fold in PB) and suggest that AML1-ETO and ABL mRNAs have comparable stability. Furthermore, these studies revealed varying kinetics of FG transcript reduction following chemotherapy. Patients with low or undetectable levels of AML1-ETO transcripts were associated with maintenance of CCR, whilst high or rising transcript numbers predicted relapse. These promising preliminary data suggest that RQ-PCR is likely to be valuable for MRD monitoring in this subset of AML, with the added advantages that the latter technique is less labor intensive, more reproducible and amenable to standardization lending itself to use in large-scale clinical trials. Preliminary studies of RQ-PCR have essentially confirmed the results obtained via competitive RT-PCR, revealing a 3.5-20 fold variation in AML1-ETO FG expression levels in diagnostic BM that was not related to blast percentage and which needs to be taken into account when assessing response to therapy. Furthermore, variability in kinetics of response to chemotherapy was noted and interestingly, AML1-ETO transcripts were also detected in patients in long-term remission from AML. However, the predictive value of RQ-PCR remains to be established in large numbers of patients subject to a consistent treatment approach.

### 9.2 EAC data

### 9.2.1 Primer design and optimization (phases I and II)

Initially, the primer and probe sequences published by Marcucci et al (7) were tested together with two "in house" sets. Whilst the former primer/probe set was superior in terms of sensitivity, inventors observed significant recurrent background signals in the negative control samples. Inventors therefore decided to design two new probes compatible with this primer set leading to the selection of ENP747 positioned on the breakpoint, in conjunction with the forward primer ENF701 positioned on AML1 exon 5 and the reverse primer ENR761 on ETO exon 2 (Figure 27 and Table 30).(5) The cell line available for testing was KASUMI-1. The AML1-ETO RQ-PCR assay was found to be particularly robust, associated with a relatively high ΔRn (see Figure 28a). Reduction in the probe concentration from 200 nM to 100 nM was therefore evaluated. The decrease in probe concentration did not affect assay sensitivity and the "baseline creeping" artifact was only very rarely observed (Figure 28b).

### 9.2.2 AML1-ETO expression in KASUMI cell line and diagnostic patient samples (phase IV)

Undiluted RNA of the KASUMI-1 cell line was tested in five different laboratories (Table 31). Analysis for archived diagnostic RNA from 22 patient samples was undertaken by four different laboratories. All differences between BM and PB per control gene were not significant on paired samples (n=10, Wilcoxon test). After applying the exclusion criteria, 12 PB and 10 BM samples were evaluated (Table 31).

AML1-ETO FG transcript expression (Ct and CN) was higher in the KASUMI-1 cell line than in the patient samples (median difference of approximately two Ct values). Among the patient samples, no difference was seen in expression of the AML1-ETO FG transcript. With regard to the control genes, the expression of ABL was comparable between the cell line and patient samples. Significant variation was seen in the expression of B2M, both between patient samples and cell line and between BM and PB samples. For GUS, intermediate results were observed (Table 31 and Figure 29).

### 9.2.3 QC rounds (phases IIIa to IVa)

No false negative results (out of a total of 112 analyzed samples) for 10⁻³ and 10⁻⁴ dilutions were observed, which is in line with the good sensitivity of the RQ-PCR assay (Table 32). Overall, the frequency of false positivity was 9.7% (15/154). Six out of 108 NAC/NTC samples (5.6%) and nine out of 46 FG negative samples (20%) were falsely positive for AML1-ETO amplification (Table 32). In coded FG negative samples, false positivity was in most cases restricted to individual laboratories.

### List of Abbreviations

- ABL:: Abelson gene
- ALL:: acute lymphoblastic leukemia
- APL:: acute promyelocytic leukemia
- B2M:: beta-2-microglobulin
- BM:: bone marrow
- BMT:: bone marrow transplantation
- CBF:: core binding factor
- CG:: control gene
- CML:: chronic myeloid leukemia
- CN:: copy number
- COR. COEF.:: correlation coefficient
- CV:: coefficient of variation
- EAC:: Europe Against Cancer
- ENF:: European network forward primer
- ENP:: European network TaqMan probe
- ENPr:: European network reverse TaqMan probe
- ENR:: European network reverse primer
- FAM:: 6-carboxy-fluorescein
- FG:: fusion gene
- FISH:: Fluorescent in-situ hybridization
- GUS:: beta-glucuronidase
- MGB:: minor groove binding
- MMLV:: murine moloney leukemia virus
- MNC:: mononuclear cells
- MRD:: minimal residual disease
- MRDv:: minimal residual disease value
- NAC:: no amplification control
- NCN:: normalized copy number
- ND:: not done
- NTC:: no template control
- PB:: peripheral blood
- PBL:: peripheral blood lymphocyte
- PBMN:: peripheral blood mononuclear cells
- PBSCT:: peripheral blood stem cell transplantation
- PCR:: polymerase chain reaction
- QC:: quality control
- RQ-PCR:: real time quantitative polymerase chain reaction
- RT:: reverse transcription
- SENSv:: sensitivity value
- TAMRA:: 6-carboxy-tetramethyl-rhodamine

### List of References

1. Lowenberg B, Downing JR, Burnett A. Acute myeloid leukemia. N Engl J Med 1999; 341: 1051-1062.
2. Verma A and Stock W. Management of adult acute lymphoblastic leukemia: moving toward a risk-adapted approach. Curr Opin Oncol 2001; 13: 14-20.
3. Appelbaum FR. Perspectives on the future of chronic myeloid leukemia treatment. Semin Hematol 2001; 38: 35-42.
4. Campana D and Pui C-H. Detection of minimal residual disease in acute leukemia: methodologic advances and clinical significance. Blood 1995; 85: 1416-1434.
5. Van Dongen JJ, Macintyre EA, Gabert JA, Delabesse E, Rossi V, Saglio G, Gottardi E, Rambaldi A, Dotti G, Griesinger F, Parreira A, Gameiro P, Diaz MG, Malec M, Langerak AW, San Miguel JF, Biondi A. Standardized RT-PCR analysis of fusion gene transcripts from chromosome aberrations in acute leukemia for detection of minimal residual disease. Report of the BIOMED-1 Concerted Action: investigation of minimal residual disease in acute leukemia. Leukemia 1999; 13: 1901-1928.
6. van der Velden V, Hochhaus A, Cazzanigia G, Szczepanski T, Gabert J, van Dongen J. Detection of minimal residual disease in hematopoietic malignancies by real-time quantitative PCR: Principles, approaches, and laboratory aspects. Leukemia, in press 2003;
7. Marcucci G., Livak KJ, Bi W, Strout MP, Bloomfield CD, Caligiuri MA. Detection of minimal residual disease in patients with AML1/ETO-associated acute myeloid leukemia using a novel quantitative reverse transcription polymerase chain reaction assay. Leukemia 1998 ; 12:1482-1489.

## Claims

1. In a real-time quantitative reverse transcriptase polymerase chain reaction (RQ-PCR) method for minimal residual disease detection in leukemic patients through amplification of a fusion gene transcript, the improvement comprising :
(i) selecting amplifiable and qualified patient samples for subsequent analysis ;
(ii) defining optimal conditions for performing the RT reaction ;
(iii) defining optimal conditions for RQ-PCR protocol ; and
(iv) establishing a standardized procedure for data analysis,
wherein GUS is amplified in parallel to the fusion gene transcript, as control gene transcript.

2. The method according to claim 1, wherein forward primer, probe, and reverse primer sequences for *GUS* are respectively as follows :
SEQ ID N°7 (ENF1102), SEQ ID N°8 (ENPr1142) and SEQ ID N°9 (ENR1162).

3. The method according to claims 1 or 2, wherein step (ii) is as follows :
Add 1 µg of total RNA in 10 µl of H₂O ;
Incubate at 70°C for 10 min ;
Cool on ice and add following reagents to final volume of 20 µl :
Reverse Transcriptase (either MMLV or Superscript I or II): 100 U:
RT Buffer (according to the RTase used)
DNTP: 1 Mm
DTT: 10 Mm
Random Hexamers: 25 µM
RNAse inhibitor: 20 U
Incubate subsequently at:
Room temperature for 10 min ;
42°C for 45 min ; and
99°C for 3 min.
Place the sample at 4°C after RT step; and
Dilute the final cDNA with 30 µl of H₂O.

4. The method according to anyone of claims 1 to 3, wherein step (iii) is as follows :
Final volume: 25 µl ;
5 µl of final cDNA (100 ng RNA equivalent) ;
Primers: 300 nM each ;
Probe: 200 nM ;
Master Mix: 12.5 µl (1X)
Incubate the sample:
At 50°C for 2 min ; and
At 95°C for 10 min ;
Followed by 50 cycles:
95°C for 15 sec ; and
60°C for 1 min.

5. The method according to anyone of claims 1 to 4, wherein step (iv) is as follows :
- Set a common threshold at 0,1 ; and
- Set a common baseline between cycle 3 and 15.
